# EUROPEAN PATENT APPLICATION

(11) **EP 2 380 890 A1**
(43) Date of publication of application: **26.10.2011**
(21) Application number: 10382093.2
(22) Date of filing: 23.04.2010
(51) Int. Cl.: C07D 471/04, C07D 519/00, A61K 31/4375, A61K 31/4468, A61K 31/444, A61K 31/4709, A61K 31/519, A61K 31/4725, A61P 11/06, A61P 17/00, A61P 25/00, A61P 29/00, A61P 35/00

(54) **New 7,8-dihydro-1,6-naphthyridin-5(6h)-one-derivatives as PDE4 inhibitors**

(71) Applicant: Almirall, S.A., 08022 Barcelona (ES)
(72) Inventor: Richard Spurring, Roberts, 08980 Sant Feliu de Llobregat (ES); Sevilla Gomez, Sara, 08980 Sant Feliu de Llobregat (ES)
(74) Representative: Elzaburu Marquez, Alberto

(57) **Abstract**

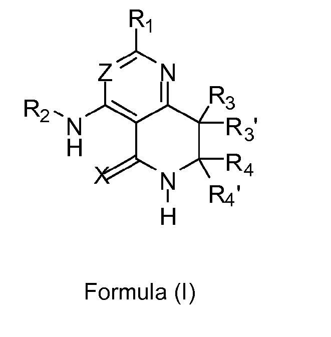

New 7,8-dihydro-1,6-naphthyridin-5(6H)-one derivatives derivatives having the chemical structure of formula (I) are disclosed; as well as process for their preparation, pharmaceutical compositions comprising them and their use in therapy as inhibitors of the phosphodiesterase IV (PDE4).

## Description

The present invention relates to new therapeutically useful 7,8-dihydro-1,6-naphthyridin-5(6H)-one derivatives, to processes for their preparation and to pharmaceutical compositions comprising them. These compounds are potent and selective inhibitors of phosphodiesterase 4 (PDE4) and are thus useful in the treatment, prevention or suppression of pathological conditions, diseases and disorders known to be susceptible of being improved by inhibition of PDE4.

Phosphodiesterases (PDEs) comprise a superfamily of enzymes responsible for the hydrolysis and inactivation of the second messenger cyclic adenosine monophosphate (cAMP) and cyclic guanosine monophosphate (cGMP). Eleven different PDE families have been identified to date (PDE1 to PDE11) which differ in substrate preference, catalytic activity, sensitivity to endogenous activators and inhibitors, and encoding genes.

The PDE4 isoenzyme family exhibits a high affinity for cyclic AMP but has weak affinity for cyclic GMP. Increased cyclic AMP levels caused by PDE4 inhibition are associated with the suppression of cell activation in a wide range of inflammatory and immune cells, including lymphocytes, macrophages, basophils, neutrophils, and eosinophils. Moreover, PDE4 inhibition decreases the release of the cytokine Tumor Necrosis Factor α (TNFα). The biology of PDE4 is described in several recent reviews, for example M. D. Houslay, P. Schafer, K. Y. Zhang, Drug Discov Today 2005, 10, 1503-19.

In view of these physiological effects, PDE4 inhibitors of varied chemical structures have been recently disclosed for the treatment or prevention of chronic and acute inflammatory diseases and other pathological conditions, diseases and disorders known to be susceptible to amelioration by inhibition of PDE4. See, for example, US 5449686, US 5710170, WO 98/45268, WO 99/06404, WO 01/57025, WO 01/57036, WO 01/46184, WO 97/05105, WO 96/40636, WO03/097613, US 5786354, US 5773467, US 5753666, US 5728712, US 5693659, US 5679696, US 5596013, US 5541219, US 5508300, US 5502072 or H. J. Dyke and J. G. Montana, Exp. Opin. Invest. Drugs 1999, 8, 1301-1325.

A few compounds having the capacity to selectively inhibit phosphodiesterase 4 are in active development. Examples of these compounds are roflumilast, GSK-256066, apremilast, tetomilast, rolipram, MK-0873 and oglemilast.

It is known that the clinical developement in man of early PDE4 inhibitors such as rolipram has been hampered by the appearance of side effects such as nausea and vomiting at therapeutic plasma levels (Curr. Pharm. Des. 2002, 8, 1255-96). The compounds described in the present invention are potent and selective PDE4 inhibitors. This property makes them useful for the treatment or prevention of pathological conditions or diseases such as respiratory diseases, skin disease, inflammatory diseases, diseases of the central or peripheral nervous system and cancer. These diseases include but are not limited to asthma, chronic obstructive pulmonary disease (COPD), allergic rhinitis, rheumatoid arthritis, multiple sclerosis, atopic dermatitis, psoriasis and inflammatory bowel disease.

PDE4 inhibitors, such as the compounds of the present invention referred to below, can also be used in combination with other drugs known to be effective in the treatment of these diseases. For example, they can be used in combination with bronchodilators such as β2-adrenergic agonists, antagonists of M3 muscarinic receptors or dual acting molecules combining β2 agonism with M3 antagonism, anti-allergics such as anti-histamines, mast cell stabilizers, CRTH2 antagonists, anti-inflammatory agents such as corticosteroids, LTD4 receptor antagonists, leukotriene synthesis inhibitors, COX inhibitors and PDE inhibitors, immunosuppressive agents such as calcineurin inhibitors, cyclosporin A, rapamycin, T-cell receptor blockers, B cell receptor blockers, and/or antiinfectives such as antibiotics, antimycotics or antiviral agents.

Accordingly, the present invention provides novel compounds of formula (I): 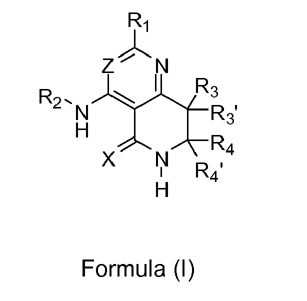 wherein:
Z represents a nitrogen atom or a -CR group;
X represents an oxygen atom or a -NR₅ group;
R represents a hydrogen atom or a linear or branched C₁-C₄ alkyl group;
R₁ represents a C₃-C₁₀ cycloalkyl group or a C₃-C₁₀ cycloalkenyl group, a monocyclic or polycyclic C₅-C₁₄ aryl group or a 5- to 14- membered heteroaryl group containing at least one heteroatom selected from O, S and N;
   wherein the cycloalkyl, cycloalkenyl, aryl and heteroaryl groups are unsubstituted or substituted by one or more substituents selected from a halogen atom, a C₁-C₄ alkoxy group, a -SR₆ group or a phenyl group, which phenyl group is unsubstituted or substituted by one or more substituents selected from a halogen atom, a hydroxy group, a linear or branched C₁-C₄ alkyl group, a -C(O)OR₇ group, a -C(O)NR₈R₉ group, or a -C(O)N(R₇)-(CH₂)ₙ-NR₈R₉ group, wherein n is 1, 2, 3 or 4;
R₂ represents a hydrogen atom, a linear or branched C₁-C₄ alkyl group, a C₁-C₄ haloalkyl group, a monocyclic or polycyclic C₅-C₁₄ aryl group, a 5- to 14- membered heteroaryl group containing at least one heteroatom selected from O, S and N, or a 5- to 14- membered heterocyclyl group containing at least one heteroatom selected from O, S and N,
   wherein the alkyl and haloalkyl groups are unsubstituted or substituted by one or more substituents selected from a cyano group, a pyridyl group, a phenyl group, a benzyl group, a -C(O)OR₇' group or a -C(O)NR₈'R₉' group; and the aryl, heteroaryl and heterocyclyl groups are unsubstituted or substituted by one or more substituents selected from a halogen atom, a cyano group, a linear or branched C₁-C₄ alkyl group, a C₁-C₄ alkoxy group, a C₁-C₄ hydroxyalkyl group, a phenyl group, a benzyl group, a -C(O)OR₇' group or a -C(O)NR₈'R₉' group;
R₃ and R₃' independently represent a hydrogen atom or a linear or branched C₁-C₄ alkyl group,
R₄ and R₄' independently represent a hydrogen atom or a linear or branched C₁-C₄ alkyl group,
R₅ represents a hydrogen atom, a hydroxy group, a cyano group, a linear or branched C₁-C₄ alkyl group or a C₁-C₄ alkoxy group;
R₆ represents a phenyl group which is unsubstituted or substituted by one or more substituents selected from a halogen atom, a linear or branched C₁-C₄ alkyl group or a C₁-C₄ alkoxy group; and
R₇, R₇', R₈, R₈', R₉ and R₉' independently represent a hydrogen atom, a linear or branched C₁-C₄ alkyl group or a C₃-C₆ cycloalkyl group; or R₈ and R₉ or R₈' and R₉', together with the nitrogen atom to which they are bounded optionally form a 3- to 10- membered, saturated N-containing heterocyclyl group, containing 1, 2 or 3 other heteroatoms selected from O, S and N, wherein said heterocyclyl group is unsubstituted or substituted by one or more substituents selected from a halogen atom, a hydroxy group or a linear or branched C₁-C₄ alkyl group,
and the pharmaceutically acceptable salt, or solvate, or N-oxide, or stereoisomer or deuterated derivate thereof.

The invention further provides synthetic processes and intermediates described herein, which are useful for preparing said compounds.

The invention also provides a pharmaceutical composition comprising at least a compound of the invention and a pharmaceutically acceptable diluent or carrier.

The invention also provides a compound of the invention for use in the treatment of the human or animal body by therapy.

The invention is also directed to the compounds of the invention as described herein, for use in the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention also provides the use of the compounds of the invention as described herein, for the manufacture of a medicament for the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention is also directed to a method of treatment of a pathological condition or disease susceptible to amelioration by inhibiton of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease; comprising administering a therapeutically effective amount of the compounds of the invention or a pharmaceutical composition of the invention to a subject in need of such treatment.

The invention also provides a combination product comprising (i) at least a compound of the invention as described herein; and (ii) one or more active ingredients selected from the group consisting of β2-adrenergic agonist, anti-cholinergic, an anti-allergic agent, an anti-inflammatory agent, an immunosuppressant and an anti-infective agent, for simultaneous, separate or sequential use in the treatment of the human or animal body.

As used herein the term C₁-C₄ alkyl embraces linear or branched radicals having 1 to 4 carbon atoms. Examples include methyl, ethyl, n-propyl, i-propyl, n-butyl, sec-butyl and t-butyl radicals.

As used herein, the term C₁-C₄ haloalkyl group is an alkyl group, for example a C₁-C₄ or C₁-C₂ alkyl group, which is bonded to one or more, preferably 1, 2 or 3 halogen atoms. Preferably, said haloakyl group is chosen from -CCl₃ and -CF₃.

As used herein, the term C₁-C₄ hydroxyalkyl embraces linear or branched alkyl radicals having 1 to 4 carbon atoms, any one of which may be substituted by one or more, preferably 1 or 2, more preferably 1 hydroxyl radicals. Examples of such radicals include hydroxymethyl, hydroxyethyl, hydroxypropyl, 2,3-dihydroxypropyl and hydroxybutyl.

As used herein, the term C₁-C₄ alkoxy (or alkyloxy) embraces linear or branched oxy-containing radicals each having alkyl portions of 1 to 4 carbon atoms. Preferred alkoxy radicals include methoxy, ethoxy, n-propoxy, i-propoxy, n-butoxy, sec-butoxy, t-butoxy, trifluoromethoxy, difluoromethoxy, hydroxymethoxy, 2-hydroxyethoxy and 2-hydroxypropoxy.

As used herein, the term C₃-C₁₀ cycloalkyl embraces saturated monocyclic or polycyclic carbocyclic radicals having from 3 to 10 carbon atoms, preferably from 3 to 7 carbon atoms, more preferably from 3 to 6 carbon atoms. Polycyclic cycloalkyl radicals contain two or more fused cycloalkyl groups, preferably two cycloalkyl groups. Typically, polycyclic cycloalkyl radicals are selected from decahydronaphthyl (decalyl), bicyclo[2.2.2]octyl, adamantyl, camphyl or bornyl groups.Examples of monocyclic cycloalkyl groups include cyclopropyl, cyclobutyl, cyclopentyl, cyclohexyl, cycloheptyl, cyclooctyl, cyclononyl and cyclodecyl.

As used herein, the term C₃-C₁₀ cycloalkenyl embraces partially unsaturated carbocyclic radicals having from 3 to 10 carbon atoms, preferably from 3 to 7 carbon atoms, more preferably from 3 to 6 carbon atoms. A C₃-C₁₀ cycloalkenyl radical is typically unsubstituted or substituted by 1, 2 or 3 substituents which may be the same or different. When a C₃-C₁₀ cycloalkenyl radical carries 2 or more substituents, the substituents may be the same or different. Typically, the substituents on a cycloalkenyl group are themselves unsubstituted.

Examples include cyclobutenyl, cyclopentenyl, cyclohexenyl, cycloheptenyl, cyclooctenyl, cyclononenyl and cyclodecenyl.

As used herein, the term C₅-C₁₄ aryl radical embraces typically a C₅-C₁₄, preferably C₆-C₁₄, more preferably C₆-C₁₀ monocyclic or polycyclic aryl radical such as phenyl, naphthyl, naphthalenyl, anthranyl and phenanthryl. Phenyl is preferred. A C₅-C₁₄ aryl radical is typically unsubstituted or substituted by 1, 2 or 3 substituents which may be the same or different. When a C₅-C₁₄ aryl radical carries 2 or more substituents, the substituents may be the same or different. Unless otherwise specified, the substituents on a C₅-C₁₄ aryl group are typically themselves unsubstituted.

As used herein, the term 5- to 14- membered heteroaryl radical embraces typically a 5- to 14- membered ring system, preferably a 5- to 10- membered ring system, more preferably a 5- to 6- membered ring system, comprising at least one heteroaromatic ring and containing at least one heteroatom selected from O, S and N. Preferably a 5- to 14-membered heteroaryl radical has 1, 2 or 3, more preferably 1 or 2, heteroatoms selected from O, S and N, preferably from O and N. A 5- to 14- membered heteroaryl radical may be a single ring or two or more fused rings wherein at least one ring contains a heteroatom.

A 5- to 14- membered heteroaryl radical is typically unsubstituted or substituted by 1, 2 or 3 substituents which may be the same or different. When a 5- to 14- membered heteroaryl radical carries 2 or more substituents, the substituents may be the same or different. Unless otherwise specified, the substituents on a 5- to 14- membered heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, 1H-pyrazolo[3,4-d]pyridinyl, thieno[2,3-d] pyrimidnyl and the various pyrrolopyridyl radicals.

As used herein, the term 5- to 14-membered heterocyclyl radical embraces typically a non-aromatic, saturated or unsaturated C₅-C₁₄ carbocyclic ring system, preferably C₅-C₁₀ carbocyclic ring system, more preferably C₅-C₆ carbocyclic ring system, in which one or more, for example 1, 2, 3 or 4 of the carbon atoms preferably 1 or 2 of the carbon atoms are replaced by a heteroatom selected from N, O and S. A heterocyclyl radical may be a single ring or two or more fused rings wherein at least one ring contains a heteroatom.

A 5- to 14-membered heterocyclyl radical is typically unsubstituted or substituted by 1, 2 or 3 substituents which may be the same or different. Where a 5- to 14-membered heterocyclyl radical carries 2 or more substituents, the substituents may be the same or different. Typically, the substituents on a 5 to 14-membered heterocyclyl radical are themselves unsubstituted.

Examples of 5- to 14-membered heterocyclyl radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, 4,5-dihydro-oxazolyl, 2-benzofuran-1(3H)-one, 1,3-dioxol-2-one and 3-aza-tetrahydrofuranyl radicals.

As used herein, the term 3- to 10-membered, saturated N-containing heterocyclyl group embraces typically a non-aromatic, saturated C₃-C₁₀ carbocyclic ring system, preferably C₄-C₉ carbocyclic ring system, more preferably C₅-C₆ carbocyclic ring system, in which one carbon atom has been replaced by a nitrogen atom and in which further carbon atoms, for example 1, 2, 3 or 4 of further carbon atoms, preferably 1 or 2 further carbon atoms are replaced by a heteroatom selected from N, O and S. A heterocyclyl radical may be a single ring or two or more fused rings wherein at least one ring contains a heteroatom. When a 3 to 10-membered, saturated N-containing heterocyclyl group carries 2 or more substituents, the substituents may be the same or different.

Examples of 3- to 10-membered, saturated N-containing heterocyclyl group include piperidyl and piperazinyl radicals.

As used herein, some of the atoms, radicals, moieties, chains and cycles present in the general structures of the invention are "optionally substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

Typically when a cyclic radical is bridged by an alkylene or alkylenedioxy radical, the bridging alkylene radical is bonded to the ring at non-adjacent atoms.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclyl amines.

Other preferred salts according to the invention are quaternary ammonium compounds wherein an equivalent of an anion (X⁻) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

Typically, in the compound of formula (I) Z represents a nitrogen atom or a -CR group, wherein R is as hereinabove defined. Preferably, Z represents a -CR group wherein R is as hereinabove defined. More preferably Z represents a -CR group wherein R is a hydrogen atom or a methyl group. Most preferably Z represents a -CH group.

Typically, in the compound of formula (I) X represents an oxygen atom or a -NR₅ group. Preferably, X represents an oxygen atom.

Typically, in the compound of formula (I) R₁ represents a cyclopropyl group, a phenyl group or a pyridyl group, wherein the cyclopropyl, phenyl and pyridyl groups are unsubstituted or substituted by 1, 2 or 3 substituents selected from a halogen atom, a C₁-C₄ alkoxy group, a -SR₆ group or a phenyl group, which phenyl group is unsubstituted or substituted by one or more substituents selected from a halogen atom, a hydroxy group, a linear or branched C₁-C₄ alkyl group, a -C(O)OR₇ group, a -C(O)NR₈R₉ group or a - C(O)N(R₇)-(CH₂)ₙ-NR₈R₉ group, wherein n is 1, 2, 3 or 4; and wherein R₆, R₇, R₈ and R₉ are as hereinabove defined.

In a particularly preferred embodiment, R₁ represents a pyridyl group, wherein said pyridyl group is optionally in the N-oxide form.

In a further particularly preferred embodiment, R₁ represents a phenyl group which is unsubstituted or substituted by 1, 2 or 3 substituents selected from a halogen atom, a C₁-C₄ alkoxy group, a -SR₆ group or a phenyl group, which phenyl group is substituted by 1, 2 or 3 substituents selected from a halogen atom, a hydroxy group, a linear or branched C₁-C₄ alkyl group, a -C(O)OR₇ group, a -C(O)NR₈R₉ group or a -C(O)N(R₇)-(CH₂)ₙ-NR₈R₉ group, wherein n is 1, 2, 3 or 4; and wherein R₆, R₇, R₈ and R₉ are as hereinabove defined. More preferably R₁ represents a phenyl group which is unsubstituted or substituted by 1 or 2 substituents selected from a halogen atom, a methoxy group, a -SR₆ group or a phenyl group, which phenyl group is substituted by 1, 2 or 3 substituents selected from a halogen atom, a hydroxy group, a methyl group, a -C(O)OR₇ group, a -C(O)NR₈R₉ group or a -C(O)N(R₇)-(CH₂)ₙ-NR₈R₉ group, wherein n is 1, 2, 3 or 4; and wherein R₆ represents a phenyl group substituted by 1, 2 or 3 C₁-C₄ alkoxy groups; and wherein R₇, R₈ and R₉ independently represent a hydrogen atom, a linear or branched C₁-C₄ alkyl group or a C₃-C₆ cycloalkyl group.

Typically, in the compound of formula (I) R₂ represents a linear or branched C₁-C₄ alkyl group, a phenyl group, a naphthalenyl group, a 5- to 14- membered heteroaryl group containing 1, 2 or 3 nitrogen atoms, or a 5- to 6- membered, saturated N-containing heterocyclyl ring; wherein the alkyl group is unsubstituted or substituted by 1, 2 or 3 substituents selected from a pyridyl group, a phenyl group, a benzyl group or -C(O)NR₈'R₉' group; and the phenyl, naphthalenyl, heteroaryl and heterocyclyl groups are unsubstituted or substituted by 1, 2 or 3 substituents selected from a halogen atom, a cyano group, a linear or branched C₁-C₄ alkyl group, a C₁-C₄ alkoxy group, a C₁-C₄ hydroxyalkyl group, a phenyl group, a -C(O)OR₇' group, or a -C(O)NR₈'R₉' group; and wherein R₇', R₈' and R₉' independently represent a hydrogen atom, a linear or branched C₁-C₄ alkyl group or a C₃-C₆ cycloalkyl group.

Preferably, R₂ represents a linear or branched C₁-C₄ alkyl group substituted by a pyridyl group, or R₂ represents a phenyl group, a naphthalenyl group, a pyridyl group, a quinolyl group, an isoquinolyl group, a cinnolinyl group, a phthalazinyl group, a quinazolinyl group, a quinoxalinyl group, a 1,8-naphthyridinyl group, a 1,6-naphthyridinyl group, a 1H-pyrazolo[3,4-b]pyridinyl group or a piperidinyl group, wherein the phenyl, naphthalenyl, pyridyl, quinolyl, isoquinolyl, cinnolinyl, phthalazinyl, quinazolinyl, quinoxalinyl, 1,8-naphthyridinyl, 1,6-naphthyridinyl, 1H-pyrazolo[3,4-b]pyridinyl or piperidinyl groups are unsubstituted or substituted by 1, 2 or 3 substituents selected from a halogen atom, a methyl group, a methoxy group, a C₁-C₄ hydroxyalkyl group, a phenyl group, a -C(O)OR₇' group or a -C(O)NR₈'R₉' group; wherein R₇', R₈' and R₉' independently represent a hydrogen atom, a linear or branched C₁-C₃ alkyl group or a C₃-C₄ cycloalkyl group.

In a preferred embodiment of the present invention, when R₂ represents a heteroaryl group or a heterocyclyl group; preferably when R₂ represents a pyridyl group, a quinolyl group, an isoquinolyl group, a cinnolinyl group, a phthalazinyl group, a quinazolinyl group, a quinoxalinyl group, a 1,8-naphthyridinyl group, a 1,6-naphthyridinyl group, a 1H-pyrazolo[3,4-b]pyridinyl group or a piperidinyl group, being the phenyl, naphthalenyl, pyridyl, quinolyl, isoquinolyl, cinnolinyl, phthalazinyl, quinazolinyl, quinoxalinyl, 1,8-naphthyridinyl, 1,6-naphthyridinyl, 1H-pyrazolo[3,4-b]pyridinyl or a piperidinyl group; more preferably when R₂ represents a pyridyl group, a quinolyl group, an isoquinolyl group, a cinnolinyl group, a phthalazinyl group, a quinazolinyl group, a quinoxalinyl group, a 1,8-naphthyridinyl group, a 1,6-naphthyridinyl group, a 1H-pyrazolo[3,4-b]pyridinyl group or a piperidinyl group; said group is in the N-oxide form.

Typically, in the compound of formula (I) R₃ and R₃' independently represent a hydrogen atom or a methyl group. Preferably R₃ and R₃' independently represent a hydrogen atom.

Typically, in the compound of formula (I) R₄ and R₄' independently represent a hydrogen atom or a methyl group. Preferably R₄ and R₄' independently represent a hydrogen atom.

Typically, in the compound of formula (I) R₅ represents a hydrogen atom, a hydroxy group or a cyano group. Preferably R₅ represents a hydroxy group or a cyano group.

Typically, R₇ represents a hydrogen atom or a linear or branched C₁-C₃ alkyl group.

Typically, R₈ represents a hydrogen atom, a linear or branched C₁-C₃ alkyl group or a C₃-C₄ cycloalkyl group.

Typically R₉ represents a hydrogen atom, a linear or branched C₁-C₃ alkyl group or a C₃-C₄ cycloalkyl group.

Typically, R₇' represents a hydrogen atom or a linear or branched C₁-C₃ alkyl group.

Typically, R₈' represents a hydrogen atom, a linear or branched C₁-C₃ alkyl group or a C₃-C₄ cycloalkyl group.

Typically, R₉' represents a hydrogen atom, a linear or branched C₁-C₃ alkyl group or a C₃-C₄ cycloalkyl group.

Typically, n is 2 or 3.

Typically, R₆ represents a phenyl group substituted by 1, 2 or 3 C₁-C₄ alkoxy groups, preferably by 1 C₁-C₄ alkoxy group.

In a particularly preferred embodiment, in the compound of formula (I):
Z represents a nitrogen atom or a -CH group;
X represents an oxygen atom or a -NR₅ group;
R₁ represents a cyclopropyl group, a pyridyl group, wherein said pyridyl group is optionally in the N-oxide form, or a phenyl group, wherein the phenyl group is unsubstituted or substituted by 1 or 2 substituents selected from a halogen atom, a methoxy group, a -SR₆ group or a phenyl group, which phenyl group is substituted by 1, 2 or 3 substituents selected from a halogen atom, a hydroxy group, a methyl group, a -C(O)OR₇ group, a - C(O)NR₈R₉ group or a -C(O)N(R₇)-(CH₂)ₙ-NR₈R₉ group, wherein n is 1, 2, 3 or 4; and wherein R₆ represents a phenyl group substituted by 1, 2 or 3 C₁-C₄ alkoxy groups; and wherein R₇, R₈ and R₉ independently represent a hydrogen atom, a linear or branched C₁-C₃ alkyl group or a C₃-C₄ cycloalkyl group;
R₂ represents a hydrogen atom, a linear or branched C₁-C₄ alkyl group, a phenyl group, a naphthalenyl group, a pyridyl group, a quinolyl group, an isoquinolyl group, a 1H-pyrazolo[3,4-b]pyridinyl group or a piperidinyl group, wherein the pyridyl, quinolyl, isoquinolyl, 1H-pyrazolo[3,4-b]pyridinyl group or piperidinyl groups are optionally in the N-oxide form; wherein the alkyl group is substituted by a pyridyl group, a phenyl group, a benzyl group or -C(O)NR₈'R₉' group; and the phenyl, naphthalenyl, pyridyl, quinolyl, isoquinolyl, 1H-pyrazolo[3,4-b]pyridinyl or piperidinyl groups are unsubstituted or substituted by 1, 2 or 3 substituents selected from a halogen atom, a cyano group, a methyl group, a C₁-C₄ alkoxy group, a C₁-C₄ hydroxyalkyl group, a phenyl group, a -C(O)OR₇' group, or a -C(O)NR₈'R₉' group; and wherein R₇', R₈' and R₉' independently represent a hydrogen atom, a linear or branched C₁-C₃ alkyl group or a C₃-C₄ cycloalkyl group;
R₃ and R₃' independently represent a hydrogen atom or a methyl group;
R₄ and R₄' independently represent a hydrogen atom or a methyl group; and
R₅ represents a hydroxy group or a cyano group.

In a further particular preferred embodiment, in the compound of formula (I):
Z represents a -CH group;
X represents an oxygen atom;
R₁ represents a cyclopropyl group, a pyridyl group, wherein said pyridyl group is optionally in the N-oxide form, and a phenyl group, wherein the phenyl group isunsubstituted or substituted by 1 or 2 substituents selected from a halogen atom, a methoxy group, a -SR₆ group or a phenyl group, which phenyl group is substituted by 1, 2 or 3 substituents selected from a halogen atom, a hydroxy group, a methyl group, a -C(O)OR₇ group, a - C(O)NR₈R₉ group or a -C(O)N(R₇)-(CH₂)ₙ-NR₈R₉ group, wherein n is 1, 2, 3 or 4; and wherein R₆ represents a phenyl group substituted by 1, 2 or 3 C₁-C₄ alkoxy groups; and wherein R₇, R₈ and R₉ independently represent a hydrogen atom, a linear or branched C₁-C₃ alkyl group or a C₃-C₄ cycloalkyl group,
R₂ represents a hydrogen atom, a linear or branched C₁-C₄ alkyl group substituted by a pyridyl group, or R₂ represents a phenyl group, a naphthalenyl group, a pyridyl group, a quinolyl group, an isoquinolyl group, a 1H-pyrazolo[3,4-b]pyridinyl group or a piperidinyl group, being the pyridyl, quinolyl, isoquinolyl, 1H-pyrazolo[3,4-b]pyridinyl group or piperidinyl groups optionally in the N-oxide form; wherein the phenyl, naphthalenyl, pyridyl, quinolyl, isoquinolyl, 1H-pyrazolo[3,4-b]pyridinyl or piperidinyl groups are unsubstituted or substituted by 1, 2 or 3 substituents selected from a halogen atom, a cyano group, a methyl group, a C₁-C₄ alkoxy group, a C₁-C₄ hydroxyalkyl group, a phenyl group, a -C(O)OR₇' group, or a -C(O)NR₈'R₉' group; and wherein R₇', R₈' and R₉' independently represent a hydrogen atom, a linear or branched C₁-C₃ alkyl group or a C₃-C₄ cycloalkyl group;
R₃ and R₃' independently represent a hydrogen atom;
R₄ and R₄' independently represent a hydrogen atom.

Particular individual compounds of the invention include:
4-[(3-methoxyphenyl)amino]-2-phenyl-7,8-dihydro-1,6-naphthyridin-5(6H)-one;
4-{[4-(2-hydroxyethyl)phenyl]amino}-2-phenyl-7,8-dihydro-1,6-naphthyridin-5(6H)-one;
4-(1-naphthylamino)-2-phenyl-7,8-dihydro-1,6-naphthyridin-5(6H)-one;
2-phenyl-4-(piperidin-4-ylamino)-7,8-dihydro-1,6-naphthyridin-5(6H)-one;
3-[(5-oxo-2-phenyl-5,6,7,8-tetrahydro-1,6-naphthyridin-4-yl)amino]benzonitrile;
4-{[3-(hydroxymethyl)phenyl]amino}-2-phenyl-7,8-dihydro-1,6-naphthyridin-5(6H)-one;
2-(3-bromophenyl)-4-[(3-methoxyphenyl)amino]-7,8-dihydro-1,6-naphthyridin-5(6H)-one;
3'-{4-[(3-methoxyphenyl)amino]-5-oxo-5,6,7,8-tetrahydro-1,6-naphthyridin-2-yl}biphenyl-3-carboxamide;
3'-{4-[(3-methoxyphenyl)amino]-5-oxo-5,6,7,8-tetrahydro-1,6-naphthyridin-2-yl}biphenyl-4-carboxylic acid;
N-[2-(dimethylamino)ethyl]-3'-{4-[(3-methoxyphenyl)amino]-5-oxo-5,6,7,8-tetrahydro-1,6-naphthyridin-2-yl}biphenyl-3-carboxamide;
2-(3'-hydroxybiphenyl-3-yl)-4-[(3-methoxyphenyl)amino]-7,8-dihydro-1,6-naphthyridin-5(6H)-one;
2-(3-methoxyphenyl)-4-[(3-methoxyphenyl)amino]-7,8-dihydro-1,6-naphthyridin-5(6H)-one;
2-(2-methoxyphenyl)-4-[(3-methoxyphenyl)amino]-7,8-dihydro-1,6-naphthyridin-5(6H)-one;
4-[(3-methoxyphenyl)amino]-2-{3-[(3-methoxyphenyl)thio]phenyl}-7,8-dihydro-1,6-naphthyridin-5(6H)-one;
4-[(3-fluoro-2-methylphenyl)amino]-2-phenyl-7,8-dihydro-1,6-naphthyridin-5(6H)-one;
4-[(3,5-dimethoxyphenyl)amino]-2-phenyl-7,8-dihydro-1,6-naphthyridin-5(6H)-one;
2-phenyl-4-[(pyridin-4-ylmethyl)amino]-7,8-dihydro-1,6-naphthyridin-5(6H)-one;
4-[(5-oxo-2-phenyl-5,6,7,8-tetrahydro-1,6-naphthyridin-4-yl)amino]benzamide;
2-methoxy-4-[(5-oxo-2-phenyl-5,6,7,8-tetrahydro-1,6-naphthyridin-4-yl)amino]benzoic acid;
3-[(5-oxo-2-phenyl-5,6,7,8-tetrahydro-1,6-naphthyridin-4-yl)amino]benzamide;
2-phenyl-4-[(2-pyridin-4-ylethyl)amino]-7,8-dihydro-1,6-naphthyridin-5(6H)-one;
4-[(3-methoxyphenyl)amino]-2-pyridin-4-yl-7,8-dihydro-1,6-naphthyridin-5(6H)-one;
4-[(3-methoxyphenyl)amino]-2-(1-oxidopyridin-4-yl)-7,8-dihydro-1,6-naphthyridin-5(6H)-one;
2-phenyl-4-(quinolin-5-ylamino)-7,8-dihydro-1,6-naphthyridin-5(6H)-one;
4-[(5-oxo-2-phenyl-5,6,7,8-tetrahydro-1,6-naphthyridin-4-yl)amino]benzoic acid;
4-[(3-methoxyphenyl)amino]-7-methyl-2-phenyl-7,8-dihydro-1,6-naphthyridin-5(6H)-one;
4-amino-2-phenyl-7,8-dihydro-1,6-naphthyridin-5(6H)-one;
2-chloro-4-[(5-oxo-2-phenyl-5,6,7,8-tetrahydro-1,6-naphthyridin-4-yl)amino]benzoic acid;
N-(5-oxo-2-phenyl-5,6,7,8-tetrahydro-1,6-naphthyridin-4-yl)-L-phenylalaninamide;
4-[(3-fluorophenyl)amino]-2-phenyl-7,8-dihydro-1,6-naphthyridin-5(6H)-one;
4-[(3-chlorophenyl)amino]-2-phenyl-7,8-dihydro-1,6-naphthyridin-5(6H)-one;
4-[(3-methoxyphenyl)amino]-2-phenyl-7,8-dihydropyrido[4,3-d]pyrimidin-5(6H)-one;
2-cyclopropyl-4-[(3-methoxyphenyl)amino]-7,8-dihydro-1,6-naphthyridin-5(6H)-one;
N-cydopropyl-3'-{4-[(3-methoxyphenyl)amino]-5-oxo-5,6,7,8-tetrahydro-1,6-naphthyridin-2-yl}biphenyl-3-carboxamide;
6-chloro-3'-(4-[(3-methoxyphenyl)amino]-5-oxo-5,6,7,8-tetrahydro-1,6-naphthyridin-2-yl}biphenyl-3-carboxylic acid;
ethyl 3'-(4-[(3-methoxyphenyl)amino]-5-oxo-5,6,7,8-tetrahydro-1,6-naphthyridin-2-yl}biphenyl-4-carboxylate;
4-(isoquinolin-5-ylamino)-2-phenyl-7,8-dihydro-1,6-naphthyridin-5(6H)-one;
2-phenyl-4-(quinolin-6-ylamino)-7,8-dihydro-1,6-naphthyridin-5(6H)-one;
4-[(4-methylpyridin-3-yl)amino]-2-phenyl-7,8-dihydro-1,6-naphthyridin-5(6H)-one;
methyl 6-hydroxy-3'-{4-[(3-methoxyphenyl)amino]-5-oxo-5,6,7,8-tetrahydro-1,6-naphthyridin-2-yl}biphenyl-3-carboxylate;
2-phenyl-4-(1H-pyrazolo[3,4-b]pyridin-3-ylamino)-7,8-dihydro-1,6-naphthyridin-5(6H)-one;
4-[(1-methyl-1H-pyrazolo[3,4-b]pyridin-3-yl)amino]-2-phenyl-7,8-dihydro-1,6-naphthyridin-5(6H)-one;
6-hydroxy-3'-{4-[(3-methoxyphenyl)amino]-5-oxo-5,6,7,8-tetrahydro-1,6-naphthyridin-2-yl}biphenyl-3-carboxylic acid;
4-[(3-methoxyphenyl)amino]-8-methyl-2-phenyl-7,8-dihydro-1,6-naphthyridin-5(6H)-one;
2-phenyl-4-[(1-phenyl-1H-pyrazolo[3,4-b]pyridin-3-yl)amino]-7,8-dihydro-1,6-naphthyridin-5(6H)-one;
2-(3'-hydroxybiphenyl-3-yl)-4-(quinolin-5-ylamino)-7,8-dihydro-1,6-naphthyridin-5(6H)-one;
4-({4-[(2R)-2,3-dihydroxypropyl]phenyl}amino)-2-phenyl-7,8-dihydro-1,6-naphthyridin-5(6H)-one;
3'-[5-oxo-4-(quinolin-5-ylamino)-5,6,7,8-tetrahydro-1,6-naphthyridin-2-yl]biphenyl-4-carboxylic acid;
N-cyclopropyl-3'-[5-oxo-4-(quinolin-5-ylamino)-5,6,7,8-tetrahydro-1,6-naphthyridin-2-yl]biphenyl-3-carboxamide;
ethyl 3'-[5-oxo-4-(quinolin-5-ylamino)-5,6,7,8-tetrahydro-1,6-naphthyridin-2-yl]biphenyl-4-carboxylate;
4-[(1-oxidoquinolin-5-yl)amino]-2-phenyl-7,8-dihydro-1,6-naphthyridin-5(6H)-one;
2-(3,4-difluorophenyl)-4-(quinolin-5-ylamino)-7,8-dihydro-1,6-naphthyridin-5(6H)-one;
[(5Z)-2-(3-methoxyphenyl)-4-[(3-methoxyphenyl)amino]-7,8-dihydro-1,6-naphthyridin-5(6H)-ylidene]cyanamide;
(5Z)-2-(3-methoxyphenyl)-4-[(3-methoxyphenyl)amino]-7,8-dihydro-1,6-naphthyridin-5(6H)-one oxime;
N-cyclopropyl-3'-[4-(isoquinolin-4-ylamino)-5-oxo-5,6,7,8-tetrahydro-1,6-naphthyridin-2-yl]biphenyl-3-carboxamide;
N-cyclopropyl-3'-{4-[(1-oxidoquinolin-5-yl)amino]-5-oxo-5,6,7,8-tetrahydro-1,6-naphthyridin-2-yl}biphenyl-3-carboxamide;
[(5Z)-2-(3,4-difluorophenyl)-4-(quinolin-5-ylamino)-7,8-dihydro-1,6-naphthyridin-5(6H)-ylidene]cyanamide;
N-cyclopropyl-3'-[5-oxo-4-(quinolin-4-ylamino)-5,6,7,8-tetrahydro-1,6-naphthyridin-2-yl]biphenyl-3-carboxamide;
N-[3-(dimethylamino)propyl]-3'-[5-oxo-4-(quinolin-5-ylamino)-5,6,7,8-tetrahydro-1,6-naphthyridin-2-yl]biphenyl-3-carboxamide;
N-cyclopropyl-6-methyl-3'-[5-oxo-4-(quinolin-5-ylamino)-5,6,7,8-tetrahydro-1,6-naphthyridin-2-yl]biphenyl-3-carboxamide;
N-cyclopropyl-5-fluoro-6-methyl-3'-[5-oxo-4-(quinolin-5-ylamino)-5,6,7,8-tetrahydro-1,6-naphthyridin-2-yl]biphenyl-3-carboxamide;
and pharmaceutically acceptable salts, solvates, N-oxides or deuterated derivatives thereof.

Of outstanding interest are:
4-[(3-Methoxyphenyl)amino]-2-phenyl-7,8-dihydro-1,6-naphthyridin-5(6H)-one;
3-[(5-Oxo-2-phenyl-5,6,7,8-tetrahydro-1,6-naphthyridin-4-yl)amino]benzonitrile;
3'-{4-[(3-Methoxyphenyl)amino]-5-oxo-5,6,7,8-tetrahydro-1,6-naphthyridin-2-yl}biphenyl-3-carboxamide;
3'-{4-[(3-Methoxyphenyl)amino]-5-oxo-5,6,7,8-tetrahydro-1,6-naphthyridin-2-yl}biphenyl-4-carboxylic acid;
N-[2-(Dimethylamino)ethyl]-3'-{4-[(3-methoxyphenyl)amino]-5-oxo-5,6,7,8-tetrahydro-1,6-naphthyridin-2-yl}biphenyl-3-carboxamide;
2-(3'-Hydroxybiphenyl-3-yl)-4-[(3-methoxyphenyl)amino]-7,8-dihydro-1,6-naphthyridin-5(6H)-one;
4-[(3-Methoxyphenyl)amino]-2-{3-[(3-methoxyphenyl)thio]phenyl}-7,8-dihydro-1,6-naphthyridin-5(6H)-one;
4-[(5-Oxo-2-phenyl-5,6,7,8-tetrahydro-1,6-naphthyridin-4-yl)amino]benzamide; 2-Methoxy-4-[(5-oxo-2-phenyl-5,6,7,8-tetrahydro-1,6-naphthyridin-4-yl)amino]benzoic acid;
4-[(3-Methoxyphenyl)amino]-2-pyridin-4-yl-7,8-dihydro-1,6-naphthyridin-5(6H)-one;
2-Phenyl-4-(quinolin-5-ylamino)-7,8-dihydro-1,6-naphthyridin-5(6H)-one;
2-Chloro-4-[(5-oxo-2-phenyl-5,6,7,8-tetrahydro-1,6-naphthyridin-4-yl)amino]benzoic acid;
N-Cyclopropyl-3'-{4-[(3-methoxyphenyl)amino]-5-oxo-5,6,7,8-tetrahydro-1,6-naphthyridin-2-yl}biphenyl-3-carboxamide;
6-Chloro-3'-{4-[(3-methoxyphenyl)amino]-5-oxo-5,6,7,8-tetrahydro-1,6-naphthyridin-2-yl}biphenyl-3-carboxylic acid;
2-Phenyl-4-(quinolin-6-ylamino)-7,8-dihydro-1,6-naphthyridin-5(6H)-one;
2-(3'-Hydroxybiphenyl-3-yl)-4-(quinolin-5-ylamino)-7,8-dihydro-1,6-naphthyridin-5(6H)-one;
3'-[5-Oxo-4-(quinolin-5-ylamino)-5,6,7,8-tetrahydro-1,6-naphthyridin-2-yl]biphenyl-4-carboxylic acid;
N-Cyclopropyl-3'-[5-oxo-4-(quinolin-5-ylamino)-5,6,7,8-tetrahydro-1,6-naphthyridin-2-yl]biphenyl-3-carboxamide;
Ethyl 3'-[5-oxo-4-(quinolin-5-ylamino)-5,6,7,8-tetrahydro-1,6-naphthyridin-2-yl]biphenyl-4-carboxylate;
4-[(1-Oxidoquinolin-5-yl)amino]-2-phenyl-7,8-dihydro-1,6-naphthyridin-5(6H)-one;
2-(3,4-Difluorophenyl)-4-(quinolin-5-ylamino)-7,8-dihydro-1,6-naphthyridin-5(6H)-one;
N-Cyclopropyl-3'-[4-(isoquinolin-4-ylamino)-5-oxo-5,6,7,8-tetrahydro-1,6-naphthyridin-2-yl]biphenyl-3-carboxamide;
N-Cyclopropyl-3'-{4-[(1-oxidoquinolin-5-yl)amino]-5-oxo-5,6,7,8-tetrahydro-1,6-naphthyridin-2-yl}biphenyl-3-carboxamide;
[(5Z)-2-(3,4-Difluorophenyl)-4-(quinolin-5-ylamino)-7,8-dihydro-1,6-naphthyridin-5(6H)-ylidene]cyanamide;
N-Cyclopropyl-3'-[5-oxo-4-(quinolin-4-ylamino)-5,6,7,8-tetrahydro-1,6-naphthyridin-2-yl]biphenyl-3-carboxamide;
N-[3-(Dimethylamino)propyl]-3'-[5-oxo-4-(quinolin-5-ylamino)-5,6,7,8-tetrahydro-1,6-naphthyridin-2-yl]biphenyl-3-carboxamide;
N-Cyclopropyl-6-methyl-3'-[5-oxo-4-(quinolin-5-ylamino)-5,6,7,8-tetrahydro-1,6-naphthyridin-2-yl]biphenyl-3-carboxamide;
N-Cyclopropyl-5-fluoro-6-methyl-3'-[5-oxo-4-(quinolin-5-ylamino)-5,6,7,8-tetrahydro-1,6-naphthyridin-2-yl]biphenyl-3-carboxamide;
and pharmaceutically acceptable salts, solvates, N-oxides or deuterated derivatives thereof.

According to another embodiment the present invention covers pharmaceutical compositions comprising at least a compound of formula (I), as hereinabove described, in admixture with pharmaceutically acceptable diluents or carriers.

In still another embodiment the present invention covers a combination product comprising
(i) at least a compound of formula (I), as hereinabove described, and
(ii) one or more active ingredients selected from the group consisting of (a) β2-adrenergic agonists, (b) anti-cholinergics such as antagonists of M3 muscarinic receptors (c) antiinflammatory agents, (d) anti-allergic agents, (e) immunosuppressants and (f) anti-infectives;
for simultaneous, separate or sequential use in the treatment of the human or animal body.

The invention is also directed to the compounds of the invention as described herein, for use in the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention also provides the use of the compounds of the invention as described herein, for the manufacture of a medicament for the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention is also directed to a method of treatment of a pathological condition or disease susceptible to amelioration by inhibiton of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease; comprising administering a therapeutically effective amount of the compounds of the invention or a pharmaceutical composition of the invention to a subject in need of such treatment.

The compounds of the present invention may be prepared by methods such as those illustrated in the following Schemes. Solvents, temperatures, pressures and other reaction conditions may readily be selected by one of ordinary skill in the art. Starting materials are commercially available or can be readily prepared by one of ordinary skill in the art using known methods.

Compounds of general formula (I) may be prepared following the synthetic scheme depicted in figure 1.

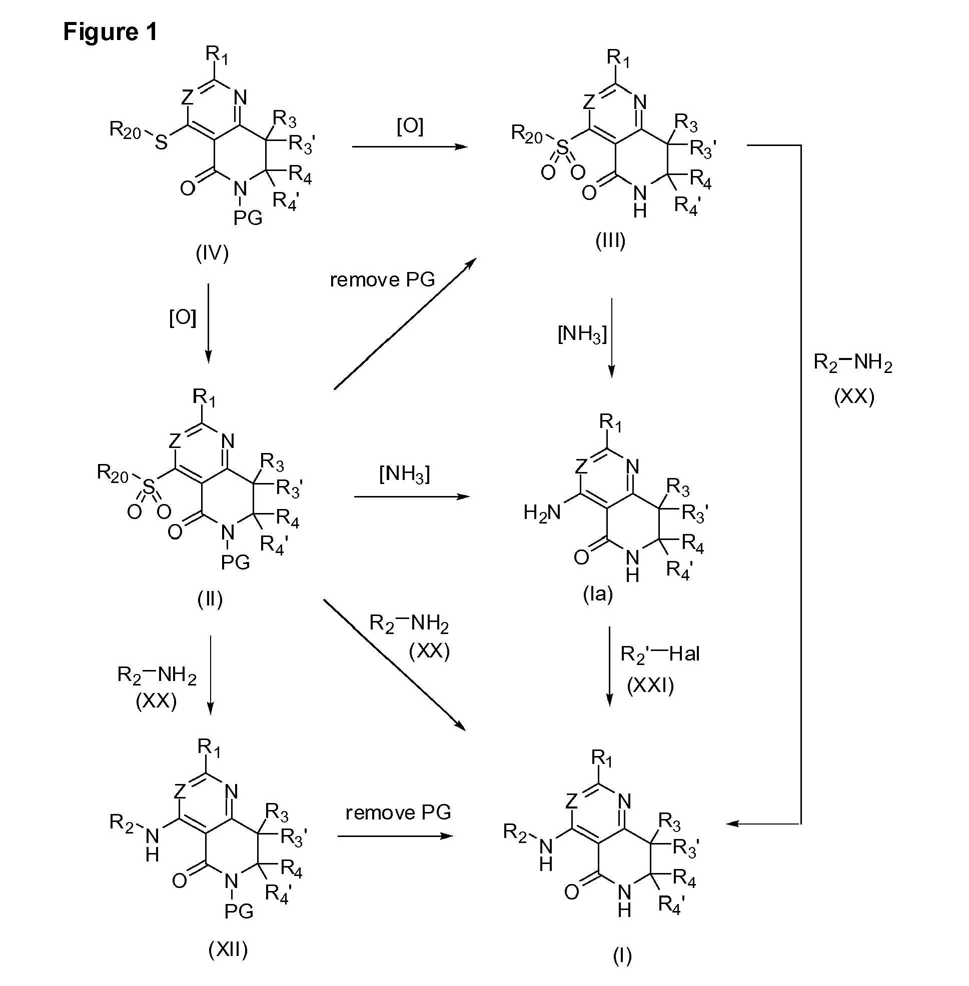

Compounds of general formula (I) may be prepared by removal of the protecting group (PG) from a compound of formula (XII) where PG represents a carbamate protecting group such as tert-butoxycarbonyl, methoxycarbonyl, ethoxycarbonyl or benzyloxycarbonyl. The reaction is carried out by mixing the compound of formula (XII) with an acid such as trifluoroacetic acid or hydrochloric acid in a solvent such as dichloromethane, chloroform or 1,4-dioxane at a temperature from -20°C to 80 °C to yield the compound of formula (I).

Alternatively, compounds of general formula (I) may be prepared by condensation of an amine of formula (XX), wherein R₂ is as hereinbefore defined, with the intermediate sulfone derivative of formula (II) wherein R₂₀ represents an alkyl group such as methyl, ethyl, propyl or butyl and wherein PG represents a carbamate protecting group such as tert-butoxycarbonyl, methoxycarbonyl, ethoxycarbonyl or benzyloxycarbonyl, and with concurrent removal of said protecting group. The reaction is carried out in a sealed tube by mixing a compound of formula (II) wherein R₂₀ and PG are as defined above with an amine of formula (XX), wherein R₂ is as hereinbefore defined, in the presence or absence of a base such as N,N'-diisopropylethylamine, in a solvent such as ethanol or tetrahydrofuran or mixtures thereof, under microwave irradiation at a temperature from 100°C to 200 °C to yield the compound of formula (I).

Alternatively, compounds of general formula (I) may be prepared by condensation of an amine of formula (XX), wherein R₂ is as hereinbefore defined, with the intermediate sulfone derivative of formula (III) wherein R₂₀ is as defined above.. The reaction is carried out in a sealed tube by mixing a compound of formula (III) wherein R₂₀ is as defined above with an amine of formula (XX), wherein R₂ is as defined above, in a solvent such as ethanol, under microwave irradiation at a temperature from 100°C to 200 °C to yield the compound of formula (I).

Alternatively, compounds of general formula (I) may be prepared by a coupling reaction between an intermediate of formula (Ia) and a compound of formula (XXI) wherein R₂' is a monocyclic or polycyclic C₅-C₁₄ aryl group, a 5- to 14- membered heteroaryl group containing at least one heteroatom selected from O, S and N, or a 5- to 14- membered heterocyclyl group containing at least one heteroatom selected from O, S and N, wherein the aryl, heteroaryl and heterocyclyl groups are unsubstituted or substituted by one or more substituents selected from a halogen atom, a cyano group, a linear or branched C₁-C₄ alkyl group, a C₁-C₄ alkoxy group, a C₁-C₄ hydroxyalkyl group, a phenyl group, a benzyl group, a -C(O)OR₇' group or a -C(O)NR₈'R₉' group;
and wherein Hal is a pseudo-halogen (any one of a group of molecules that exhibit significant similarity to the halogens) such as trifluoromethanesulfonate or a halide such as bromide or iodide. The reaction is carried out in the presence of a transition metal catalyst such as tris(dibenzylidene acetone)dipalladium(0) and suitable ligands such as phosphine ligands such as bis(diphenylphosphino)-9,9-dimethylxanthene in solvents such as toluene or dioxane, in the presence of a base such as sodium or cesium carbonate and under an inert atmosphere such as argon or nitrogen and at a temperature from ambient to the boiling point of the solvent to yield the compound of formula (I).

Compounds of general formula (Ia) may be prepared by condensation of an ammonia source, such as methanol saturated with ammonia, with the intermediate sulfone derivative of formula (II) wherein R₂₀ and PG are as defined above, and with concurrent removal of said protecting group (PG). The reaction is carried out in a sealed tube by mixing a compound of formula (II) wherein R₂₀ and PG are as defined above with the ammonia source in a solvent such as methanol, under microwave irradiation at a temperature from 50°C to 200 °C to yield the compound of formula (Ia).

Alternatively, compounds of general formula (Ia) may be prepared by condensation of an ammonia source, such as methanol saturated with ammonia, with the intermediate sulfone derivative of formula (III) wherein R₂₀ is as defined above. The reaction is carried out in a sealed tube by mixing a compound of formula (III) wherein R₂₀ is as defined above with the ammonia source in a solvent such as methanol, under microwave irradiation at a temperature from 50°C to 200 °C to yield the compound of formula (Ia).

Compounds of general formula (XII) may be prepared by condensation of an amine of formula (XX), wherein R₂ is as defined above, with the intermediate sulfone derivative of formula (II) wherein R₂₀ and PG are as defined above. The reaction is carried out by mixing the compound of formula (XII) with an amine of formula (XX), wherein R₂ is as defined above, in a solvent such as ethanol at a temperature from ambient to the boiling point of the solvent to yield the compound of formula (XII).

Compounds of general formula (II) may be prepared by oxidation of the corresponding sulphide compounds of formula (IV), wherein R₂₀ and PG are as defined above.. The reaction is carried out in the presence of an appropriate oxidizing agent such as a peroxide, a peracid, or oxone in an appropriate solvent such as dichloromethane, chloroform, tetrahydrofuran or dimethylformamide at a temperature from 0°C to the boiling point of the solvent to yield the compound of formula (II).

Compounds of general formula (III) may be prepared by removal of the protecting group from a compound of formula (II) where PG is as defined above.. The reaction is carried out by mixing the compound of formula (II) with an acid such as trifluoroacetic acid or hydrochloric acid in a solvent such as dichloromethane, chloroform or 1,4-dioxane at a temperature from -20°C to 80 °C to yield the compound of formula (III).

Alternatively, compounds of general formula (III) may be prepared by oxidation of the corresponding sulphide compounds of formula (IV), wherein R₂₀ is as defined above and wherein the group PG representing a carbamate protecting group such as tert-butoxycarbonyl, methoxycarbonyl, ethoxycarbonyl or benzyloxycarbonyl has previously been removed and hence in this instance PG represents hydrogen. The reaction is carried out in the presence of an appropriate oxidizing agent such as a peroxide, a peracid, or oxone in an appropriate solvent such as dichloromethane, chloroform, tetrahydrofuran or dimethylformamide at a temperature from 0°C to the boiling point of the solvent to yield the compound of formula (III).

Compounds of general formula (IV) may be prepared following the synthetic scheme depicted in figure 2.

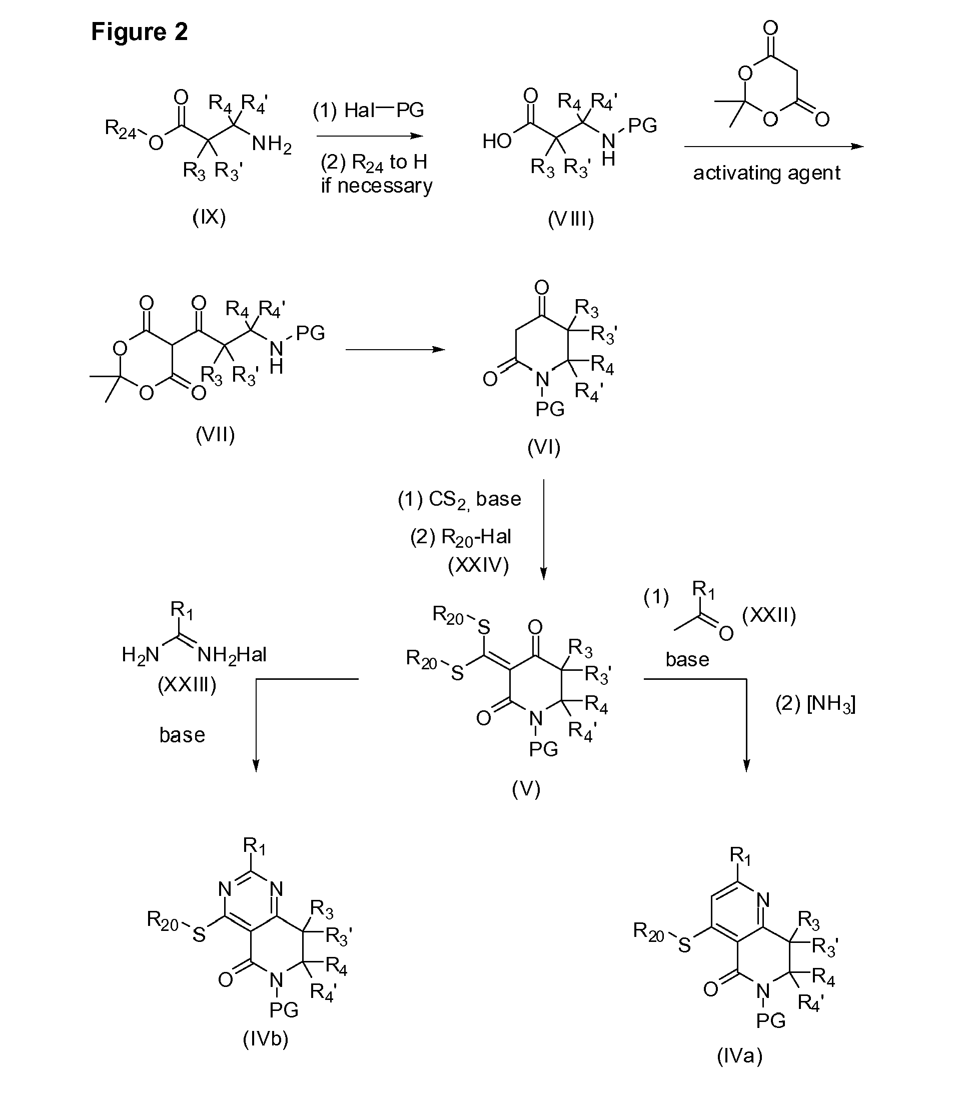

Compounds of general formula (IVa) wherein R₃, R₃', R₄ and R₄' are as hereinbefore defined may be prepared by addition of a ketone of formula (XXII) wherein R₁ is as hereinbefore defined, with an intermediate of formula (V) wherein R₂₀ represents an alkyl group such as methyl, ethyl, propyl or butyl and wherein PG represents a carbamate protecting group such as tert-butoxycarbonyl, methoxycarbonyl, ethoxycarbonyl or benzyloxycarbonyl, and then condensation with an ammonia source. The reaction is carried out by mixing the ketone of formula (XXII) wherein R₁ is as hereinbefore defined, with an intermediate of formula (V) wherein R₂₀ and PG are as defined above with a base such as potassium tert-butoxide in a polar solvent such as tetrahydrofuran, 1,4-dioxane or dimethylsulphoxide or mixtures thereof at a temperature from 0°C to 80°C. To the mixture is then added an ammonia source [NH₃] such as ammonium acetate in the presence of an acid such as acetic acid at a temperature from ambient to 120°C to yield the compound of formula (IVa).

Compounds of general formula (IVb) may be prepared by condensation of an amidine hydrohalide salt of formula (XXIII) wherein R₁ is as hereinbefore defined, and Hal is as defined above with an intermediate of formula (V) wherein R₂₀ and PG are as defined above. The reaction is carried out by mixing the compound of formula (XXIII) with a base such as sodium hydride or a metal carbonate in a solvent such as toluene, dimethylformamide or 1,4-dioxane or mixtures thereof at a temperature from -20°C to 50 °C. To the above mixture is then added a compound of formula (V) wherein R₂₀ and PG are as defined above in a solvent such as toluene, dimethylformamide or 1,4-dioxane or mixtures thereof at a temperature from ambient to the boiling point of the solvent to yield the compound of formula (IVb).

Compounds of general formula (V) may be prepared by sequential addition of carbon disulfide and two equivalents of an alkylating agent R₂₀-Hal (XXIV) wherein R₂₀ is as defined above and Hal represents a halogen such as iodide to an intermediate of formula (VI) wherein PG is as defined above in the presence of a base such as a group (I) metal carbonate. The reaction is carried out by mixing the intermediate of formula (VI) wherein PG is defined as above, with carbon disulfide in the presence of a base such as potassium carbonate in a solvent such as dimethylformamide at a temperature from 0°C to 50°C. To the mixture is added greater than two molar equivalents of an alkyl halide of formula (XXIV) wherein R₂₀ and Hal are as defined above, such as methyl iodide in a solvent such as dimethylformamide at a temperature from 0°C to 50°C to yield the compound of formula (V).

Compounds of general formula (VI) wherein R₃, R₃', R₄ and R₄' are as hereinbefore defined may be prepared from a compound of formula (VII) wherein PG represents a carbamate protecting group such as tert-butoxycarbonyl, methoxycarbonyl, ethoxycarbonyl or benzyloxycarbonyl by thermal rearrangement. The reaction is carried out by heating a compound of formula (VII) wherein PG is defined as above in a solvent such as ethyl acetate at a temperature from 30°C to the boiling point of the solvent to yield the compound of formula (VI).

Compounds of general formula (VII) may be prepared from a compound of formula (VIII) wherein PG represents a carbamate protecting group such as tert-butoxycarbonyl, methoxycarbonyl, ethoxycarbonyl or benzyloxycarbonyl with Meldrum's acid (2,2-dimethyl-1,3-dioxane-4,6-dione) in the presence of an activating agent such as a peptide coupling reagent (eg N,N'-dialkylcarbodiimide), an uronium salt, a chloroformate, a sterically hindered acid chloride or such like and a base such as N,N-dimethylaminopyridine. The reaction is carried out by mixing a compound of formula (VIII) wherein PG is defined as above with Meldrum's acid, a peptide coupling reagent such as N,N'-dicyclohexylcarbodiimide or 1-(3-diaminopropyl)-3-ethylcarbodiimide hydrochloide and a base such as N,N-dimethylaminopyridine, in a solvent such as dichloromethane or chloroform at a temperature from -20°C to 80°C to yield the compound of formula (VII).

Compounds of general formula (VIII) may be prepared from a compound of formula (IX) and a chloroformate wherein PG is as defined above, Hal is as defined above and R₂₄ represents a group such as methyl, ethyl, propyl, benzyl or a hydrogen atom, according to standard literature methods.

In particular cases, some specific compounds of the general formula (I) and, in particular, those of formula (Ic), (Id), (Ie), (If) and (Ig) may be obtained following the synthetic scheme depicted in figure 3.

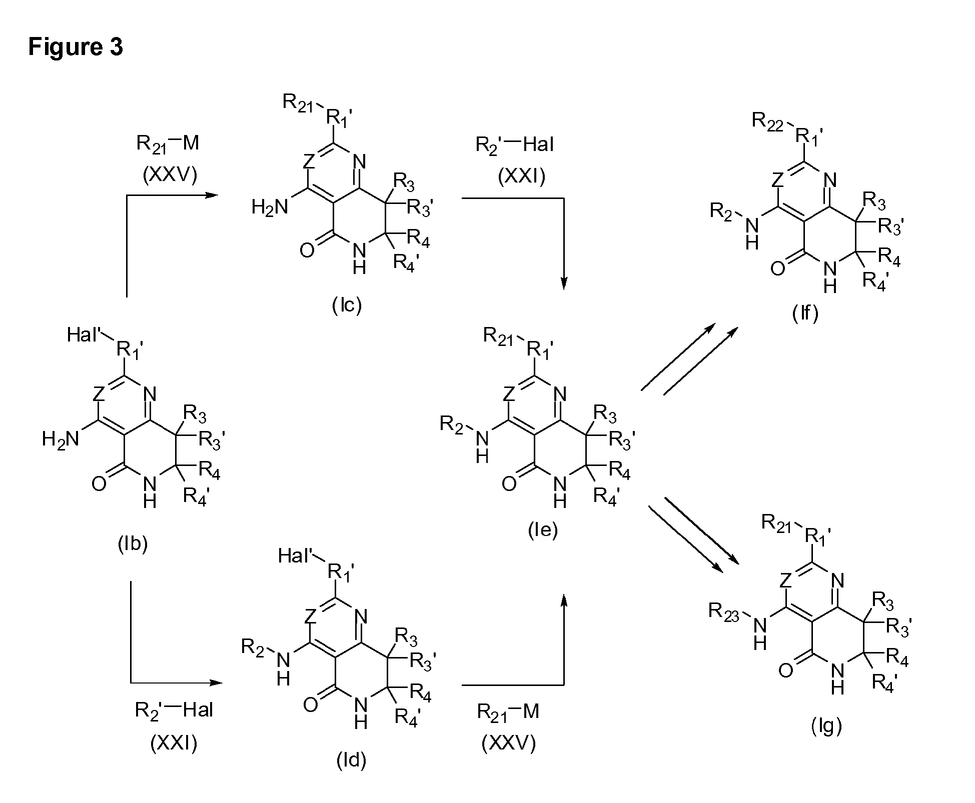

In one particular case, compounds of formula (Ic) or (Id) can be obtained from compounds of formula (Ib) wherein Z, R₃, R₃', R₄ and R₄' are as hereinbefore defined and R₁' is selected from monocyclic or polycyclic C₅-C₁₄ aryl group or a 5- to 14- membered heteroaryl group containing at least one heteroatom selected from O, S and N, wherein the aryl and heteroaryl groups are substituted by a halogen atom (Hal'), preferably bromine or iodine, and are optionally further substituted by one or more substituents as hereinbefore defined for the aryl and heteroaryl groups of R₁.

In this case, compounds of general formula (Ic) may be prepared from a compound of formula (Ib) defined as above, through reaction with an organometallic compound of formula (XXV), wherein R₂₁ is a monocyclic or polycyclic C₅-C₁₄ aryl group or a 5- to 14-membered heteroaryl group containing at least one heteroatom selected from O, S and N, wherein the aryl and heteroaryl group are optionally substituted by one or more substituents selected from a halogen atom, a C₁-C₄ alkoxy group, a -SR₆ group, a phenyl group optionally substituted by one or more substituents selected from a halogen atom, a hydroxy group, a linear or branched C₁-C₄ alkyl group, a C(O)OR₇ group, -C(O)NR₈R₉ group, or a -C(O)N(R₇)-(CH₂)ₙ-NR₈R₉ group, wherein R₆, R₇, R₈ and R₉ are as hereinbefore defined and wherein n is 1, 2, 3 or 4; and wherein M is selected from metal containing group such as lithium, magnesium halide, zinc halide, copper, copper halide, boronic acid, boronate ester derivative, trifluoroborate, trialkyl tin, trialkoxy tin, or trialkoxy silicon.

In a particular embodiment, R₁' is a phenyl group and R₂₁ is a phenyl group unsubstituted or substituted by one or more substituents as hereinbefore defined; M is a boronic acid or a boronate ester derivative. The reaction is carried out using typical Suzuki-Miyaura reaction conditions (Miyaura, N.; Suzuki, A. Chem. Rev. 1995, 95, 2457) such as in the presence of tetrakis(triphenylphosphine)palladium(0) or [1,1'-bis(diphenylphosphino)ferrocene] palladium(II) dichloride dichloromethane complex (1:1) and a base such as sodium or cesium carbonate in solvents such as toluene or dioxane under an inert atmosphere such as argon or nitrogen to yield the compound of formula (Ic).

Also in this case, compounds of general formula (Id) may be prepared by a coupling reaction between a compound of formula (Ib) defined as above, and a compound of formula (XXI) wherein R₂' is a monocyclic or polycyclic C₅-C₁₄ aryl group, a 5- to 14-membered heteroaryl group containing at least one heteroatom selected from O, S and N, or a 5- to 14- membered heterocyclyl group containing at least one heteroatom selected from O, S and N, wherein the aryl, heteroaryl and heterocyclyl groups are unsubstituted or substituted by one or more substituents selected from a halogen atom, a cyano group, a linear or branched C₁-C₄ alkyl group, a C₁-C₄ alkoxy group, a C₁-C₄ hydroxyalkyl group, a phenyl group, a benzyl group, a -C(O)OR₇' group or a -C(O)NR₈'R₉' group;
and wherein Hal is a pseudo-halogen (any one of a group of molecules that exhibit significant similarity to the halogens) such as trifluoromethanesulfonate or a halide such as bromide or iodide... The reaction is carried out in the presence of a transition metal catalyst such as tris(dibenzylidene acetone)dipalladium(0) and suitable ligands such as phosphine ligands such as bis(diphenylphosphino)-9,9-dimethylxanthene in solvents such as toluene or dioxane, in the presence of a base such as sodium or cesium carbonate and under an inert atmosphere such as argon or nitrogen and at a temperature from ambient to the boiling point of the solvent to yield the compound of formula (Id).

Furthermore, compounds of general formula (Ie) may be prepared by a coupling reaction between a compound of formula (Ic) defined as above, and a functionalised aryl group of formula (XXI) wherein R₂' and Hal are as defined above.. The reaction is carried out in the presence of a transition metal catalyst such as tris(dibenzylidene acetone)dipalladium(0) and suitable ligands such as phosphine ligands such as bis(diphenylphosphino)-9,9-dimethylxanthene in solvents such as toluene or dioxane, in the presence of a base such as sodium or cesium carbonate and under an inert atmosphere such as argon or nitrogen and at a temperature from ambient to the boiling point of the solvent to yield the compound of formula (Ie).

Alternatively, compounds of general formula (Ie) may be prepared from a compound of formula (Id) defined as above, through reaction with an organometallic compound of formula (XXV), wherein R₂₁ and M are defined as above. In a particular embodiment, R₁' is a phenyl group and R₂₁ is a phenyl group unsubstituted or substituted by one or more substituents as hereinbefore defined; M is a boronic acid or a boronate ester derivative.The reaction is carried out using typical Suzuki-Miyaura reaction conditions (Miyaura, N.; Suzuki, A. Chem. Rev. 1995, 95, 2457) such as in the presence of tetrakis(triphenylphosphine)palladium(0) or [1,1'-bis(diphenylphosphino)ferrocene] palladium(II) dichloride dichloromethane complex (1:1) and a base such as sodium or cesium carbonate in solvents such as toluene or dioxane under an inert atmosphere such as argon or nitrogen to yield the compound of formula (Ie)

In other particular cases, compounds of formula (Ie) wherein R₁' and R₂₁ are defined as above, can be further transformed to compounds of formula (If) wherein R₂₂ is defined as a phenyl group optionally substituted by one or more substituents selected from a halogen atom, a hydroxy group, a linear or branched C₁-C₄ alkyl group, a C(O)OR₇ group, a -C(O)NR₈R₉ group or a -C(O)N(R₇)-(CH₂)ₙ-NR₈R₉ group, wherein R₇, R₈ and R₉ are as hereinbefore defined and wherein n is 1, 2, 3 or 4, through standard synthetic techniques.

In other particular cases, compounds of formula (Ie) wherein R₁' and R₂₁ are defined as above, can be further transformed to compounds of formula (Ig) wherein R₂₃ is a hydrogen atom, a linear or branched C₁-C₄ alkyl group, a monocyclic or polycyclic C₅-C₁₄ aryl group, a 5- to 14- membered heteroaryl group containing at least one heteroatom selected from O, S and N, or a 5- to 14- membered heterocyclyl group containing at least one heteroatom selected from O, S and N, wherein the alkyl-, aryl, heteroaryl and heterocyclyl group is optionally substituted by one or more substituents selected from
a halogen atom,
a cyano group,
a linear or branched C₁-C₄ alkyl group, optionally substituted by one or more 5- to 6- membered N-containing heteroaryl groups,
a C₁-C₄ alkoxy group,
a C₁-C₄ hydroxyalkyl group, optionally substituted by one or more hydroxy groups,
a phenyl group,
a benzyl group, or
a C(O)OR₇ group or a -C(O)NR₈R₉ group
wherein R₇ and R₈ independently represent a hydrogen atom, a linear or branched C₁-C₄ alkyl group or a C₃-C₆ cycloalkyl group,
through standard synthetic techniques.

In another particular case, further compounds can be obtained from compounds of formula (Ib) wherein Z, R₃, R₃', R₄ and R₄' are as hereinbefore defined and R₁' is selected from monocyclic or polycyclic C₅-C₁₄ aryl group or a 5- to 14- membered heteroaryl group containing at least one heteroatom selected from O, S and N, wherein the aryl and heteroaryl groups are substituted by a halogen atom (Hal'), preferably bromine or iodine, and are optionally further substituted by one or more substituents as hereinbefore defined for the aryl and heteroaryl groups of R₁.

In this case, compounds of general formula (Ii) may be prepared from a compound of formula (Ib) defined as above as depicted in figure 4.

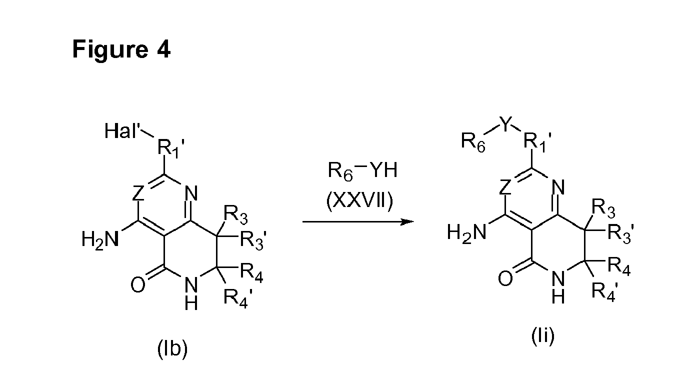

In this case, compounds of general formula (Ii) may be prepared from a compound of formula (Ib) by addition of a nucleophile of formula (XXVII) wherein R₆ is as hereinbefore defined and Y represents a sulphur atom. The reaction is carried out by mixing a compound of formula (Ib) wherein Z, R₃, R₃', R₄ and R₄' are as hereinbefore defined and wherein R₁' and Hal' are defined as above with a nucleophile of formula (XXVII) wherein R₆ and Y are as defined above in a sealed tube in the presence of a transition metal catalyst such as tetrakis(triphenylphosphine)palladium (0) and a base such as tributyltin methoxide and in a solvent such as xylene at a temperature from 40°C to 160 °C to yield the compound of formula (Ii)

In the particular case of compounds of formula (I) where X is nitrogen, compounds of general formula (Ih) may be prepared following the synthetic scheme depicted in figure 5.

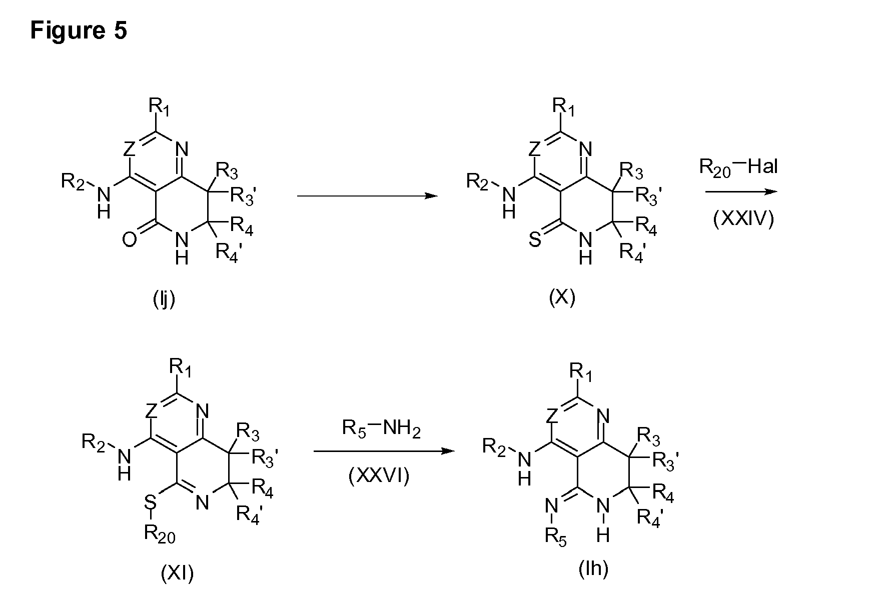

Compounds of general formula (Ih) wherein Z, R₁ R₂, R₃, R₃', R₄ and R₄' are as hereinbefore defined may be prepared by the addition of a nucleophile of formula (XXVI) wherein R₅ is as hereinbefore defined, to an intermediate of formula (XI) wherein R₂₀ represents an alkyl group such as methyl, ethyl, propyl or butyl in the presence of absence of a base such as an alkali-earth metal carbonate or a tertiary aliphatic amine in a solvent such as ethanol at a temperature from ambient to the boiling point of the solvent. The reaction is carried out by mixing the nucleophile of formula (XXVI) wherein R₅ is as hereinbefore defined, with an intermediate of formula (XI) wherein R₂₀ is as defined above in the presence or absence of a base such as diisopropylethylamine in a solvent such as ethanol at a temperature from ambient to the boiling point of the solvent to yield the compound of formula (Ih).

Compounds of general formula (XI) may be prepared by addition of an alkylating agent of formula (XXIV) wherein R₂₀ represents an alkyl group such as methyl, ethyl, propyl or butyl and Hal represents a halogen such as iodide to an intermediate of formula (X) in the presence of a base such as sodium hydride or an alkali-earth metal carbonate in a solvent such as tetrahydrofuran or dimethylformamide at a temperature from 0°C to 120 °C. The reaction is carried out by mixing a compound of formula (X) with a base such as sodium hydride in a solvent such as tetrahydrofuran at a temperature from 0°C to the boiling point of the solvent and then by adding an alkylating agent of formula (XXIV) wherein R₂₀ is defined as above to yield the compound of formula (XI).

Compounds of general formula (X) may be prepared by reaction of a compound of formula (Ij) with a thionating agent such as phosphorus pentasulfide or [2,4-bis(4-methoxyphenyl)-2,4-dithioxo-1,3,2,4-dithiadiphosphetane in the presence of a base such as diisopropylethylamine in a solvent such as tetrahydrofuran or 1,4-dioxane at a temperature from 0°C to the boiling point of the solvent. The reaction is carried out by mixing a compound of formula (Ij) wherein Z, R₁ R₂, R₃, R₃', R₄ and R₄' are as hereinbefore defined with a thionating agent such as phosphorus pentasulfide in the presence of a base such as diisopropylethylamine in a solvent such as tetrahydrofuran at a temperature from 0°C to the boiling point of the solvent to yield the compound of formula (X).

When the defined R groups are susceptible to chemical reaction under the conditions of the hereinbefore described processes or are incompatible with said processes, conventional protecting groups may be used in accordance with standard practice, for example see T. W. Greene and P. G. M. Wuts in 'Protective Groups in Organic Chemistry', 3rd Edition, John Wiley & Sons (1999). It may be that deprotection will form the last step in the synthesis of compounds of formula (I).

The syntheses of the compounds of the invention and of the intermediates for use therein are illustrated by the following Examples (1-61) (including Preparation Examples (Preparations 1 to 57) which do not limit the scope of the invention in any way.

¹H Nuclear Magnetic Resonance Spectra were recorded on a Varian Gemini 300 spectrometer. ¹H NMR spectra were not recorded for the following compounds: Preparations 22, 31, 32, 34, 35, 40, 41, 42, 44 and 54.

Low Resolution Mass Spectra (m/z) were recorded on a Micromass ZMD mass spectrometer using ESI ionization.

Melting points were recorded using a Perkin Elmer DSC-7 apparatus.

Purifications in reverse phase were made in a Biotage SP1® automated purification system equipped with a C18 column and using a gradient of H2O/Acetonitrile/MeOH (1:1) (0.1% v/v HCOONH4 both phases) from 0% to 100% acetonitrile/MeOH (1:1) in 80 column volumes. The appropriate fractions were collected and freeze dried.

The chromatographic separations were obtained using a Waters 2690 system equipped with a Symmetry C18 (2.1 x 10 mm, 3.5 mM) column. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A): initially from 0% to 95% of B in 20 min, and then 4 min. with 95% of B. The reequilibration time between two injections was 5 min. The flow rate was 0.4 mL/min. The injection volume was 5 microliter. Diode array chromatograms were collected at 210 nM.

### PREPARATION 1

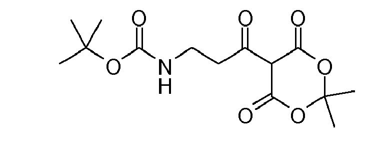

### tert-Butyl [3-(2,2-dimethyl-4,6-dioxo-1,3-dioxan-5-yl)-3-oxopropyl]carbamate

Boc-beta alanine (83 g, 0.44 mol) was dissolved in 2.7 L dichloromethane, which had been previously cooled to 5 °C in the fridge. Meldrum's acid (66.6 g, 0.46 mol) was added and the mixture shaken to dissolve. 4,4-Dimethylaminopyridine (75 g, 0.61 mol) was added and shaken to dissolve. The solution was left in the fridge for 1 h to cool to approx 5 °C. Dicyclohexylcarbodiimide (100 g, 0.48 mol) was added, the mixture shaken to dissolve and then left to stand in the fridge (5 °C) for 2 d. The mixture was filtered and the solid washed twice with cold dichloromethane. The combined filtrates were evaporated to approx 700 mL volume. The organics were washed three times with 10% w/v potassium hydrogen sulfate solution, once with brine and were dried over sodium sulfate. Filtration, evaporation of the filtrate and re-evaporation from ether gave 136 g (0.414 mol, 94%) of the title compund as a white solid. Purity 95%. Used as such without further purification.
¹H NMR (200 MHz, CHLOROFORM-*d*) δ ppm 4.85 (1H, br. s.), 3.55 (2 H, q, *J*=5.9 Hz), 3.30 (2 H, t, *J*=6.4 Hz), 1.75 (6 H, s), 1.43 (9 H, s).
HPLC/MS (5 min) retention time 3.07 min.
LRMS: *m*/*z* 316 (M+1).

### PREPARATION 2

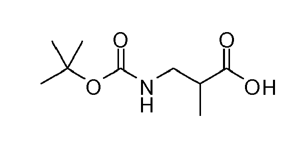

### 3-[(tert-Butoxycarbonyl)amino]-2-methylpropanoic acid

Beta-amino isobutyric acid (4.45 g, 43.2 mmol) was suspended in 80 mL dioxane and 40 mL water. 21 mL 2N sodium hydroxide (42 mmol) was added, dissolving the solid and the mixture was cooled to 5 °C in an ice-bath. Di-tert-butyl carbonate (9.38 g, 43 mmol) was added, followed by 10 mL 2N sodium hydroxide (20 mmol). The mixture was removed from the ice-bath and was stirred for 15 min. A further 10 mL 2N sodium hydroxide (20 mmol) was added and the mixture stirred for a further 30 min, giving a turbid solution. A further 9 mL 2N sodium hydroxide (18 mmol) was added and the mixture stirred for 10 min, giving a white precipitate. The mixture was neutralised with 30 mL 2N hydrochloric acid (60 mmol), dissolving the precipitate. The volume was reduced under vacuum to approx 1/3 of the original. Water and brine were added and the solution was acidified to approx pH 3 with 10% w/v potassium hydrogen sulfate solution, forming a white emulsion. The solution was extracted three times with ethyl acetate. The combined organics were washed with brine, dried over magnesium sulfate. Filtration and evaporation gave 8.37 g (41.2 mmol, 96%) of the title compound as a colourless oil which solidified upon standing overnight to give a white solid. Purity 100%.
¹H NMR (200 MHz, DMSO*-d*₆) δ ppm 12.21 (1H, br. s.), 6.84 (1H, t, *J*=5.3 Hz), 3.16 (1H, ddd, *J*=13.5, 6.6, 6.0 Hz), 2.91 (1H, ddd, *J*=13.5, 6.6, 6.4 Hz), 2.35 - 2.57 (1H, m), 1.37 (9 H, s), 1.01 (3 H, d, *J*=7.0 Hz).

### PREPARATION 3

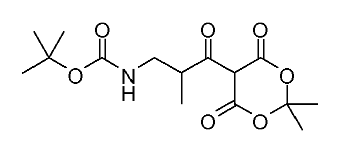

### tert-Butyl [3-(2,2-dimethyl-4,6-dioxo-1,3-dioxan-5-yl)-2-methyl-3-oxopropyl]-carbamate

Reaction of the title compound of Preparation 2 (10.1 g, 49.7 mmol) with Meldrum's acid (7.2 g, 50.0 mmol), 4,4-dimethylaminopyridine (7.3 g, 59.7 mmol) and dicyclohexylcarbodiimide (10.8 g, 52.3 mmol) according to the method described in Preparation 1 gave 16.25 g (45.9 mmol, 92%) of the title compound as pale yellow solid. Purity 93%.
¹H NMR (200 MHz, DMSO-*d*₆) δ ppm 7.03 (1H, t, *J*=5.5 Hz), 4.02 - 4.22 (1H, m), 3.02 - 3.37 (2 H, m), 1.71 (6 H, s), 1.34 (9 H, s), 1.11 (3 H, d).
HPLC/MS (5 min) retention time 2.99 min.
LRMS: *m*/*z* 330 (M+1).

### PREPARATION 4

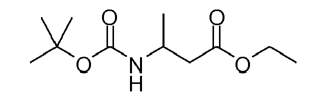

### Ethyl 3-[(tert-butoxycarbonyl)amino]butanoate

Ethyl 3-aminobutyrate (5.49 g, 41.0 mmol) was dissolved in 80 mL acetonitrile. Di-tert-butyl dicarbonate (9.14 g, 41.9 mmol) was added, dissolving rapidly with a gentle evolution of gas. The mixture was cooled in an ice-bath and zirconium chloride (0.90 g, 3.9 mmol) was added, remaining insoluble. The mixture was stirred for 5 min in the ice-bath and then for 20 min at room temperature. The mixture was evaporated and the residue taken up in 250 mL water. The aqueous was extracted three times with 250 mL ethyl acetate. The combined organics were washed with brine and dried over sodium sulfate. Filtration and evaporation gave 9.41 g (38.2 mmol, 91%) of the title compund as a colourless glassy oil. Purity 94%. Used as such without further purification
¹H NMR (200 MHz, CHLOROFORM-*d*) δ ppm 4.94 (1H, br. s.), 4.15 (2 H, q, *J*=7.2 Hz), 3.95 - 4.10 (1H, m), 2.50 (2 H, d, *J*=5.5 Hz), 1.44 (9 H, s), 1.27 (3 H, t, *J*=7.0 Hz), 1.21 (3 H, d, *J*=7.0 Hz).

### PREPARATION 5

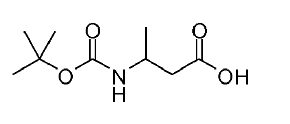

### 3-[(tert-Butoxycarbonyl)amino]butanoic acid

The crude title compound from Preparation 4 (9.41 g, 38.2 mmol) was dissolved in 30 mL of ethanol. 30 mL 2N sodium hydroxide was added and the mixture stirred at room temperature for 1 h, becoming slightly turbid. The mixture was diluted with 40 mL water and then carefully acidified to pH 4 with 2N hydrochloric acid. The aqueous was extracted three times with ethyl acetate. The combined organics were washed with brine and dried over sodium sulfate and Filtration and evaporation gave 7.70 g (37.9 mmol, 99%) of the title compound as a glassy oil which solidified upon standing overnight. Purity 100%.
¹H NMR (200 MHz, CHLOROFORM*-d*) δ ppm 4.95 (1H, br. s.), 3.91 - 4.18 (1H, m), 2.55 (2 H, d, *J*=4.7 Hz), 1.45 (9 H, s), 1.25 (3 H, d).
LRMS: *m*/*z* 202 (M-1).

### PREPARATION 6

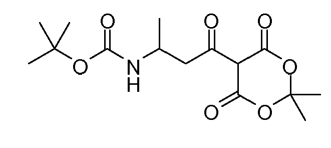

### tert-Butyl [3-(2,2-dimethyl-4,6-dioxo-1,3-dioxan-5-yl)-1-methyl-3-oxopropyl]carbamate

Reaction of the title compound of Preparation 5 (7.7 g, 37.9 mmol) with Meldrum's acid (5.9 g, 40.9 mmol), 4,4-dimethylaminopyridine (6.7 g, 54.6 mmol) and dicyclohexylcarbodiimide (8.8 g, 42.6 mmol) according to the method described in Preparation 1 gave 13.8 g (93 mmol, 103%) of the title compound as a pale yellow solid. Purity 93%. Used as such without further purification.
¹H NMR (200 MHz, CHLOROFORM*-d*) δ ppm 4.70 (2 H, d, *J*=6.6 Hz), 4.13 - 4.36 (1H, m), 3.04 - 3.36 (2 H, m), 1.75 (6 H, br. s.), 1.40 (9 H, s), 1.28 (3 H, d, *J*=6.6 Hz).
HPLC/MS (5 min) retention time 3.09 min.
LRMS: *m*/*z* 328 (M-1).

### PREPARATION 7

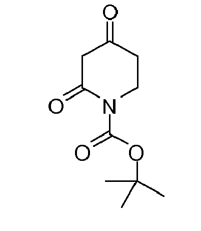

### tert-Butyl 2,4-dioxopiperidine-1-carboxylate

The crude title compound of Preparation 1 (136 g) was suspended in 250 mL ethyl acetate and heated at reflux (bath temperature 120 °C) for 2.5 h. The mixture was allowed to cool and was filtered. The filtrate was evaporated, giving an orange semisolid residue. The residue was dilssolved in 200 mL diisopropyl ether. The volume of solvent was then reduced to around 40 mL, precipitating a yellow solid. The solid was collected by filtration, washed twice with diisopropyl ether and dried in a stream of air to give 6.4 g the title compound 6.4 g (30.0 mmol, 62%) as a pale yellow solid. Purity 100%.
¹H NMR (200 MHz, CHLOROFORM*-d*) δ ppm 4.11 (2 H, t, *J*=5.9 Hz), 3.52 (2 H, s), 2.63 (2 H, t, *J*=6.1 Hz), 1.56 (9 H, s).
HPLC/MS (5 min) retention time 2.41 min.
LRMS: *m*/*z* 212 (M-1).

### PREPARATION 8

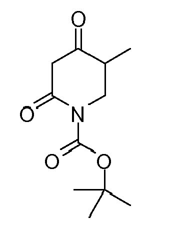

### tert-Butyl 5-methyl-2,4-dioxopiperidine-1-carboxylate

Reaction of the crude title compound of Preparation 3 (16.25 g) according to the method described in Preparation 7 gave 9.13 g (40.2 mmol, 88%) of the title compound as a pale yellow solid. Purity 100%.
¹H NMR (200 MHz, CHLOROFORM*-d*) δ ppm 4.29 (1H, dd, *J*=14.1, 5.1 Hz), 3.55 (1H, dd, *J*=14.0, 10.0 Hz), 3.54 (1H, d, *J*=18.5 Hz), 3.43 (1H, d, *J*=18.5 Hz), 2.47 - 2.69 (1H, ddq, *J*=9.7, 5, 7.1 Hz), 1.56 (9 H, s), 1.20 (3 H, d, *J*=7.0 Hz).
HPLC/MS (5 min) retention time 2.61 min.
LRMS: *m*/*z* 228 (M+1).

### PREPARATION 9

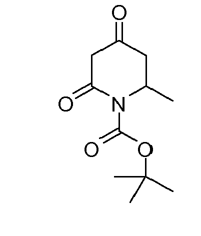

### tert-Butyl 2-methyl-4,6-dioxopiperidine-1-carboxylate

Reaction of the crude title compound of Preparation 6 (13.8 g) according to the method described in Preparation 7 gave 4.65 g (20.5 mmol, 49%) of the title compound as a pale yellow solid. Purity 100%.
¹H NMR (200 MHz, CHLOROFORM*-d*) δ ppm 4.74 (1H, dquin, *J*=1.95, 6.4 Hz), 3.44 (2 H, s), 2.84 (1H, dd, *J*=16.5, 5.5 Hz), 2.58 (1H, dd, *J*=16.5, 2.2 Hz), 1.56 (9 H, s), 1.34 (3 H, d, *J*=7.0 Hz).
HPLC/MS (5 min) retention time 2.66 min.
LRMS: *m*/*z* 226 (M-1).

### PREPARATION 10

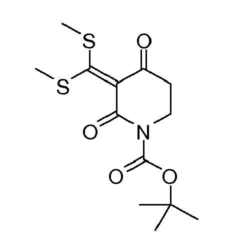

### tert-Butyl 3-[bis(methylthio)methylene]-2,4-dioxopiperidine-1-carboxylate

The title compound of Preparation 7 (22.7 g, 106 mmol) and carbon disulfide (14.0 mL, 232 mmol) were dissolved in 180 mL dry dimethyllformamide. Potassium carbonate (31 g, 224 mmol, previously powdered in a mortar) was added and the mixture stirred for 2h at room temperature, rapidly turning a deep red colour. Methyl iodide (14.0 mL, 225 mmol) dissolved in 50 mL dry dimethylformamide was added to the mixture drop-wise and with stirring over 1 h. cooling the solution during addition using a water bath. Upon addition the mixture was stirred for a further 1h at room temperature. 400 mL Water and 200 mL 4% w/v sodium carbonate solution were added and the mixture was extracted four times with ether. The combined bright red ether layer was washed with 5% w/v sodium thiosulfate solution, brine and then dried over sodium sulfate. Evaporation of the mixture gave a residue which solidified. The solid was broken up in a little diisopropyl ether and was collected by filtration. The solid was washed with a little diisopropyl ether and was dried in a stream of air to give 21.2 g (67 mmol, 63%) of the title compound as a pale yellow solid. Purity 100%.
¹H NMR (200 MHz, CHLOROFORM*-d*) δ ppm 3.90 (2 H, t, *J*=6.1 Hz), 2.60 (2 H, t), 2.53 (6 H, s), 1.54 (9H,s).
HPLC/MS (5 min) retention time 2.88 min.
LRMS: *m*/*z* 318 (M+1).

### PREPARATION 11

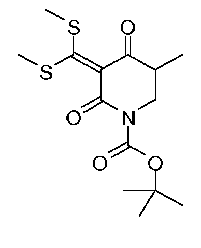

### tert-Butyl 3-[bis(methylthio)methylene]-5-methyl-2,4-dioxopiperidine-1-carboxylate

The title compound of Preparation 8 (2.27 g, 10 mmol) was treated with carbon disulfide (1.2 mL, 19.9 mmol), potassium carbonate (3.0 g, 21.7 mmol) and methyl iodide (1.25 mL, 20.1 mmol) according to the method of Preparation 10 to give 2.05 g (6.18 mmol, 62%) of the title compound as a yellow solid. Purity 100%.
¹H NMR (200 MHz, CHLOROFORM*-d*) δ ppm 4.08 (1H, dd, *J*=14.0, 5.0 Hz), 3.43 (1H, dd, *J*=13.3, 9.8 Hz), 2.52 - 2.65 (1H, m), 2.51 (6 H, s), 1.54 (9 H, s), 1.18 (3 H, d, *J*=7.0 Hz).
HPLC/MS (5 min) retention time 3.02 min.
LRMS: *m*/*z* 332 (M+1).

### PREPARATION 12

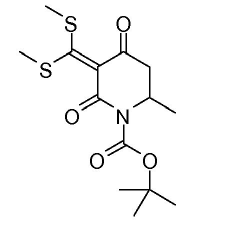

### tert-Butyl 3-[bis(methylthio)methylene]-6-methyl-2,4-dioxopiperidine-1-carboxylate

The title compound of Preparation 9 (4.6 g, 20.2 mmol) was treated with carbon disulfide (2.6 mL, 43 mmol), potassium carbonate (5.9 g, 42.7 mmol) and methyl iodide (2.5 mL, 40.2 mmol) according to the method of Preparation 10. The crude material so obtained was purified by column chromatography (ethyl acetate-hexane, 25:75) to give 4.96 g (14.5 mmol, 72%) of the title compound as a yellow solid. Purity 97%.
¹H NMR (200 MHz, CHLOROFORM*-d*) δ ppm 4.55 (1H, dquin, *J*=1.95, 6.4 Hz), 2.87 (1H, dd, *J*=17.2, 5.9 Hz), 2.52 (6 H, s), 2.47 (1H, dd, *J*=17.0, 2.0 Hz), 1.54 (9 H, s), 1.33 (3 H, d, *J*=6.6 Hz).
HPLC/MS (5 min) retention time 3.07 min.
LRMS: *m*/*z* 332 (M+1).

### PREPARATION 13

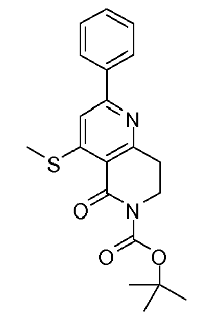

### tert-Butyl 4-(methylthio)-5-oxo-2-phenyl-7,8-dihydro-1,6-naphthyridine-6(5H)-carboxylate

The title compound of Preparation 10 (9.95 g, 31.3 mmol) and acetophenone (7.6 g, 63.3 mmol) were dissolved in 250 mL dimethylsulfoxide and 200 mL tetrahydrofuran. Potassium tert-butoxide (10.5 g, 93.6 mmol) was added in three equal portions at 30 min intervals, giving a dark red solution. After addition, the mixture was stirred for 2 h. Ammonium acetate (25 g, 324 mmol) was added followed by 200 mL acetic acid. The solution warmed and paled slightly. The mixture was heated at 90 °C for 2 h., allowed to cool to room temperature and was diluted with 200 mL water. The mixture was then cooled in an ice-water bath and was carefully neutralised with slow addition of approx 400 mL 8N sodium hydroxide solution. Solid sodium carbonate was then carefully added to basify the solution to pH 11. The mixture was extracted four times with ether. The combined ether layers were washed with brine, dried over sodium sulphate, filtered and evaporated. The residue purified by column chromatography (ethyl acetate-hexane, 20:80) to give 4.02 g (10.7 mmol, 37%) of the title compound as a pale yellow solid. Purity 99%
¹H NMR (400 MHz, CHLOROFORM*-d*) δ ppm 8.01 (2 H, d, *J*=6.7 Hz), 7.45 - 7.55 (4 H, m), 4.03 (2 H, t, J=6.3 Hz), 3.23 (2 H, t, *J*=6.3 Hz), 2.51 (3 H, s), 1.60 (9 H, s).
HPLC/MS (5 min) retention time 3.60 min.
LRMS: *m*/*z* 371 (M+1).

### PREPARATION 14

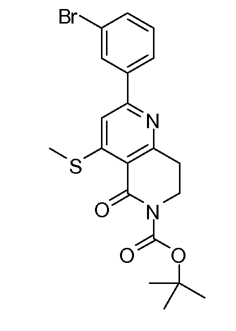

### tert-Butyl 2-(3-bromophenyl)-4-(methylthio)-5-oxo-7,8-dihydro-1,6-naphthyridine-6(5H)-carboxylate

The title compound of Preparation 10 (1.00 g, 3.15 mmol) was treated with 3-bromoacetophenone (0.83 mL, 6.30 mmol), potassium tert-butoxide (1.1 g, 9.45 mmol) and ammonium acetate (2.2 g, 28.4 mmol) according to the method of Preparation 13 to give, after purification by column chromatography using the SP1 Purification system (ethyl acetate-hexane gradient) 0.78 g (1.75 mmol, 55%) of the title compound as a pale yellow solid. Purity 100%.
¹H NMR (200 MHz, CHLOROFORM*-d*) δ ppm 8.19 (1H, s), 7.93 (1H, d, *J*=7.4 Hz), 7.61 (1H, d, *J*=7.8 Hz), 7.46 (1H, s), 7.37 (1H, t, *J*=8.0 Hz), 4.03 (2 H, t, *J*=6.2 Hz), 3.23 (2 H, t, *J*=6.2 Hz), 2.52 (3 H, s), 1.60 (9 H, s).
HPLC/MS (9 min) retention time 7.51 min.
LRMS: *m*/*z* 449, 451 (M+1).

### PREPARATION 15

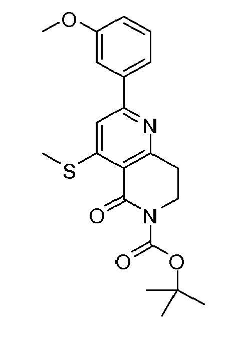

### tert-Butyl 2-(3-methoxyphenyl)-4-(methylthio)-5-oxo-7,8-dihydro-1,6-naphthyridine-6(5H)-carboxylate

The title compound of Preparation 10 (1.51 g, 4.76 mmol) was treated with 3-methoxyacetophenone (1.31 mL, 9.5 mmol), potassium tert-butoxide (1.74 g, 15.6 mmol) and ammonium acetate (3.6 g, 46.7 mmol) according to the method of Preparation 13 to give, after purification by column chromatography (ethyl acetate-hexane gradient) 0.60 g (1.50 mmol, 32%) of the title compound as a pale yellow solid. Purity 100%.
¹H NMR (200 MHz, CHLOROFORM*-d*) δ ppm 7.51 - 7.64 (2 H, m), 7.49 (1H, s), 7.41 (1H, t, *J*=7.8 Hz), 7.02 (1H, d, *J*=7.8 Hz), 4.02 (2 H, t, *J*=6.2 Hz), 3.90 (3 H, s), 3.23 (2 H, t, *J*=6.2 Hz), 2.50 (3 H, s), 1.60 (9 H, s).
HPLC/MS (5 min) retention time 3.66 min.
LRMS: *m*/*z* 401 (M+1).

### PREPARATION 16

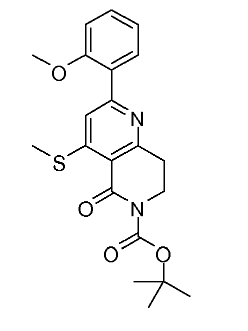

### tert-Butyl 2-(2-methoxyphenyl)-4-(methylthio)-5-oxo-7,8-dihydro-1,6-naphthyridine-6(5H)-carboxylate

The title compound of Preparation 10 (1.50 g, 4.75 mmol) was treated with 2-methoxyacetophenone (1.30 mL, 9.5 mmol), potassium tert-butoxide (1.64 g, 14.6 mmol) and ammonium acetate (3.6 g, 46.7 mmol) according to the method of Preparation 13 to give, after purification by column chromatography (ethyl acetate-hexane gradient) 0.143 g (0.36 mmol, 8%) of the title compound as a pale yellow solid. Purity 100%.
¹H NMR (200 MHz, CHLOROFORM*-d*) δ ppm 7.85 (1H, d, *J*=7.0 Hz), 7.74 (1H, br. s.), 7.40 (1H, t, *J*=7.0 Hz), 6.95 - 7.17 (2 H, m), 4.00 (2 H, t, *J*=6.0 Hz), 3.87 (3 H, s.), 3.20 (2 H, t, *J*=6.0 Hz), 2.43 (3 H, s.), 1.58 (9 H, s.).
HPLC/MS (5 min) retention time 3.56 min.
LRMS: *m*/*z* 401 (M+1).

### PREPARATION 17

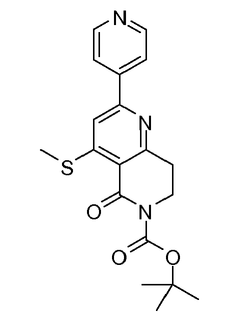

### tert-Butyl 4-(methylthio)-5-oxo-2-pyridin-4-yl-7,8-dihydro-1,6-naphthyridine-6(5H)-carboxylate

The title compound of Preparation 10 (1.50 g, 4.75 mmol) was treated with (4-pyridyl) methyl ketone (1.05 mL, 9.5 mmol), potassium tert-butoxide (2.12 g, 18.8 mmol) and ammonium acetate (3.6 g, 46.7 mmol) according to the method of Preparation 13 to give, after purification by column chromatography (methanol-dichloromethane gradient) 0.214 g (0.524 mmol, 11 %) of the title compound as a colourless oil. Purity 91 %.
¹H NMR (200 MHz, CHLOROFORM*-d*) δ ppm 8.77 (2 H, d, *J*=5.1 Hz), 7.91 (2 H, d, *J*=5.5 Hz), 7.56 (1H, s), 4.04 (2 H, t, *J*=6.2 Hz), 3.24 (2 H, t, *J*=6.1 Hz), 2.53 (3 H, s), 1.60 (9H,s).
HPLC/MS (5 min) retention time 3.10 min.
LRMS: *m*/*z* 372 (M+1).

### PREPARATION 18

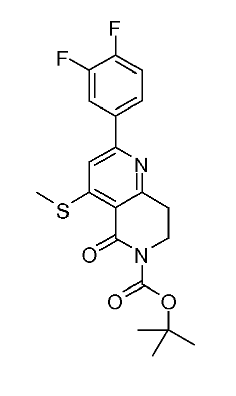

### tert-Butyl 2-(3,4-difluorophenyl)-4-(methylthio)-5-oxo-7,8-dihydro-1,6-naphthyridine-6(5H)-carboxylate

The title compound of Preparation 10 (1.75 g, 5.5 mmol) was treated with 3,4-difluoroacetophenone (1.7 g, 10.9 mmol), potassium tert-butoxide (1.8 g, 16.0 mmol) and ammonium acetate (4.2 g, 54.5 mmol) according to the method of Preparation 13 to give, after purification by column chromatography (methanol-dichloromethane gradient) 0.556 g (1.37 mmol, 25%) of the title compound as a pale yellow solid. Purity 100%.
¹H NMR (200 MHz, CHLOROFORM*-d*) δ ppm 7.91 (1H, ddd, *J*=12.0, 7.5, 2.0 Hz), 7.77 (1H, ddd, *J*=8.5, 4.0, 2.0 Hz), 7.43 (1H, s), 7.28 (1H, q, *J*=8.6 Hz), 4.02 (2 H, t, *J*=6.2 Hz), 3.20 (2 H, t, *J*=6.4 Hz), 2.51 (3 H, s), 1.60 (9 H, s).
HPLC/MS (15 min) retention time 10.53 min.
LRMS: *m*/*z* 407 (M+1).

### PREPARATION 19

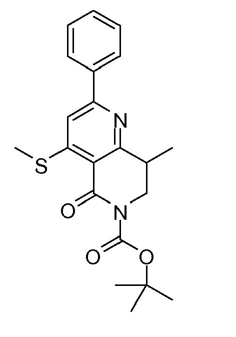

### tert-Butyl 8-methyl-4-(methylthio)-5-oxo-2-phenyl-7,8-dihydro-1,6-naphthyridine-6(5H)-carboxylate

The title compound of Preparation 11 (0.74 g, 2.12 mmol) was treated with acetophenone (0.51 g, 4.24 mmol), potassium tert-butoxide (0.70 g, 6.2 mmol) and ammonium acetate (1.6 g, 20.8 mmol) according to the method of Preparation 13 to give, after purification by column chromatography (ethyl acetate-hexane, 20:80) 0.29 g (0.76 mmol, 36%) of the title compound as a pale yellow solid. Purity 100%.
¹H NMR (200 MHz, CHLOROFORM*-d*) δ ppm 7.98 - 8.12 (2 H, m), 7.49 (4 H, d, *J*=4.7 Hz), 4.04 (1H, dd, *J*=13.5, 4.5 Hz), 3.79 (1H, dd, *J*=13.5, 7.5 Hz), 3.13 - 3.37 (1H, m), 2.50 (3 H, s), 1.59 (9 H, s), 1.47 (3 H, d).
HPLC/MS (5 min) retention time 3.78 min.
LRMS: *m*/*z* 385 (M+1).

### PREPARATION 20

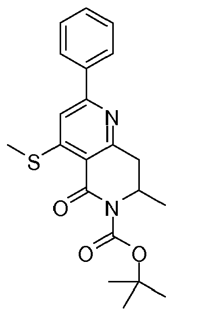

### tert-Butyl 7-methyl-4-(methylthio)-5-oxo-2-phenyl-7,8-dihydro-1,6-naphthyridine-6(5H)-carboxylate

The title compound of Preparation 12 (1.05 g, 3.17 mmol) was treated with acetophenone (0.76 g, 6.33 mmol), potassium tert-butoxide (1.05 g, 9.36 mmol) and ammonium acetate (2.5 g, 32.4 mmol) according to the method of Preparation 13. Purification first by column chromatography (ethyl acetate-hexane, 30:70), then by column chromatography (methanol-dichloromethane, 1:99) gave 0.36 g (0.92 mmol, 29%) of the title compound as a pale yellow solid. Purity 99%.
¹H NMR (200 MHz, CHLOROFORM*-d*) δ ppm 7.98 - 8.06 (2 H, m), 7.46 - 7.54 (4 H, m), 4.72 (1 H, dquin, *J*=2.0, 6.4 Hz), 3.53 (1 H, dd, *J*=16.5, 6.5 Hz), 3.03 (1 H, dd, *J*=16.6, 1.8 Hz), 2.52 (3 H, s), 1.60 (9 H, s), 1.28 (3 H, d, *J*=6.6 Hz).
HPLC/MS (5 min) retention time 3.77 min.
LRMS: *m*/*z* 385 (M+1).

### PREPARATION 21

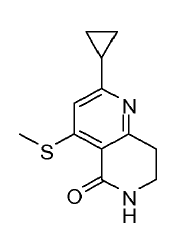

### 2-Cyclopropyl-4-(methylthio)-7,8-dihydro-1,6-naphthyridin-5(6H)-one

The title compound of Preparation 10 (1.50 g, 4.75 mmol) was treated with cyclopropyl methyl ketone (0.92 mL, 9.3 mmol), potassium tert-butoxide (1.71 g, 15.1 mmol) and ammonium acetate (5.9 g, 76.7 mmol) according to the method of Preparation 13, extending the reaction time with ammonium acetate to 2 d. Purification by column chromatography (methanol-dichloromethane gradient) gave 90 mg of the crude title compound as a pale yellow solid. Purity 70%. Used as such without further purification.
¹H NMR (partial, 200 MHz, CHLOROFORM*-d*) δ ppm 6.90 (1 H, s), 6.36 (1 H, br. s.), 3.52 (2 H, dt, *J*=6.8, 3.0 Hz), 3.03 (2 H, t, *J*=6.8 Hz), 2.41 (3 H, s).
HPLC/MS (5 min) retention time 2.02 min.
LRMS: *m*/*z* 235 (M+1).

### PREPARATION 22

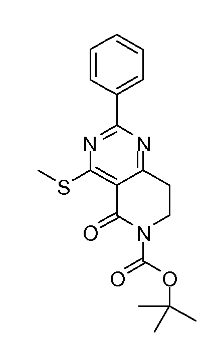

### tert-Butyl 4-(methylthio)-5-oxo-2-phenyl-7,8-dihydropyrido[4,3-d]pyrimidine-6(5H)-carboxylate

Phenylamidine hydrochloride (0.25 g, 1.58 mmol) was suspended in 5 mL toluene and 1 mL dimethylformamide. Sodium hydride (60% dispersion in oil, 0.125 g, 3.15 mmol) was added and the mixture was stirred for 5 min. The title compound from Preparation 10 (0.50 g, 1.58 mmol) was added along with 4 mL dimethylformamide and the mixture was stirred at 100 °C for 2 d. The mixture was evaporated and the crude residue purified by reverse-phase chromatography using the SP1 Purification system. 60 mg (0.15 mmol, 9%) of the title compound was recovered as a colourless oil. Purity 93%. Used as such without further purification.
HPLC/MS (9 min) retention time 6.31 min.
LRMS: *m*/*z* 372 (M+1).

### PREPARATION 23

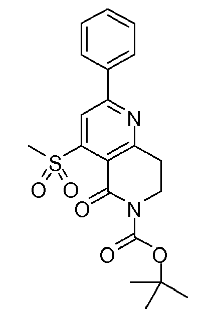

### tert-Butyl 4-(methylsulfonyl)-5-oxo-2-phenyl-7,8-dihydro-1,6-naphthyridine-6(5H)-carboxylate

The title compound of Preparation 13 (4.02 g, 10.8 mmol) was dissolved in 150 mL dichloromethane and cooled to 5 °C in an ice-bath. meta-Chloroperbenzoic acid (max purity 77%, 7.25 g, 32.4 mmol) was added and the mixture was stirred for 30 min at 5 °C. The mixture was then stirred at room temperature for 2 h. The organics were washed three times with 4% w/v sodium carbonate solution, once with brine and dried over sodium sulfate. Filtration and evaporation gave 4.40 g of the title compound (10.7 mmol, 99%) as a white solid. Purity 98%.
¹H NMR (400 MHz, CHLOROFORM*-d*) δ ppm 8.50 (1 H, s), 8.14 (2 H, dd, *J*=6.5, 2.9 Hz), 7.50 - 7.57 (3 H, m), 4.10 (2 H, t, *J*=6.1 Hz), 3.69 (3 H, s), 3.33 (2 H, t, *J*=6.1 Hz), 1.60 (9H,s).
HPLC/MS (5 min) retention time 3.40 min.
LRMS: *m*/*z* 420 (M+18), 347 (M+1-isobutene).

### PREPARATION 24

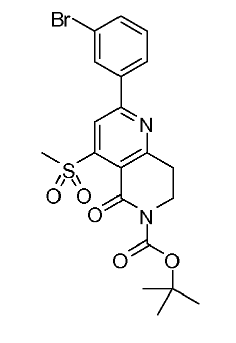

### tert-Butyl 2-(3-bromophenyl)-4-(methylsulfonyl)-5-oxo-7,8-dihydro-1,6-naphthyridine-6(5H)-carboxylate

The title compound of Preparation 14 (0.78 g, 1.75 mmol) was treated with meta-chloroperbenzoic acid (max purity 77%, 1.2 g, 5.3 mmol) according to the method of Preparation 23, to give 0.84 mg (1.75 mmol, 100%) of the title compound as a white solid. Purity 100%.
¹H NMR (200 MHz, CHLOROFORM*-d*) δ ppm 8.47 (1 H, s), 8.33 (1 H, s), 8.05 (1 H, d, *J*=7.8 Hz), 7.66 (1 H, d, *J*=7.8 Hz), 7.41 (1 H, t, *J*=8.0 Hz), 4.11 (2 H, t, *J*=6.1 Hz), 3.69 (3 H, s), 3.33 (2 H, t, *J*=6.1 Hz), 1.60 (9 H, s).
HPLC/MS (5 min) retention time 3.74 min.
LRMS: *m*/*z* 498,500 (M+18).

### PREPARATION 25

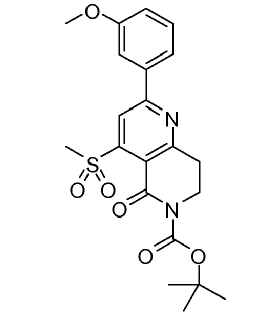

### tert-Butyl 2-(3-methoxyphenyl)-4-(methylsulfonyl)-5-oxo-7,8-dihydro-1,6-naphthyridine-6(5H)-carboxylate

The title compound of Preparation 15 (0.60 g, 1.50 mmol) was treated with meta-chloroperbenzoic acid (max purity 77%, 1.0 g, 4.46 mmol) according to the method of Preparation 23, to give 0.56 g (1.27 mmol, 85%) of the title compound as a white solid. Purity 98%.
¹H NMR (200 MHz, CHLOROFORM*-d*) δ ppm 8.47 (1 H, s), 7.63 - 7.75 (2 H, m), 7.43 (1 H, t, *J*=8.0 Hz), 7.07 (1 H, dd, *J*=8.0, 1.8 Hz), 4.09 (2 H, t, *J*=6.2 Hz), 3.91 (3 H, s), 3.68 (3 H, s), 3.32 (2 H, t, *J*=6.1 Hz), 1.59 (9 H, s).
HPLC/MS (5 min) retention time 3.46 min.
LRMS: *m*/*z* 450 (M+18).

### PREPARATION 26

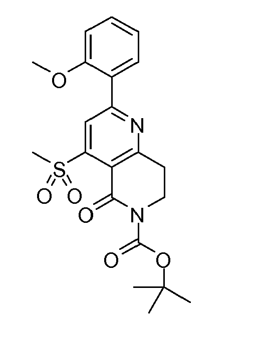

### tert-Butyl 2-(2-methoxyphenyl)-4-(methylsulfonyl)-5-oxo-7,8-dihydro-1,6-naphthyridine-6(5H)-carboxylate

The title compound of Preparation 16 (0.143 g, 0.357 mmol) was treated with meta-chloroperbenzoic acid (max purity 77%, 0.27 g, 1.20 mmol) according to the method of Preparation 23, to give after purification by column chromatography (ethyl acetate-hexane, 50:50) 103 mg (0.24 mmol, 67%) of the title compound as a white solid. Purity 100%.
¹H NMR (200 MHz, CHLOROFORM*-d*) δ ppm 8.70 (1 H, s), 7.92 (1 H, d, *J*=7.8 Hz), 7.46 (1 H, t, *J*=7.8 Hz), 6.97 - 7.18 (2 H, m), 4.08 (2 H, t, *J*=6.1 Hz), 3.91 (3 H, s), 3.66 (3 H, s), 3.30 (2 H, t, *J*=6.1 Hz), 1.58 (9 H, s).
HPLC/MS (5 min) retention time 3.42 min.
LRMS: *m*/*z* 433 (M+1), 450 (M+18).

### PREPARATION 27

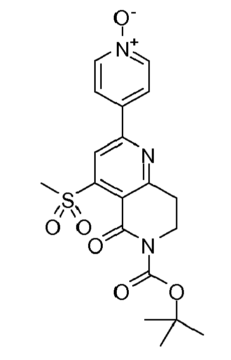

### tert-Butyl 4-(methylsulfonyl)-2-(1-oxidopyridin-4-yl)-5-oxo-7,8-dihydro-1,6-naphthyridine-6(5H)-carboxylate

The title compound of Preparation 17 (0.214 g, 0.524 mmol) was treated with meta-chloroperbenzoic acid (max purity 77%, 0.60 g, 3.46 mmol) according to the method of Preparation 23, extending the reaction time to 28 h, to give 191 mg (0.45 mmol, 77%) of the title compound as a white solid. Purity 98%.
¹H NMR (200 MHz, CHLOROFORM*-d*) δ ppm 8.42 (1 H, s), 8.26 (2 H, d, *J*=7.0 Hz), 8.07 (2 H, d), 4.06 (2 H, t, *J*=6.1 Hz), 3.63 (3 H, s), 3.27 (2H, t, *J*=6.1 Hz), 1.53 (9 H, s).
HPLC/MS (5 min) retention time 2.68 min.
LRMS: *m*/*z* 420 (M+1).

### PREPARATION 28

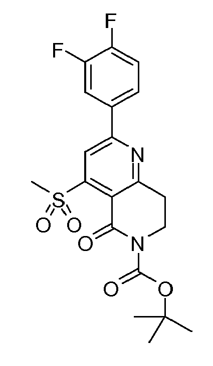

### tert-Butyl 2-(3,4-difluorophenyl)-4-(methylsulfonyl)-5-oxo-7,8-dihydro-1,6-naphthyridine-6(5H)-carboxylate

The title compound of Preparation 18 (0.55 g, 1.35 mmol) was treated with meta-chloroperbenzoic acid (max purity 77%, 0.92 g, 4.1 mmol) according to the method of Preparation 23, to give 0.48 g (1.09 mmol, 81%) of the title compound as a white solid. Purity 100%.
¹H NMR (200 MHz, CHLOROFORM*-d*) δ ppm 8.43 (1 H, s), 8.06 (1 H, ddd, J=11.3, 8.6, 2.3 Hz), 7.83 - 7.96 (1 H, m), 7.32 (1 H, q, J=8.5 Hz), 4.10 (2 H, t, J=6.2 Hz), 3.69 (3 H, s), 3.32 (2 H, t, J=6.1 Hz), 1.60 (9 H, s).
HPLC/MS (5 min) retention time 3.53 min.
LRMS: m/z 439 (M+1)

### PREPARATION 29

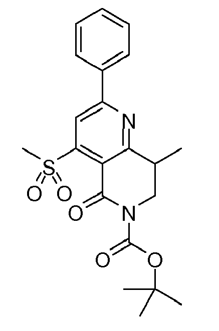

### tert-Butyl 8-methyl-4-(methylsulfonyl)-5-oxo-2-phenyl-7,8-dihydro-1,6-naphthyridine-6(5H)-carboxylate

The title compound of Preparation 19 (290 mg, 0.76 mmol) was treated with meta-chloroperbenzoic acid (max purity 77%, 0.50 g, 2.2 mmol) according to the method of Preparation 23, to give 264 mg (0.63 mmol, 84%) of the title compound as a white solid. Purity 100%.
¹H NMR (200 MHz, CHLOROFORM*-d*) δ ppm 8.50 (1 H, s), 8.11 - 8.23 (2 H, m), 7.49 - 7.59 (3 H, m), 4.14 (1 H, dd, *J*=13.3, 3.9 Hz), 3.83 (1 H, dd, *J*=13.5, 7.5 Hz), 3.69 (3 H, s), 3.29 - 3.48 (1 H, m, *J*=7.5, 7.0, 7.0, 7.0, 4.0 Hz), 1.60 (9 H, s), 1.53 (3 H, d, *J*=7.0 Hz).
HPLC/MS (5 min) retention time 3.58 min.
LRMS: *m*/*z* 434 (M+18).

### PREPARATION 30

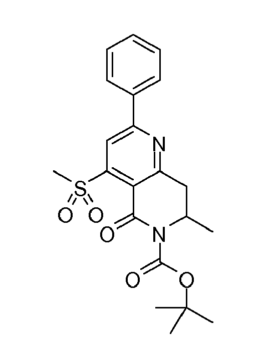

### tert-Butyl 7-methyl-4-(methylsulfonyl)-5-oxo-2-phenyl-7,8-dihydro-1,6-naphthyridine-6(5H)-carboxylate

The title compound of Preparation 20 (166 mg, 0.43 mmol) was treated with meta-chloroperbenzoic acid (max purity 77%, 0.33 g, 1.47 mmol) according to the method of Preparation 23, to give 124 mg (0.29 mmol, 70%) of the title compound as a white solid. Purity 98%.
¹H NMR (200 MHz, CHLOROFORM*-d*) δ ppm 8.52 (1 H, s), 8.09 - 8.22 (2 H, m), 7.50 - 7.63 (3 H, m), 4.82 (1 H, dquin, *J*=1.8, 6.6 Hz), 3.69 (3 H, s), 3.62 (1 H, dd, *J*=17.0, 6.0 Hz), 3.17 (1 H, dd, *J*=16.8, 1.8 Hz), 1.60 (9 H, s), 1.29 (3 H, d, *J*=6.6 Hz).
HPLC/MS (9 min) retention time 6.86 min.
LRMS: *m*/*z*418 (M+1), 434 (M+18).

### PREPARATION 31

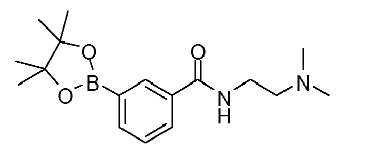

### N-[2-(Dimethylamino)ethyl]-3-(4,4,5,5-tetramethyl-1,3,2-dioxaborolan-2-yl)benzamide

*N*,*N-*Dimethylethane-1,2-diamine (0.71 g, 8.06 mmol) was added to a solution of 3-(4,4,5,5-tetramethyl-1,3,2-dioxaborolan-2-yl)benzoic acid (1.00 g, 4.03 mmol), 1-hydroxybenzotriazole hydrate (0.82 g, 6.05 mmol) and *N-*ethyl-*N*'-(3-dimethylaminopropyl)carbodiimide hydrochloride (1.16 g, 6.05 mmol) in 8 mL dimethylformamide. The mixture was stirred at room temperature for 3 h. The solvent was removed under reduced pressure and the residue taken up in 4% sodium bicarbonate solution. The aqueous phase was extracted three times with ethyl acetate. The combined organic layers were washed with water and brine, and dried over anhydrous sodium sulphate. The solvent was removed in vacuum to give 0.81 g (2.55 mmol, 63%) of the title compound.
HPLC/MS (9 min) retention time 4.23 min.
LRMS: *m*/*z* 319 (M+1)

### PREPARATION 32

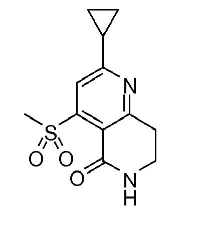

### 2-Cyclopropyl-4-(methylsulfonyl)-7,8-dihydro-1,6-naphthyridin-5(6H)-one

The title compound of Preparation 21 (63 mg, 0.27 mmol) was treated with meta-chloroperbenzoic acid (max purity 77%, 0.15 g, 0.67 mmol) according to the method of Preparation 23, to give 13 mg (0.04 mmol, 15%) of the title compound.
Purity 80%.
HPLC/MS (5 min) retention time 2.46 min.
LRMS: *m*/*z* 267 (M+1).

### PREPARATION 33

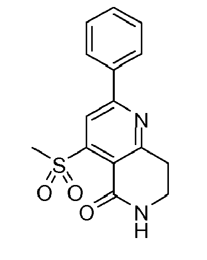

### 4-(Methylsulfonyl)-2-phenyl-7,8-dihydro-1,6-naphthyridin-5(6H)-one

The title compound of Preparation 23 (212 mg, 0.53 mmol) was dissolved in 5 mL of dichloromethane and trifluoroacetic acid (1 mL, 13 mmol) was added dropwise and the mixture was allowed to stand at room temperatre for 1 h. The mixture was diluted with 50 mL of chloroform and was evaporated to give a residue. Water (5 mL) was added and a solid precipitated. The acidic solution was basified to pH 10 with 4% w/v sodium carbonate solution and the mixture was sonicated to finely divide the solid, which was collected by filtration, washed twice with water and dried in a stream of air for 1 h to give 160 mg (0.53 mmol, 100%) of the desired product as a tan solid.
¹H NMR (200 MHz, DMSO*-d*₆) δ ppm 8.52 (1 H, br. s.), 8.32 (1 H, s), 8.09 - 8.20 (2 H, m), 7.57 (3 H, dd, *J*=4.9, 1.8 Hz), 3.69 (3 H, s), 3.38 - 3.56 (2 H, m), 3.21 (2 H, t, *J*=5.1 Hz).
HPLC/MS (5 min) retention time 2.77 min
LRMS: *m*/*z* 303 (M+1).

### PREPARATION 34

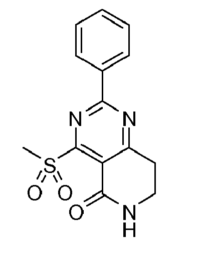

### 4-(Methylsulfonyl)-2-phenyl-7,8-dihydropyrido[4,3-d]pyrimidin-5(6H)-one

The title compound of Preparation 22 (60 mg, 0.15 mmol) was treated with meta-chloroperbenzoic acid (max purity 77%, 0.10 g, 0.45 mmol) according to the method of Preparation 23 to give 84 mg of the crude title compound. Purity 70%. Used as such without further purification.
HPLC/MS (9 min) retention time 5.13 min.
LRMS: **m/z** 304 (M+1)

### PREPARATION 35

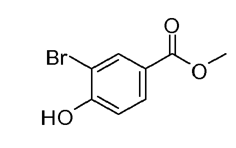

### Methyl 3-bromo-4-hydroxybenzoate

3-Bromo-4-hydroxybenzoic acid (1.50 g, 6.77 mmol) was dissolved in 15 mL of methanol and acetyl chloride (0.59 mL, 8.12 mmol) was added. The mixture was stirred at 60°C for 5 h. The solution was allowed to cool and was evaporated. Ethyl acetate was added to the residue, the organic phase was washed twice with water, brine and dried over magnesium sulphate. Filtration and evaporation gave 1.51 g (6.53 mmol, 93%) of the title compound as a white solid. Purity 96%.
HPLC/MS (9 min) retention time 5.36 min.
LRMS: *m*/*z* 232 (M+1)

### PREPARATION 36

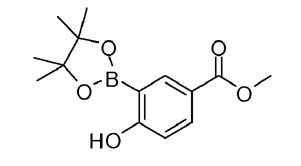

### Methyl 4-hydroxy-3-(4,4,5,5-tetramethyl-1,3,2-dioxaborolan-2-yl)benzoate

The title compound of Preparation 35 (1.51 g, 6.53 mmol) was dissolved in 40 mL of previously degassed dioxane. Bis(pinacolate)diboron (3.32 g, 13.07 mmol), (diphenylphosphino)ferrocene (0.18 g, 0.32 mmol) and potassium acetate (1.92 g, 19.56 mmol) were added and the mixture was subjected to three cycles of evacuation-backfilling with argon. 1,1'-Bis(diphenylphosphino)ferrocene-palladium(II) dichloride dichloromethane complex (0.27 g, 0.33 mmol) was added and the mixture was subjected again to three cycles of evacuation-backfilling with argon and heated at 80°C for 18 h. The mixture was cooled to room temperature, water and ethyl acetate were added, the organic layer was separated and washed with brine. The aqueous layer was extracted with ethyl acetate (x2) and the combined organic layers were dried with magnesium sulphate, filtered and evaporated. After purification by column chromatography using the SP1 Purification system (ethyl acetate-hexane gradient), 1.07 g (3.75 mmol, 57%) of the title compound were obtained as a white solid.
¹H NMR (200 MHz, CHLOROFORM*-d*) δ ppm 8.33 (1 H, d, *J*=2.3 Hz), 8.23 (1 H, s), 8.06 (1 H, dd, *J*=8.6, 2.3 Hz), 6.91 (1 H, d, *J*=8.6 Hz), 3.88 (3 H, s), 1.39 (12 H, s).
HPLC/MS (9 min) retention time 4.67 min.
LRMS: *m*/*z* 279 (M+1).

### PREPARATION 37

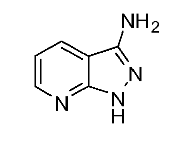

### 1H-Pyrazolo[3,4-b]pyridin-3-amine

2-Chloronicotinonitrile (0.75 g, 5.30 mmol), cupper iodide (I) (50 mg, 0.05 mmol), cesium carbonate (2.59 g, 7.96 mmol) and 1,10-phenanthroline (96 mg, 0.53 mmol) were suspended in 20 mL of dimethylformamide. Hydrazine hydrate (1.6 mL, 31.83 mmol) was added and the mixture was stirred at 60°C overnight. The mixture was cooled to room temperature, water was added and the aqueous phase was extracted with dichloromethane (x3). The combined organic layers were dried with magnesium sulphate, filtered and evaporated. The crude was purified by column chromatography using the SP1 Purification system (methanol-dichloromethane gradient) to yield 0.34 g (2.51 mmol, 47%) of the title compound as a yellow solid. Purity:100%.
¹H NMR (200 MHz, DMSO*-d*₆) δ ppm 11.91 (1 H, br. s.), 8.33 (1 H, dd, *J*=4.3, 1.6 Hz), 8.10 (1 H, dd, *J*=7.8, 1.6 Hz), 6.94 (1 H, dd, *J*=7.8, 4.7 Hz), 5.55 (2 H, s).
LRMS: *m*/*z* 135 (M+1).

### PREPARATION 38

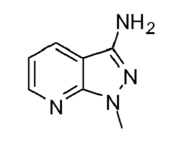

### 1-Methyl-1H-pyrazolo[3,4-b]pyridin-3-amine

2-Chloronicotinonitrile (4.00 g, 28.87 mmol), cupper iodide (I) (0.3 g, 1.58 mmol), cesium carbonate (14.00 g, 42.97 mmol) and 1,10-phenanthroline (0.50 g, 2.77 mmol) and methylhyrazine (10 mL, 198 mmol) were treated according to the method of Preparation 37. Purification by reverse-phase chromatography using the SP1 Purification system gave 1.90 g (12.69 mmol, 44%) of the title compound. Purity: 99%.
¹H NMR (200 MHz, CHLOROFORM*-d*) δ ppm 8.48 (1 H, dd, *J*=4.7, 1.6 Hz), 7.88 (1 H, dd, *J*=8.2, 1.6 Hz), 6.85 - 7.02 (1 H, m), 3.95 (3 H, s).
HPLC/MS (9 min) retention time 3.35 min.
LRMS: *m*/*z* 149 (M+1).

### PREPARATION 39

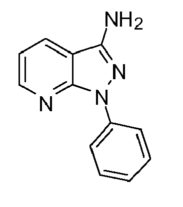

### 1-Phenyl-1H-pyrazolo[3,4-b]pyridin-3-amine

2-Chloronicotinonitrile (3.00 g, 21.65 mmol), cupper iodide (I) (0.20 g, 1.05 mmol), cesium carbonate (11.00 g, 33.76 mmol) and 1,10-phenanthroline (0.40 g, 2.22 mmol) and phenylhyrazine (13 mL, 132 mmol) were treated according to the method of Preparation 37. After purification by reverse-phase chromatography using the SP1 Purification system, the fractions containing the product were concentrated. 5N hydrochloric acid was added and this aqueous phase was extracted with ethyl acetate, dried with magnesium sulphate, filtered and evaporated to give 1.00 g (4.37 mmol, 20%) of the title compound. Purity: 92%.
¹H NMR (200 MHz, CHLOROFORM*-d*) δ ppm 8.58 (1 H, dd, *J*=4.7, 1.6 Hz), 8.22 (2 H, d, *J*=8.6 Hz), 7.95 (1 H, dd, *J*=7.8, 1.6 Hz), 7.47 (2 H, m), 7.16 - 7.30 (1 H, m), 7.10 (1 H, dd, *J*=7.8, 4.7 Hz), 4.33 (2 H, br. s.).
HPLC retention time (9 min) 5.24 min.
LRMS 211 (M+1)

### PREPARATION 40

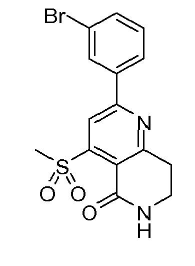

### 2-(3-Bromophenyl)-4-(methylsulfonyl)-7,8-dihydro-1,6-naphthyridin-5(6H)-one

The title compound of Preparation 24 (13.10 g, 27.21 mmol) was treated with trifluoroacetic acid (50 mL, 114 mmol) according to the method of Preparation 33, to give 10.00 g (26.2 mmol, 96%) of the title compound as a solid. Purity: 97%.
HPLC retention time (9 min) 3.13 min.
LRMS 381,383 (M+1)

### PREPARATION 41

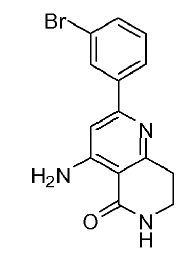

### 4-Amino-2-(3-bromophenyl)-7,8-dihydro-1,6-naphthyridin-5(6H)-one

The title compound of Preparation 40 (1.00 g, 2.62 mmol) was suspended in 2 mL of 7N ammonia in methanol. The mixture was heated at 140 °C in the microwave for 4 hours. The solution was allowed to cool and was evaporated. The resulting suspension was filtered, the solid was washed with diethyl ether and dried to give 0.77 g (2.32 mmol, 88%) of the title compound as a solid. Purity: 96%.
HPLC/MS (9 min) retention time 3.66 min.
LRMS: *m*/*z* 318/320 (M+1).

### PREPARATION 42

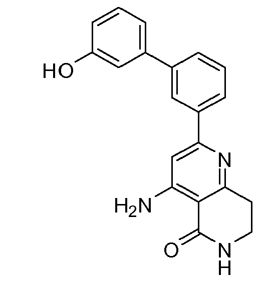

### 4-Amino-2-(3'-hydroxybiphenyl-3-yl)-7,8-dihydro-1,6-naphthyridin-5(6H)-one

The title compound of Preparation 41 (0.15 g, 0.45 mmol), 3-hydroxyphenylboronic acid (94 mg, 0.68 mmol) and cesium carbonate (0.68 g, 1.36 mmol) were suspended in 4 mL of degassed dioxane in a sealed tube. The mixture was subjected to three cycles of evacuation-backfilling with argon, 1'-bis(diphenylphosphino)ferrocene-palladium(II) dichloride dichloromethane complex (18 mg, 0.02 mmol) was added and the mixture was subjected again to three cycles of evacuation-backfilling with argon and heated at 90°C for 3 h. The mixture was allowed to cool to room temperature and the solvent was evaporated. The crude was purified by column chromatography using the SP1 Purification system (ethyl acetate-hexane gradient) to yield 102 mg (0.31 mmol, 68%) of the title compound. Purity: 100%.
HPLC/MS (9 min) retention time 4.06 min.
LRMS: *m*/*z* 332 (M+1).

### PREPARATION 43

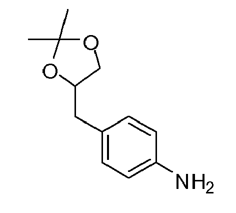

### {4-[(2,2-Dimethyl-1,3-dioxolan-4-yl)methyl]phenyl}amine

2,2-Dimethyl-4-(4-nitrobenzyl)-1,3-dioxolane (1.15 g, 4.85 mmol) was dissolved in 50 mL of ethanol and 10% Pd/C (0.26 g, 0.24 mmol) was added. The mixture was stirred under nitrogen atmosphere at 20 psi for 2 hours. The catalyst was filtered and the solvent was evaporated to yield 965 mg (4.51 mmol, 96%) of the title compound as an orange solid. Purity: 100%.
¹H NMR (200 MHz, CHLOROFORM*-d*) δ ppm 7.02 (2 H, d, *J*=8.4 Hz), 6.68 (2 H, d, *J*=8.4 Hz), 4.14 - 4.40 (1 H, m), 3.95 (1 H, dd, *J*=8.2, 5.9 Hz), 3.54 - 3.73 (1 H, m), 2.86 - 3.06 (1 H, m), 2.50 - 2.78 (1 H, m), 1.44 (3 H, s), 1.36 (3 H, s).
HPLC/MS (9 min) retention time 6.63 min.
LRMS: *m*/*z* 208 (M+1).

### PREPARATION 44

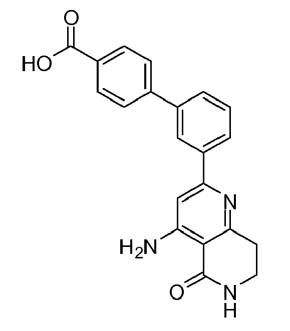

### 3'-(4-Amino-5-oxo-5,6,7,8-tetrahydro-1,6-naphthyridin-2-yl)biphenyl-4-carboxylic acid

The title compound of Preparation 41 (150 mg, 0.46 mmol), 4-carboxybenzene boronic acid (115 mg, 0.69 mmol), cesium carbonate (0.70 g, 1.39 mmol) and 1'-bis(diphenylphosphino)ferrocene-palladium(II) dichloride dichloromethane complex (19 mg, 0.02 mmol) were treated according to the method of Preparation 42. Purification by column chromatography using the SP1 purification system (ethyl acetate-hexane-methanol gradient) allowed to yield 102 mg (0.28 mmol, 61%) of the title compound as an off-white solid.
HPLC/MS (9 min) retention time 4.10 min.
LRMS: *m*/*z* 360 (M+1).

### PREPARATION 45

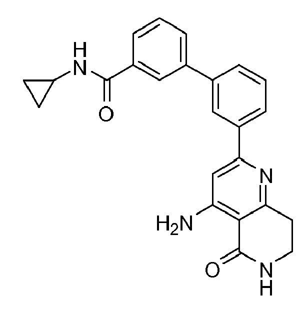

### 3'-(4-Amino-5-oxo-5,6,7,8-tetrahydro-1,6-naphthyridin-2-yl)-N-cyclopropylbiphenyl-3-carboxamide

The title compound of Preparation 41 (700 mg, 2.20 mmol), 3-(cyclopropylcarbamoyl)phenylboronic acid (676 mg, 3.30 mmol), 2M cesium carbonate (3.30 mL, 6.60 mmol) and 1'-bis(diphenylphosphino)ferrocene-palladium(II) dichloride dichloromethane complex (90 mg, 0.11 mmol) were treated according to the method of Preparation 42 to give 0.83 g (2.08 mmol, 95%) of the title compound. Purity: 100%.
¹H NMR (200 MHz, CHLOROFORM*-d*) δ ppm 8.17 (1 H, t, *J*=1.6 Hz), 8.05 (1 H, t, *J*=1.6 Hz), 7.93 (1 H, dt, *J*=7.5, 1.5 Hz), 7.72 - 7.82 (2 H, m), 7.67 (2 H, m), 7.54 (2 H, dd, *J*=7.6, 5.3 Hz), 6.89 (2 H, s), 6.38 (1 H, br. s.), 5.71 (1 H, br. s.), 3.60 (2 H, td, *J*=6.8, 2.7 Hz), 3.15 (2 H, t, *J*=6.8 Hz), 2.82 - 3.04 (1 H, m), 0.82 - 1.01 (2 H,m), 0.67 (2H, m).
HPLC/MS (15 min) retention time 4.42 min.
LRMS: *m*/*z* 399 (M+1).

### PREPARATION 46

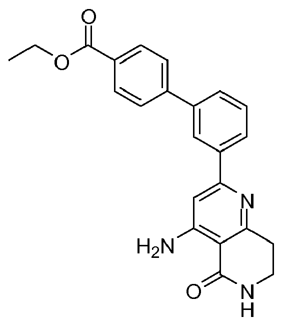

### Ethyl 3'-(4-amino-5-oxo-5,6,7,8-tetrahydro-1,6-naphthyridin-2-yl)biphenyl-4-carboxylate

The title compound of Preparation 41 (326 mg, 1.03 mmol), 4-(ethoxycarbonyl)phenylboronic acid (299 mg, 1.54 mmol), 2M cesium carbonate (1.54 mL, 3.08 mmol) and 1'-bis(diphenylphosphino)ferrocene-palladium(II) dichloride dichloromethane complex (42 mg, 0.05 mmol) were treated according to the method of Preparation 42 to give 165 mg (0.42 mmol, 41%) of the title compound as an orange solid. Purity: 100%.
¹H NMR (200 MHz, CHLOROFORM*-d*) δ ppm 8.21 (1 H, t, *J*=1.6 Hz), 8.14 - 8.17 (1 H, m), 8.10 - 8.14 (1 H, m), 7.94 (1 H, dt, *J*=7.8, 1.6 Hz), 7.73 - 7.78 (1 H, m), 7.68 - 7.74 (1 H, m), 7.66 (1 H, d, *J*=1.6 Hz), 7.57 (1 H, d, *J*=7.8 Hz), 6.88 (1 H, s), 5.72 (1 H, br. s.), 4.42 (2 H, q, *J*=7.2 Hz), 3.61 (2 H, td, *J*=6.8, 2.7 Hz), 3.15 (2 H, t, *J*=6.8 Hz), 1.43 (3 H, t, *J*=7.0 Hz).
HPLC/MS (9 min) retention time 4.72 min.
LRMS: *m*/*z* 388 (M+1).

### PREPARATION 47

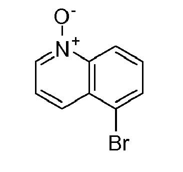

### 5-Bromoquinoline 1-oxide

5-Bromoquinoline (0.50 g, 2.40 mmol) was treated with meta-chloroperbenzoic acid (max purity 77%, 0.88 g, 3.60 mmol) according to the method of Preparation 23, to give 0.48 g (0.21 mmol, 86%) of the title compound as a brown solid. Purity: 98%.
¹H NMR (200 MHz, CHLOROFORM*-d*) δ ppm 8.76 (1 H, d, *J*=8.6 Hz), 8.58 (1 H, d, *J*=6.2 Hz), 8.11 (1 H, d, *J*=9.0 Hz), 7.93 (1 H, d, *J*=7.4 Hz), 7.60 (1 H, t, *J*=8.2 Hz), 7.40 (1 H, dd, *J*=8.8, 6.1 Hz).
HPLC/MS (9 min) retention time 4.55 min.
LRMS: *m*/*z* 226 (M+1).

### PREPARATION 48

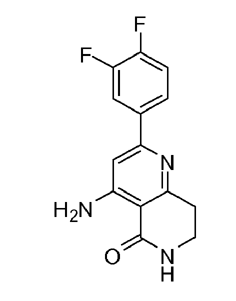

### 4-Amino-2-(3,4-difluorophenyl)-7,8-dihydro-1,6-naphthyridin-5(6H)-one

The title compound of Preparation 28 (220 mg, 0.50 mmol) was suspended in 4 mL of 7N NH3 in methanol. The mixture was at 100 °C in the microwave for 5 hours. The mixture was evaporated in a vortex of nitrogen and the residue was suspenden in 4 mL of ethanol. The mixture was heated in the microwave at 170°C for 30 minutes, then evaporated and taken up in 4% w/v sodium carbonate solution. The aqueous phase was extracted three times with dichloromethane, the combined organic layers were washed with brine, dried over sodium sulphate and evaporated. The crude was purified by flash chromatography (ethyl acetate) to give 93 mg (0.34 mmol, 67%) of the title compound as an off-white solid.
¹H NMR (200 MHz, CHLOROFORM*-d*) δ ppm 7.83 (1 H, ddd, *J*=11.0, 8.0, 2.1 Hz), 7.59 - 7.74 (1 H, m), 7.22 (1 H, dt, *J*=9.6, 8.3 Hz), 6.75 (1 H, s), 5.74 (1 H, br. s.), 3.59 (2 H, td, *J*=6.7, 2.5 Hz), 3.11 (2 H, t, *J*=6.6 Hz)
HPLC/MS (5 min) retention time 1.76 min.
LRMS: *m*/*z* 276 (M+1)

### PREPARATION 49

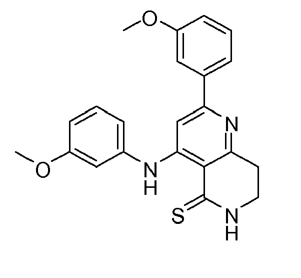

### 2-(3-Methoxyphenyl)-4-[(3-methoxyphenyl)amino]-7,8-dihydro-1,6-naphthyridine-5(6H)-thione

The title compound of Example 8 (55 mg, 0.15 mmol) was disolved in 2 mL of warm dioxane. Diisopropylethylamine (0.20 mL, 1.15 mmol) and diphosphorous pentasulfide (110 mg, 0.34 mmol) were added and the mixture was stirred at 100°C overnight, then cooled to room temperature. The suspension was dissolved in dichloromethane and loaded onto a plug of silica. The plug was flushed through with ethyl acetate, giving a yellow filtrate. The filtrate was evaporated and purified by flash chromatography (ethyl acetate/hexanes 50:50) to give 34 mg (0.08 mmol, 57%) of the title compound. Purity: 97%.
¹H NMR (200 MHz, CHLOROFORM*-d*) δ ppm 12.09 (1 H, s), 7.99 (1 H, br. s.), 7.48 (1 H, dd, *J*=2.4, 1.4 Hz), 7.34 - 7.44 (3 H, m), 7.30 (1 H, dd, *J*=8.0, 2.8 Hz), 6.88 - 7.00 (2 H, m), 6.85 (1 H, t, *J*=2.1 Hz), 6.78 (1 H, dd, *J*=7.9, 2.3 Hz), 3.85 (3 H, s), 3.82 (3 H, s), 3.61 (2 H, td, *J*=6.8, 3.5 Hz), 3.18 (2 H, t, *J*=6.8 Hz).
HPLC/MS (5 min) retention time 3.00 min.
LRMS: *m*/*z* 392 (M+1).

### PREPARATION 50

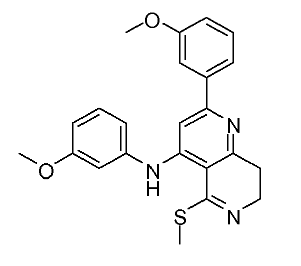

### N-2-bis(3-Methoxyphenyl)-5-(methylthio)-7,8-dihydro-1,6-naphthyridin-4-amine

Sodium hydride (60% dispersion in oil, 3.5 mg, 0.09 mmol) was suspended in 0.5 mL of dry tetrahydrofuran. A solution of the title compound of Preparation 49 (27 mg, 0.069 mmol) in 0.5 mL of tetrahydrofuran was added dropwise while stirring. The mixture was stirred at 70°C for 30 minutes and was then cooled to room temperature. A solution of methyl iodide (13.5 mg, 0.095 mmol) dissolved in 0.2 mL of dry tetrahydrofuran was added and the mixture was stirred at 70°C for 2 hours. The mixture was evaporated and the residue was partitioned between dichloromethane and water. The organic layer was separated, washed with brine and dried over sodium sulphate. Evaporation gave 24 mg (0.056 mmol, 82%) of the title compound. Purity: 95%.
¹H NMR (200 MHz, CHLOROFORM*-d*) δ ppm 7.60 (1 H, br. s.), 7.08 - 7.44 (5 H, m), 6.57 - 6.91 (4 H, m), 3.75 (3 H, s), 3.72 (3 H, s), 3.68 (2 H, br. s.), 2.79 (2 H, t, J=6.8 Hz), 2.43 (3 H, s).
HPLC/MS (5 min) retention time 2.76 min.
LRMS: *m*/*z* 406 (M+1).

### PREPARATION 51

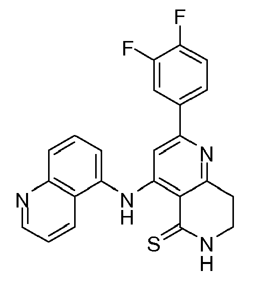

### 2-(3,4-Difluorophenyl)-4-(quinolin-5-ylamino)-7,8-dihydro-1,6-naphthyridine-5(6H)-thione

The title compound of Example 52 (37 mg, 0.09 mmol) was treated with diisopropylethylamine (0.15 mL, 0.86 mmol) and diphosphorous pentasulfide (160 mg, 0.36 mmol) according to the method of Preparation 49 to give 39 mg (0.09 mmol, 99%) of the title compound as a yellow solid. Purity: 99%.
¹H NMR (200 MHz, CHLOROFORM*-d*) δ ppm 12.45 (1 H, s), 8.99 (1 H, dd, *J*=4.0, 1.3 Hz), 8.52 (1 H, d, *J*=8.6 Hz), 8.11 (1 H, d, *J*=8.2 Hz), 7.63 - 7.91 (3 H, m), 7.60 (1 H, d, *J*=7.4 Hz), 7.34 - 7.51 (2 H, m), 7.11 (1 H, q, *J*=8.8 Hz), 6.99 (1 H, s), 3.70 (2 H, dt, *J*=6.6, 3.3 Hz), 3.22 (2 H, t, *J*=6.8 Hz).
HPLC/MS (5 min) retention time 3.40 min.
LRMS: *m*/*z* 419 (M+1).

### PREPARATION 52

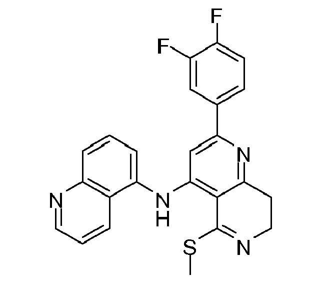

### 2-(3,4-Difluorophenyl)-5-(methylthio)-N-quinolin-5-yl-7,8-dihydro-1,6-naphthyridin-4-amine

The title compound of Preparation 51 (39 mg, 0.09 mmol) was treated with sodium hydride (60% discpersion in oil, 5.1 mg, 0.13 mmol) and methyl iodide (14 mg, 0.10 mmol) according to the method of Preparation 50 to give 35 mg (0.076 mmol, 82%) of the title compound as a yellow solid. Purity: 95%.
¹H NMR (200 MHz, CHLOROFORM*-d*) δ ppm 9.00 (1 H, br. s.), 8.41 (1 H, d, *J*=8.6 Hz), 8.02 - 8.25 (2 H, m), 7.80 (1 H, t, *J*=8.0 Hz), 7.29 - 7.71 (4 H, m), 7.07 (1 H, q, *J*=8.8 Hz), 6.71 (1 H, s), 3.84 (2 H, t, *J*=6.0 Hz), 2.91 (2 H, t, *J*=6.0 Hz), 2.59 (3 H, s).
HPLC/MS (5 min) retention time 2.77 min.
LRMS: *m*/*z* 433 (M+1).

### PREPARATION 53

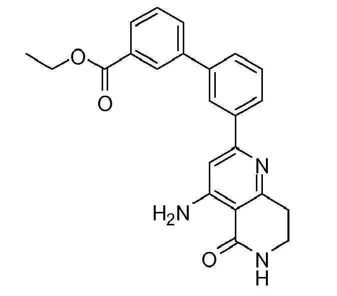

### Ethyl 3'-(4-amino-5-oxo-5,6,7,8-tetrahydro-1,6-naphthyridin-2-yl)biphenyl-3-carboxylate

The title compound of Preparation 41 (0.80 g, 2.51 mmol) was treated with 3-(ethoxycarbonyl)phenylboronic acid (0.75 g, 3.77 mmol), 2M cesium carbonate (3.8 mL, 7.6 mmol) and 1'-bis(diphenylphosphino)ferrocene-palladium(II) dichloride dichloromethane complex (102 mg, 0.13 mmol) according to the method of Preparation 42 to give 0.77 g (2.00 mmol, 79%) of the title compound as a white solid. Purity: 100%.
¹H NMR (200 MHz, DMSO-d6) δ ppm 8.20 - 8.26 (m, 2 H), 7.95 - 8.05 (m, 3 H), 7.55 - 7.83 (m, 4 H), 7.16 (s, 1 H), 4.37 (q, J=7.16 Hz, 2 H), 3.36 - 3.45 (m, 2 H), 2.93 (t, J=6.64 Hz, 2 H), 1.36 (t, J=7.22 Hz, 3 H).
HPLC/MS (9 min) retention time 4.69 min.
LRMS: *m*/*z* 388 (M+1).

### PREPARATION 54

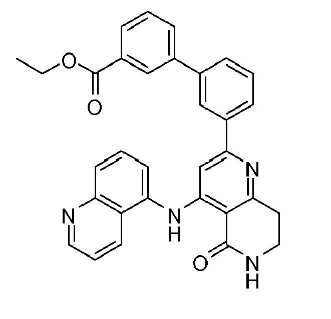

### Ethyl 3'-[5-oxo-4-(quinolin-5-ylamino)-5,6,7,8-tetrahydro-1,6-naphthyridin-2-yl]biphenyl-3-carboxylate

The title compound of Preparation 53 (0.77 g, 2.00 mmol) was dissolved in 20 mL of dioxane in a sealed tube. The mixture was subjected to three cycles of evacuation-backfilling with argon and 5-bromoquinoline (0.51 g, 2.39 mmol), cesium carbonate (0.91 g, 2.79 mmol), bis(diphenylphosphino)-9,9-dimethylxanthene (0.24 g, 0.40 mmol) and tris(dibenzylidene acetone)dipalladium (0) (0.18 g, 0.20 mmol) were added. The mixture was subjected again to three cycles of evacuation-backfilling and the mixture was stirred at 120°C under argon atmosphere overnight. The solvent was evaporated and dichloromethane and water were added. The organic layer was washed with water (x2), dried with magnesium sulphate, filtered and evaporated to yield 0.36 g (0.70 mmol, 36%) as a solid.
HPLC/MS (9 min) retention time 5.93 min.
LRMS: *m*/*z* 515 (M+1).

### PREPARATION 55

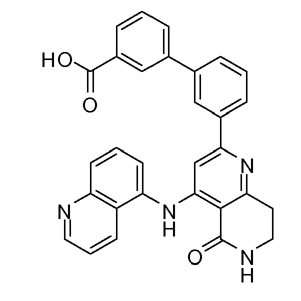

### 3'-[5-Oxo-4-(quinolin-5-ylamino)-5,6,7,8-tetrahydro-1,6-naphthyridin-2-yl]biphenyl-3-carboxylic acid

The title compound of Preparation 54 (0.86 g, 1.68 mmol) in 15 mL of tetrahydrofuran and 30 mL of ethanol, was treated with lithium hydroxide (0.35 g, 8.40 mmol) and the mixture was stirred at 50°C for 4 hours. The solvent was evaporated, water was added to the residue and the solution was neutralized with 2N hydrochloric acid. The resulting yellow precipitate was filtered, washed with water and dried to give 0.82 g (1.67 mmol, 100%) of the title compound as a deep yellow solid. Purity: 100%.
¹H NMR (200 MHz, DMSO-d6) δ ppm 12.14 (s, 1 H), 9.03 (d, *J*=3.90 Hz, 1 H), 8.64 (s, 1 H), 8.22 (s, 1 H), 7.80 - 8.15 (m, 7 H), 7.54 - 7.76 (m, 4 H), 7.11 (s, 1 H), 3.56 - 3.69 (m, 2 H), 3.25 - 3.33 (m, 2 H).
HPLC/MS (9 min) retention time 4.97 min.
LRMS: *m*/*z* 487 (M+1).

### PREPARATION 56

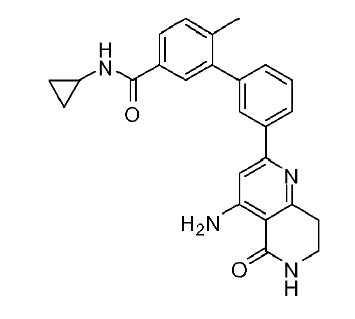

### 3'-(4-Amino-5-oxo-5,6,7,8-tetrahydro-1,6-naphthyridin-2-yl)-N-cyclopropyl-6-methylbiphenyl-3-carboxamide

The title compound of Preparation 41 (180 mg, 0.57 mmol) was treated with N-cyclopropyl-4-methyl-3-(4,4,5,5-tetramethyl-1,3,2-dioxaborolan-2-yl)benzamide (220 mg, 0.73 mmol), 2 M cesium carbonate (0.85 mL, 1.70 mmol) and 1,1'-bis(diphenylphosphino)ferrocene-palladium(II) dichloride dichloromethane complex (23 mg, 0.03 mmol) according to the method of Preparation 42 to give 176 mg (0.42 mmol, 75%) of the title compound as a green oil. Purity: 100%.
¹H NMR (200 MHz, CHLOROFORM*-d*) δ ppm 7.85 - 7.93 (m, *J*=7.8 Hz, 1 H), 7.82 (s, 1 H), 7.60 - 7.71 (m, 2 H), 7.46 (t, *J*=7.6 Hz, 1 H), 7.25 - 7.35 (m, 2 H), 6.82 (s, 1 H), 6.66 (d, *J*=2.3 Hz, 1 H), 6.01 (s, 1 H), 3.49 - 3.60 (m, 2 H), 3.08 (t, *J*=6.8 Hz, 2 H), 2.82 - 2.96 (m, 1 H), 2.28 (s, 3 H), 1.83 - 2.03 (m, *J*=5.9 Hz, 2 H), 0.80 - 0.86 (m, 2 H), 0.57 - 0.63 (m, 2 H).
HPLC/MS (9 min) retention time 4.17 min.
LRMS: *m*/*z* 413 (M+1).

### PREPARATION 57

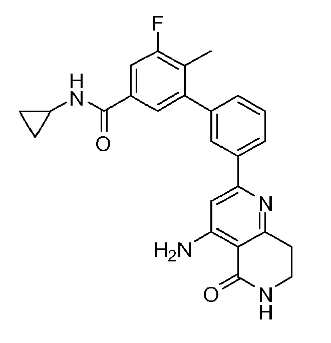

### 3'-(4-Amino-5-oxo-5,6,7,8-tetrahydro-1,6-naphthyridin-2-yl)-N-cyclopropyl-5-fluoro-6-methylbiphenyl-3-carboxamide

The title compound of Preparation 41 (200 mg, 0.63 mmol) was treated with *N-*cyclopropyl-3-fluoro-4-methyl-5-(4,4,5,5-tetramethyl-1,3,2-dioxaborolan-2-yl)benzamide (237 mg, 0.69 mmol), 2M cesium carbonate (0.94 mL, 1.89 mmol) and 1,1'-bis(diphenylphosphino)ferrocene-palladium(II) dichloride dichloromethane complex (26 mg, 0.03 mmol) according to the method of Preparation 42 to give 198 mg (0.46 mmol, 73%) of the title compound. Purity: 98%.
¹H NMR (200 MHz, CHLOROFORM*-d*) δ ppm 7.91 - 7.98 (1 H, m), 7.88 (1 H, t, *J*=1.6 Hz), 7.52 (1 H, s), 7.47 (1 H, d, *J*=3.5 Hz), 7.41 (1 H, d, *J*=2.0 Hz), 7.33 (1 H, dd, *J*=8.6, 2.0 Hz), 6.85 (1 H, s), 6.32 (1 H, br. s.), 5.68 (1 H, br. s.), 3.58 (2 H, dd, *J*=7.0, 2.7 Hz), 3.12 (2 H, t, *J*=6.8 Hz), 2.91 (1 H, td, *J*=7.0, 3.5 Hz), 2.22 (3 H, d, *J*=2.7 Hz), 2.05 (2 H, s), 0.88 (2 H, dd, *J*=7.0, 2.0 Hz), 0.62 (2 H, ddd, *J*=3.7, 1.8, 1.6 Hz).
HPLC/MS (15 min) retention time 4.99 min.
LRMS: *m*/*z* 431 (M+1).

### EXAMPLE 1

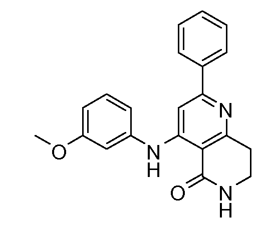

### 4-[(3-Methoxyphenyl)amino]-2-phenyl-7,8-dihydro-1,6-naphthyridin-5(6H)-one

The title compound of Preparation 23 (50 mg, 0.124 mmol) and 3-methoxyaniline (74 mg, 0.60 mmol) were suspended in 1 mL ethanol and agitated at 70 °C overnight in a capped vial. The mixture was allowed to cool and was was partitioned between saturated sodium carbonate solution and ether. The aqueous was extracted twice with ether and the combined organics were washed with brine and dried over sodium sulphate, filtered and evaporated to give a residue. The residue was purified by column chromatography (ethyl acetate-hexane, 50:50) to give a crude sample of *tert*-butyl 4-[(3-methoxyphenyl)amino]-5-oxo-2-phenyl-7,8-dihydro-1,6-naphthyridine-6(5H)-carboxylate.

This was dissolved in 0.3 mL dichloromethane and 0.5 mL trifluoroacetic acid was added. After 10 min at room temperature, the mixture was reduced in volume in a stream of nitrogen and was partitioned between saturated sodium carbonate solution and ether. The aqueous was extracted twice with ether. The combined organics were washed with brine and dried over sodium sulphate. Filtration and evaporation gave a residue which was purified by column chromatography (ethyl acetate-hexane, gradient) to give 26 mg (0.075 mmol, 60%) of the title compound as a pale yellow solid. Purity 99%.
¹H NMR (400 MHz, CHLOROFORM*-d*) δ ppm 10.77 (1 H, s), 7.87 (2 H, d, *J*=7.5 Hz), 7.38 - 7.46 (3 H, m), 7.36 (1 H, s), 7.31 (1 H, t, *J*=8.2 Hz), 6.92 (1 H, d, *J*=7.8 Hz), 6.84 (1 H, s), 6.76 (1 H, dd, *J*=8.2, 2.2 Hz), 5.80 (1 H, br. s.), 3.82 (3 H, s), 3.64 (2 H, td, *J*=6.7, 2.7 Hz), 3.18 (2 H, t, *J*=6.7 Hz).
HPLC/MS (30 min) retention time 10.62 min.
LRMS: *m*/*z* 345 (M+).

### EXAMPLE 2

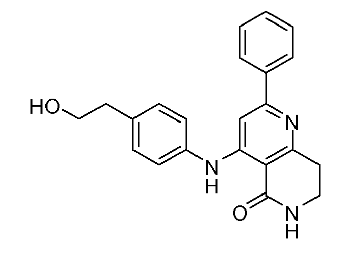

### 4-{[4-(2-Hydroxyethyl)phenyl]amino}-2-phenyl-7,8-dihydro-1,6-naphthyridin-5(6H)-one

The title compound of Preparation 23 (58 mg, 0.14 mmol) and 2-(4-aminophenyl)ethanol (99 mg, 0.72 mmol) were suspended in 1 mL ethanol and agitated at 70 °C overnight in a capped vial. The mixture was allowed to cool and was was partitioned between saturated sodium carbonate solution and ether. The aqueous was extracted twice with ether and the combined organics were washed with brine and dried over sodium sulphate, filtered and evaporated to give a residue. The residue was purified by column chromatography (ethyl acetate-hexane, 50:50) to give 37 mg of *tert*-butyl 4-{[4-(2-hydroxyethyl)phenyl]amino}-5-oxo-2-phenyl-7,8-dihydro-1,6-naphthyridine-6(5H)-carboxylate.

The intermediate was dissolved in 1 mL ethanol and was heated at 160 °C in the microwave for 30 min. The solution was allowed to cool and was evaporated to give 26 mg (0.073 mmol, 51 %) of the title compound as a pale yellow solid. Purity 100%.
¹H NMR (200 MHz, CHLOROFORM*-d*) δ ppm 10.79 (1 H, br. s.), 7.80 - 7.93 (2 H, m), 7.34 - 7.51 (4 H, m), 7.27 (4 H, s), 5.87 (1 H, br. s.), 3.91 (2 H, t, *J*=6.4 Hz), 3.65 (1 H, td, *J*=6.0, 2.0 Hz), 3.21 (2 H, t, *J*=6.6 Hz), 2.91 (2 H, t, *J*=6.4 Hz).
HPLC/MS (30 min) retention time 8.78 min.
LRMS: *m*/*z* 359 (M+).

### EXAMPLE 3

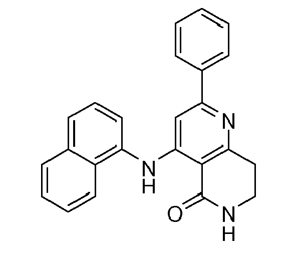

### 4-(1-Naphthylamino)-2-phenyl-7,8-dihydro-1,6-naphthyridin-5(6H)-one

The title compound of Preparation 23 (51 mg, 0.13 mmol) and 1-aminonaphthalene (49 mg, 0.34 mmol) were suspended in 1 mL ethanol and were heated at 160 °C (maximum pressure 12 bar) for 10 h in the microwave. The mixture was allowed to cool and was columned (ethyl acetate-hexane, 50:50) to give 24.6 mg of a pale pink solid upon evaporation. The solid was dissolved in ethyl acetate and was washed three times with 4% w/v sodium carbonate solution, once with brine and was dried over sodium sulphate. Filtration and evaporation gave 18 mg of the title compound (0.046 mmol, 37% yield) as a pale yellow solid. Purity 94%.
¹H NMR (200 MHz, CHLOROFORM*-d*) δ ppm 11.09 (1 H, br. s.), 8.04 - 8.16 (1 H, m), 7.84 - 7.98 (1 H, m), 7.64 - 7.85 (3 H, m), 7.43 - 7.61 (4 H, m), 7.27 - 7.40 (3 H, m), 7.00 (1 H, s), 6.10 (1 H, br. s.), 3.68 (2 H, td, *J*=6.8, 2.5 Hz), 3.22 (2 H, t, *J*=6.6 Hz).
HPLC/MS (30 min) retention time 12.52 min.
LRMS: *m*/*z* 365 (M+).

### EXAMPLE 4

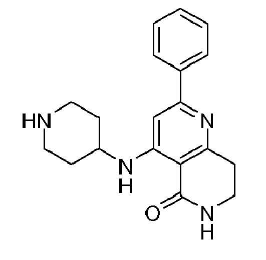

### 2-Phenyl-4-(piperidin-4-ylamino)-7,8-dihydro-1,6-naphthyridin-5(6H)-one

The title compound of Preparation 23 (50 mg, 0.13 mmol) and 4-amino-1-Boc-piperidine (47 mg, 0.23 mmol) were suspended in 1 mL EtOH and were agitated at 70 °C for 3 d. The mixture was evaporated, re-dissolved in ethyl acetate and was filtered through a short column of silica, eluting with ethyl acetate. The combined filtrate was evaporated, re-suspended in 1 mL ethanol and was heated at 160 °C for 30 min in the microwave. The mixture was evaporated and was purified by column chromatography (ethyl acetate-hexane, 50:50) to give a crude sample of *tert*-butyl 5-oxo-2-phenyl-4-(piperidin-4-ylamino)-7,8-dihydro-1,6-naphthyridine-6(5*H*)-carboxylate.

This was dissolved in 0.5 mL dichloromethane and 1 mL trifluoroacetic acid. After 20 min at room temperature, the mixture was diluted with 10 mL 1 N hydrochloric acid. The aqueous was washed three times with ether and was then adjusted to pH 11 sodium carbonate solution and was saturated with salt. The aqueous was extracted three times with ethyl acetate. The combined organics were washed with brine and dried over sodium sulphate. Filtration and evaporation gave 25.5 mg (0.077 mmol, 62%) of the title compound as a pale yellow solid. purity 98%.
¹H NMR (200 MHz, CHLOROFORM*-d*) δ ppm 9.11 (1 H, d, *J*=7.0 Hz), 7.92 (1 H, dd, *J*=7.0, 2.0 Hz), 7.45 (3 H, d, *J*=6.2 Hz), 6.82 (1 H, s), 5.92 (1 H, br. s.), 3.55 (2 H, d, *J*=5.1 Hz), 2.94 - 3.24 (5 H, m), 2.78 (2 H, t, *J*=9.8 Hz), 1.94 - 2.20 (2 H, m), 1.84 (1 H, br. s.), 1.46 - 1.68 (2 H, m).
HPLC/MS (30 min) retention time 7.95 min.
LRMS: *m*/*z* 322 (M+).

### EXAMPLE 5

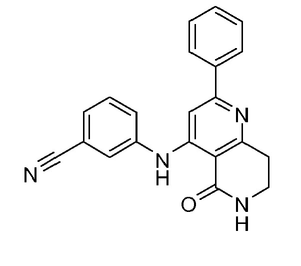

### 3-[(5-Oxo-2-phenyl-5,6,7,8-tetrahydro-1,6-naphthyridin-4-yl)amino]benzonitrile

The title compound of Preparation 23 (50 mg, 0.13 mmol) and 3-cyanoaniline (74 mg, 0.62 mmol) were suspended in 1 mL EtOH and were agitated at 70 °C for 4 d. The mixture was evaporated and the residue re-dissolved in 0.5 mL dichloromethane and 0.5 mL trifluoroacetic acid. The mixture was allowed to stand at room temperature for 4 h and was then evaporated. The residue was diluted wirh saturated potassium carbonate solution and was extracted three times with ethyl acetate. The combined organics were washed with brine and dried over sodium sulphate, filtered and evaporated. The residue was purified by column chromatography (ethyl acetate-hexane, 30:70) to give 11 mg (0.033 mmol, 26%) of the title compound as a pale yellow solid. Purity 100%.
¹H NMR (200 MHz, CHLOROFORM*-d*) δ ppm 11.00 (1 H, s), 7.81 - 7.93 (2 H, m), 7.36 - 7.63 (7 H, m), 7.29 (1 H, s), 5.94 (1 H, br. s.), 3.66 (2 H, td, *J*=6.8, 2.5 Hz), 3.20 (2 H, t, *J*=6.8 Hz).
HPLC/MS (30 min) retention time 10.69 min.
LRMS: *m*/*z* 340 (M+).

### EXAMPLE 6

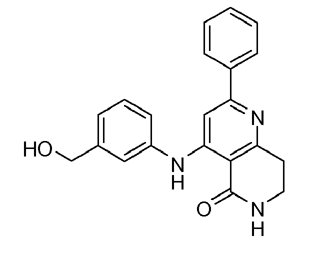

### 4-{[3-(Hydroxymethyl)phenyl]amino}-2-phenyl-7,8-dihydro-1,6-naphthyridin-5(6H)-one

The title compound of Preparation 23 (50 mg, 0.124 mmol) and 3-aminobenzylalcohol (76 mg, 0.62 mmol) were suspended in 1 mL ethanol and agitated at 70 °C overnight in a capped vial. The mixture was then dissolved in 1 mL ethanol and was heated at 160 °C for 30 min in the microwave. The mixture was allowed to cool and was evaporated. The residue was purified by column chromatography (ethyl acetate-hexane, 70:30) to give 17 mg (0.049 mmol, 40%) of the title compound as a pale yellow solid. Purity 100%.
¹H NMR (200 MHz, CHLOROFORM-d) δ ppm 10.75 (1 H, br. s.), 7.71 - 7.86 (2 H, m), 7.33 - 7.47 (4 H, m), 7.28 (4 H, d, *J*=7.0 Hz), 4.69 (2 H, s), 3.61 (2 H, t, *J*=6.8 Hz), 3.15 (2 H, t, *J*=6.8 Hz).
HPLC/MS (30 min) retention time 8.64 min.
LRMS: *m*/*z* 345 (M+).

### EXAMPLE 7

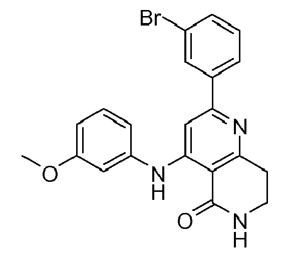

### 2-(3-Bromophenyl)-4-[(3-methoxyphenyl)amino]-7,8-dihydro-1,6-naphthyridin-5(6H)-one

The title compound of Preparation 24 (224 mg, 0.47 mmol) and 3-methoxyaniline (0.30 g mg, 2.45 mmol) were suspended in 10 mL ethanol and were heated at 160 °C (max pressure 12 bar) for 9 h in the microwave. The mixture was evaporated and the residue was purified by column chromatography (ethyl acetate-hexane, gradient) to give 150 mg (0.35 mmol, 76%) of the title compound as a white solid.
¹H NMR (200 MHz, CHLOROFORM-d) δ ppm 10.80 (1 H, s), 8.08 (1 H, s), 7.74 (1 H, d, *J*=7.8 Hz), 7.52 (1 H, d, *J*=8.2 Hz), 7.21 - 7.39 (3 H, m), 6.91 (1 H, d, *J*=8.2 Hz), 6.83 (1 H, s), 6.78 (1 H, d, *J*=8.2 Hz), 5.82 (1 H, br. s.), 3.83 (3 H, s), 3.64 (2 H, td, *J*=6.7, 2.5 Hz), 3.17 (2 H, t).
HPLC/MS (30 min) retention time 15.54 min.
LRMS: *m*/*z* 424,426 (M+1).

### EXAMPLE 8

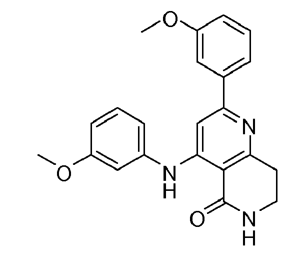

### 2-(3-Methoxyphenyl)-4-[(3-methoxyphenyl)amino]-7,8-dihydro-1,6-naphthyridin-5(6H)-one

The title compound of Preparation 25 (0.55 g, 1.28 mmol) and 3-methoxyaniline (0.30 g mg, 2.45 mmol) were suspended in 20 mL ethanol-tetrahydrofuran (50:50) and were heated at 160 °C (max pressure 13 bar) for 23 h in the microwave. The mixture was evaporated and the residue was purified by column chromatography (ethyl acetate-hexane, gradient) to give 0.246 mg (0.66 mmol, 51%) of the title compound as a pale brown solid. Purity 100%.
¹H NMR (200 MHz, CHLOROFORM-d) δ ppm 10.76 (1 H, s), 7.27 - 7.51 (5H, m), 6.86 - 6.99 (2 H, m), 6.83 (1 H, s), 6.75 (1 H, d, *J*=8.2 Hz), 6.02 (1 H, br. s.), 3.86 (3 H, s), 3.82 (3 H, s), 3.63 (2 H, td, *J*=6.5, 2.5 Hz), 3.17 (2 H, t, *J*=6.8 Hz).
HPLC/MS (30 min) retention time 11.03 min.
LRMS: *m*/*z* 375 (M+).

### EXAMPLE 9

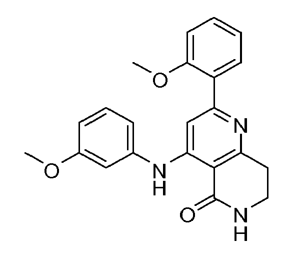

### 2-(2-Methoxyphenyl)-4-[(3-methoxyphenyl)amino]-7,8-dihydro-1,6-naphthyridin-5(6H)-one

The title compound of Preparation 26 (103 mg, 0.24 mmol) and 3-methoxyaniline (0.13 mL, 1.15 mmol) were suspended in 1 mL ethanol-tetrahydrofuran (50:50) and were heated at 160 °C (max pressure 13 bar) for 6 h in the microwave. The mixture was evaporated, the residue taken up in dichloromethane and the organics washed three times with 4% w/v sodium carbonate solution, once with brine and dried over sodium sulphate. Filtration and evaporation gave a residue which was purified by column chromatography (ethyl acetate-hexane, gradient) to give 64 mg (0.17 mmol, 71%) of the title compound as a pale brown solid. Purity 96%.
¹H NMR (200 MHz, CHLOROFORM-d) δ ppm 10.72 (1 H, s), 7.78 (1 H, d, *J*=7.4 Hz), 7.58 (1 H, s), 7.19 - 7.41 (2 H, m), 7.03 (1 H, t, *J*=7.4 Hz), 6.83 - 6.98 (3 H, m), 6.69 (1 H, d, *J*=7.4 Hz), 6.07 (1 H, br. s.), 3.84 (3 H, s), 3.81 (3 H, s), 3.61 (2 H, td, *J*=6.6, 2.5 Hz), 3.15 (2 H, t, *J*=6.6 Hz).
HPLC/MS (30 min) retention time 10.41 min.
LRMS: *m*/*z* 375 (M+).

### EXAMPLE 10

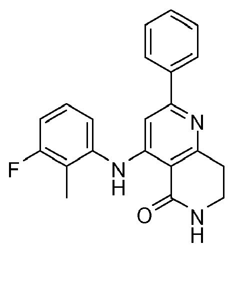

### 4-[(3-Fluoro-2-methylphenyl)amino]-2-phenyl-7,8-dihydro-1,6-naphthyridin-5(6H)-one

The title compound of Preparation 23 (85 mg, 0.20 mmol) and 3-fluoro-2-methylaniline (0.11 mL, 1.00 mmol) were suspended in 1.5 mL ethanol and were heated at 160°C (maximum pressure 12 bar) for 6 h in the microwave. The mixture was allowed to cool, the solvent was evaporated and the residue was purified by chromatography using the SP1 purification system (gradient ethyl acetate-methanol) to give a solid which was triturated with diethyl ether. The solid was filtered, washed with diethyl ether and dried to give 15 mg (0.042 mmol, 21%) of the title compound as a pale yellow solid. Purity: 98%.
¹H NMR (200 MHz, CHLOROFORM-d) δ ppm 10.65 (1 H, s), 7.78 - 7.89 (2 H, m), 7.40 (3 H, dd), 7.21 (1 H, d, *J*=7.8 Hz), 6.88 - 7.05 (2 H, m), 5.79 (1 H, br. s.), 3.66 (2 H, td, *J*=6.7, 2.5 Hz), 3.19 (2 H, t, *J*=6.8 Hz), 2.23 (3 H, d, *J*=2.0 Hz).
HPLC/MS (30 min) retention time 11.54 min.
LRMS: *m*/*z* 347 (M+).

### EXAMPLE 11

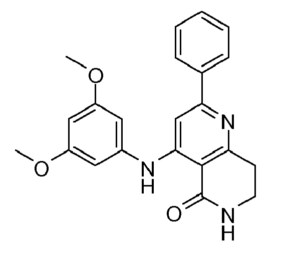

### 4-[(3,5-Dimethoxyphenyl)amino]-2-phenyl-7,8-dihydro-1,6-naphthyridin-5(6H)-one

The title compound of Preparation 23 (80 mg, 0.19 mmol) in ethanol, was treated with 3,5-dimethoxyaniline (145 mg, 0.95 mmol) and the mixture was heated at 70°C overnight and in the microwave at 160°C for 30 min. The solvent was concentrated and the crude was purified by flash chromatography (gradient ethyl acetate-methanol) to give 14 mg (0.035 mmol, 19%) of the title compound. Purity: 99%.
¹H NMR (200 MHz, CHLOROFORM-d) δ ppm 10.75 (1 H, s), 7.80 - 7.94 (2 H, m), 7.33 - 7.51 (4 H, m), 6.47 (2 H, d, *J*=2.0 Hz), 6.32 (1 H, s), 6.00 (1 H, br. s.), 3.80 (6 H, s), 3.63 (2 H, td, *J*=6.5, 2.0 Hz), 3.17 (2 H, t, *J*=6.6 Hz).
HPLC/MS (30 min) retention time 11.07 min.
LRMS: *m*/*z* 375 (M+).

### EXAMPLE 12

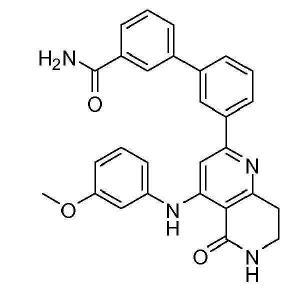

### 3'-{4-[(3-Methoxyphenyl)amino]-5-oxo-5,6,7,8-tetrahydro-1,6-naphthyridin-2-yl}biphenyl-3-carboxamide

The title compound of Example 7 (100 mg, 0.24 mmol) was treated with 3-carbamoylphenylboronic acid (58 mg, 0.35 mmol), 2M cesium carbonate (0.35 mL, 0.71 mmol) and 1,1'-bis(diphenylphosphino)ferrocene-palladium(II) dichloride dichloromethane complex (10 mg, 0.01 mmol) according to the method of Preparation 42. The crude was purified by flash chromatography (ethyl acetate) to give 72 mg (0.15 mmol, 65%) of the title compound as a white solid. Purity: 99%.
¹H NMR (200 MHz, CHLOROFORM-d) δ ppm 10.80 (1 H, s), 8.16 (2 H, s), 7.87 (1 H, d, *J*=7.8 Hz), 7.79 (1 H, t, *J*=8.0 Hz), 7.61 - 7.72 (2 H, m), 7.54 (1 H, t, *J*=7.4 Hz), 7.51 (1 H, t, *J*=7.4 Hz), 7.38 (1 H, s), 7.35 (1 H, d, *J*=8.5 Hz), 6.92 (1 H, d, *J*=7.8 Hz), 6.86 (1 H, t, *J*=2.0 Hz), 6.77 (1 H, dd, *J*=8.2, 2.5 Hz), 3.82 (3 H, s), 3.64 (2 H, t, *J*=6.8 Hz), 3.19 (2 H, t, *J*=6.8 Hz).
HPLC/MS (30 min) retention time 12.31 min.
LRMS: *m*/*z* 464 (M+).

### EXAMPLE 13

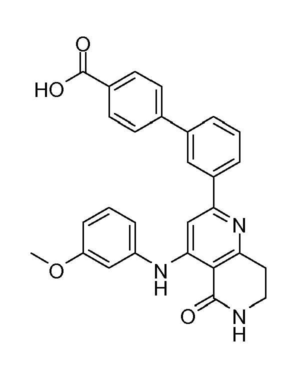

### 3'-{4-[(3-Methoxyphenyl)amino]-5-oxo-5,6,7,8-tetrahydro-1,6-naphthyridin-2-yl}biphenyl-4-carboxylic acid

The title compound of Example 7 (80 mg, 0.19 mmol) was treated with 4-boronobenzoic acid (47 mg, 0.28 mmol), 2M cesium carbonate (0.28 mL, 0.57 mmol) and 1,1'-bis(diphenylphosphino)ferrocene-palladium(II) dichloride dichloromethane complex (8 mg, 0.01 mmol) according to the method of Preparation 42 to give 58 mg (0.12 mmol, 66%) of the title compound as a beige solid. Purity: 100%.
¹H NMR (200 MHz, DMSO-*d*₆) δ ppm 11.13 (1 H, s), 8.23 (2 H, s), 8.03 (2 H, d, *J*=8.2 Hz), 7.89 (1 H, d, *J*=7.8 Hz), 7.67 - 7.83 (3 H, m), 7.58 (1 H, t, *J*=7.8 Hz), 7.50 (1 H, s), 7.35 (1 H, t, *J*=8.0 Hz), 6.89 - 7.01 (2 H, m), 6.77 (1 H, dd, *J*=8.0, 2.0 Hz), 3.79 (3 H, s), 3.51 - 3.67 (2 H, m), 3.03 (2 H, t, *J*=6.4 Hz).
HPLC/MS (30 min) retention time 13.88 min.
LRMS: *m*/*z* 465 (M+).

### EXAMPLE 14

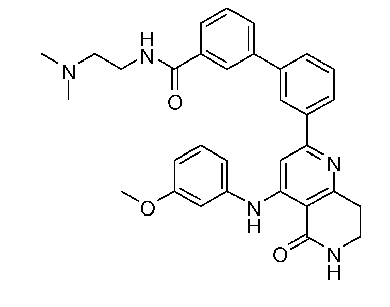

### N-[2-(Dimethylamino)ethyl]-3'-{4-[(3-methoxyphenyl)amino]-5-oxo-5,6,7,8-tetrahydro-1,6-naphthyridin-2-yl}biphenyl-3-carboxamide

The title compound of Example 7 (80 mg, 0.19 mmol) was treated with the title compound of Preparation 31 (90 mg, 0.28 mmol), 2M cesium carbonate (0.28 mL, 0.57 mmol) and 1,1'-bis(diphenylphosphino)ferrocene-palladium(II) dichloride dichloromethane complex (8 mg, 0.01 mmol) according to the method of Preparation 42 to give 82 mg (0.15 mmol, 80%) of the title compound as a brown solid. Purity: 98%.
¹H NMR (200 MHz, CHLOROFORM-d) δ ppm 10.71 (1 H, s), 8.16 (1 H, br. s.), 8.13 (1 H, br. s.), 7.86 (1 H, d, *J*=7.8 Hz), 7.57 - 7.80 (4 H, m), 7.48 (1 H, t, *J*=8.2 Hz), 7.44 (1 H, t, *J*=8.0 Hz), 7.27 - 7.38 (2 H, m), 6.89 (1 H, d, *J*=8.2 Hz), 6.83 (1 H, br. s.), 6.73 (1 H, d, *J*=8.2 Hz), 6.44 (1 H, br. s.), 3.80 (3 H, s), 3.64 - 3.77 (2 H, m), 3.47 - 3.62 (2 H, m), 3.09 (2 H, t, *J*=6.4 Hz), 2.88 (2 H, t, *J*=5.3 Hz), 2.50 (6 H, s).
HPLC/MS (30 min) retention time 8.90 min.
LRMS: *m*/*z* 535 (M+).

### EXAMPLE 15

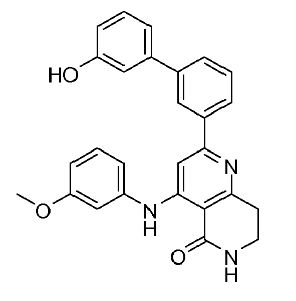

### 2-(3'-Hydroxybiphenyl-3-yl)-4-[(3-methoxyphenyl)amino]-7,8-dihydro-1,6-naphthyridin-5(6H)-one

The title compound of Example 7 (80 mg, 0.19 mmol) was treated with 3-hydroxyphenylboronic acid (39 mg, 0.28 mmol), 2M cesium carbonate solution (0.28 mL, 0.57 mmol) and 1,1'-bis(diphenylphosphino)ferrocene-palladium(II) dichloride dichloromethane complex (8 mg, 0.01 mmol) according to the method of Preparation 42 to give 54 mg (0.12 mmol, 65%) of the title compound as a white solid. Purity: 98%.
¹H NMR (200 MHz, CHLOROFORM-d) δ ppm 10.79 (1 H, s), 8.06 (1 H, s), 7.58 - 7.71 (2 H, m), 7.47 (1 H, t, *J*=7.8 Hz), 7.21 - 7.39 (4 H, m), 7.07 - 7.19 (2 H, m), 6.91 (1 H, d, *J*=7.8 Hz), 6.81 - 6.88 (2 H, m), 6.78 (1 H, dd, *J*=8.2, 2.0 Hz), 3.83 (3 H, s), 3.63 (2 H, t, *J*=6.8 Hz), 3.17 (2 H, t, *J*=6.8 Hz).
HPLC/MS (30 min) retention time 12.40 min.
LRMS: *m*/*z* 437 (M+).

### EXAMPLE 16

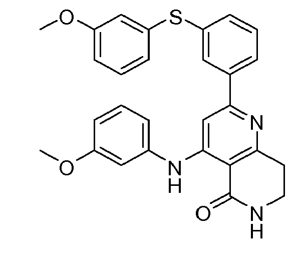

### 4-[(3-Methoxyphenyl)amino]-2-{3-[(3-methoxyphenyl)thio]phenyl}-7,8-dihydro-1,6-naphthyridin-5(6H)-one

The title compound of Example 7 (120 mg, 0.28 mmol) was dissolved in 2 mL of xylene in a sealed tube. 3-Methoxybenzenethiol (0.071 mL, 0.56 mmol) and tributyltin methoxide (167 mg, 0.56 mmol) were added and the mixture was heated at 140°C for 10 min. Tetrakis(triphenylphosphine)palladium (65 mg, 0.06 mmol) was added and the mixture was stirred at 140°C overnight. The mixture was cooled to room temperature and the solvent was evaporated. Ethyl acetate was added and the organic phase was washed with water (x3) and brine (x3). The combined aqueous layers were extracted twice with ethyl acetate and the combined organic layers were dried with magnesium sulphate, filtered and evaporated. The crude was purified by chromatography using the SP1 purification system to give 89 mg (0.18 mmol, 64%) of the title compound as a pale yellow solid. Purity: 98%.
¹H NMR (200 MHz, CHLOROFORM-d) δ ppm 10.77 (1 H, s), 7.93 (1 H, s), 7.69 - 7.80 (1 H, m), 7.24 - 7.40 (4 H, m), 7.19 (1 H, t, *J*=8.0 Hz), 6.84 - 6.96 (3 H, m), 6.69 - 6.84 (3 H, m), 5.82 (1 H, br. s.), 3.81 (3 H, s), 3.74 (3 H, s), 3.63 (2 H, td, *J*=6.7, 2.5 Hz), 3.15 (2 H, t, *J*=6.8 Hz).
HPLC/MS (30 min) retention time 17.40 min.
LRMS: *m*/*z* 483 (M+).

### EXAMPLE17

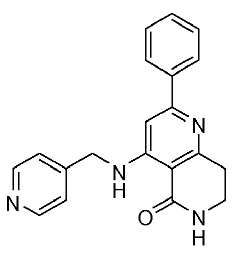

### 2-Phenyl-4-[(pyridin-4-ylmethyl)amino]-7,8-dihydro-1,6-naphthyridin-5(6H)-one

The title compound of Preparation 23 (50 mg, 0.124 mmol) was treated with pyridine-4-ylmethanamine (0.067 mL, 0.66 mmol) following the procedure as described in Example 11 to give 10 mg (0.029 mmol, 23%) of the title compound as a white solid. Purity: 93%.
¹H NMR (partial, 400 MHz, DMSO-*d*₆) δ ppm 9.59 (1 H, t, *J*=6.1 Hz), 8.53 (2 H, d, *J*=4.7 Hz), 7.95 (3 H, d), 7.38 - 7.47 (3 H, m), 7.36 (2 H, d, *J*=5.1 Hz), 6.94 (1 H, s), 4.70 (2 H, d, *J*=6.3 Hz), 2.95 (2 H, t).
HPLC/MS (30 min) retention time 9.36 min.
LRMS: *m*/*z* 330 (M+).

### EXAMPLE 18

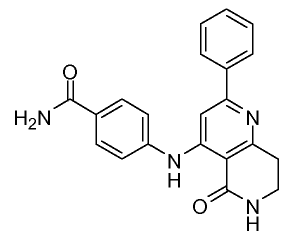

### 4-[(5-Oxo-2-phenyl-5,6,7,8-tetrahydro-1,6-naphthyridin-4-yl)amino]benzamide

The title compound of Preparation 23 (85 mg, 0.20 mmol) in 1.5 mL of ethanol was treated with 4-aminobenzamide (136 mg, 1.00 mmol). The mixture was heated in the microwave at 160°C for 5 h and cooled to room temperature. The resulting precipitate was filtered, washed with ethanol and dried to give 55 mg (0.15 mmol, 75%) of the title compound as a yellow solid. Purity: 98%.
¹H NMR (400 MHz, DMSO-*d*₆) δ ppm 7.95 (1 H, d, *J*=8.6 Hz), 7.78 (1 H, s), 7.63 (2 H, d, *J*=8.6 Hz), 7.43 - 7.49 (2 H, m), 7.37 - 7.42 (2 H, m), 6.66 (2 H, d, *J*=8.6 Hz), 3.60 (2 H, t, *J*=6.8 Hz), 3.13 (2 H, t, *J*=6.8 Hz).
HPLC/MS (30 min) retention time 7.02 min.
LRMS: *m*/*z* 358 (M+).

### EXAMPLE 19

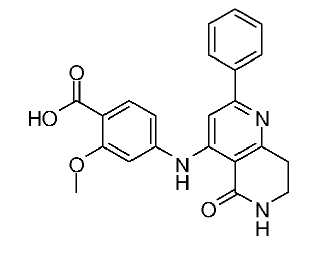

### 2-Methoxy-4-[(5-oxo-2-phenyl-5,6,7,8-tetrahydro-1,6-naphthyridin-4-yl)amino]benzoic acid

The title compound of Preparation 23 (85 mg, 0.20 mmol) was treated with 4-amino-2-methoxybenzoic acid (168 mg, 1.00 mmol) according to the method of Example 18 followed by two purifications using the SP1 purification system (hexane-ethyl acetate) and then (ethyl acetate-methanol) to give 12 mg (0.031 mmol, 16%) of the title compound as a brown solid. Purity: 98%.
¹H NMR (200 MHz, CHLOROFORM-d) δ ppm 10.76 (1 H, s), 7.80 - 7.92 (2 H, m), 7.30 - 7.49 (5 H, m), 6.91 (1 H, d, *J*=7.8 Hz), 6.84 (1 H, s), 6.76 (1 H, dd, *J*=8.2, 2.5 Hz), 5.90 (1 H, br. s.), 3.82 (3 H, s), 3.63 (2 H, td, *J*=6.8, 2.7 Hz), 3.17 (2 H, t, *J*=6.6 Hz).
HPLC/MS (30 min) retention time 10.35 min.
LRMS: *m*/*z* 389 (M+).

### EXAMPLE 20

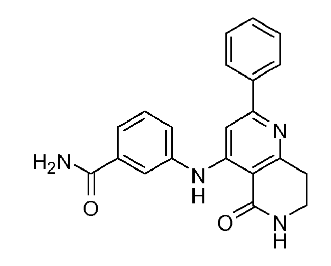

### 3-[(5-Oxo-2-phenyl-5,6,7,8-tetrahydro-1,6-naphthyridin-4-yl)amino]benzamide

The title compound of Preparation 23 (85 mg, 0.20 mmol) was treated with 3-aminobenzamide (148 mg, 1.06 mmol) according to the method of Example 18 followed by purification by reverse phase using the SP1 purification system to give 35 mg (0.09 mmol, 46%). Purity: 98%.
¹H NMR (200 MHz, DMSO-*d*₆) δ ppm 11.21 (1 H, s), 8.23 (1 H, s), 8.06 (1 H, br. s.), 7.88 - 7.98 (2 H, m), 7.83 (1 H, s), 7.67 - 7.75 (1 H, m), 7.50 - 7.57 (2 H, m), 7.41 - 7.50 (4 H, m), 7.36 (1 H, s), 3.03 (4 H, t, *J*=6.8 Hz).
HPLC/MS (30 min) retention time 6.83 min.
LRMS: *m*/*z* 358 (M+).

### EXAMPLE 21

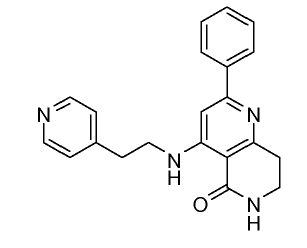

### 2-Phenyl-4-[(2-pyridin-4-ylethyl)amino]-7,8-dihydro-1,6-naphthyridin-5(6H)-one

The title compound of Preparation 23 (80 mg, 0.19 mmol) was treated with 2-(pyridin-4-yl)ethanamine (115 mg, 0.94 mmol) following the procedure as described in Example 18. The crude was purified by reverse phase using the SP1 purification system to give 13 mg (0.003 mmol, 19%) of the title compound. Purity: 97%.
¹H NMR (200 MHz, CHLOROFORM-d) δ ppm 9.09 (1 H, t, *J*=4.7 Hz), 8.56 (2 H, d, *J*=5.5 Hz), 7.93 (2 H, d, *J*=7.8 Hz), 7.37 - 7.58 (3 H, m), 7.21 (2 H, d, *J*=5.5 Hz), 6.81 (1 H, s), 5.85 (1 H, br. s.), 3.45 - 3.69 (4 H, m), 3.11 (2 H, t, *J*=7 Hz), 3.01 (2 H, t, *J*=7.2 Hz).
HPLC/MS (30 min) retention time 4.63 min.
LRMS: *m*/*z* 344 (M+).

### EXAMPLE 22

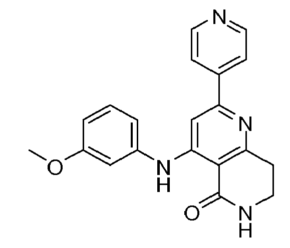

### 4-[(3-Methoxyphenyl)amino]-2-pyridin-4-yl-7,8-dihydro-1,6-naphthyridin-5(6H)-one

The title compound of Preparation 27 (95 mg, 0.23 mmol) was treated with 3-methoxyaniline (175 mg, 1.42 mmol) following the procedure as described in Example 18. The residue was suspended in 4% aqueous sodium carbonate solution and was extracted twice with dichloromethane. The combined organic layers were washed with brine, dried over sodium sulphate and evaporated. The crude was purified by flash chromatography (dichloromethane-methanol 97:3) and gave two fractions, the first one corresponding to 32 mg (0.09 mmol, 39%) of the title compound as an off-white solid. Purity: 95%.
¹H NMR (200 MHz, CHLOROFORM-d) δ ppm 10.83 (1 H, br. s.), 8.67 (2 H, d, *J*=5.5 Hz), 7.76 (2 H, d, *J*=5.9 Hz), 7.40 (1 H, s), 7.17 - 7.36 (1 H, m), 6.69 - 6.99 (3 H, m), 5.80 (1 H, br. s.), 3.83 (3 H, s), 3.65 (2 H, d), 3.18 (2 H, t, *J*=6.6 Hz).
HPLC/MS (30 min) retention time 9.78 min.
LRMS: *m*/*z* 346 (M+).

### EXAMPLE 23

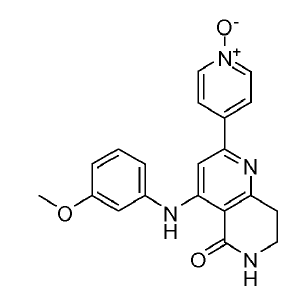

### 4-[(3-Methoxyphenyl)amino]-2-(1-oxidopyridin-4-yl)-7,8-dihydro-1,6-naphthyridin-5(6H)-one

Purification of the crude obtained in the preparation of Example 22 gave a second fraction corresponding to 8 mg (0.02 mmol, 9%) of the title compound as a yellow solid. Purity: 100%.
¹H NMR (200 MHz, CHLOROFORM-d) δ ppm 10.83 (1 H, br. s.), 8.22 (2 H, d, *J*=7.0 Hz), 7.84 (2 H, d, *J*=7.0 Hz), 7.28 - 7.42 (2 H, m), 6.89 (1 H, d, *J*=8.2 Hz), 6.73 - 6.85 (2 H, m), 5.92 (1 H, br. s.), 3.83 (3 H, s), 3.64 (2 H, td, *J*=6.6, 2.5 Hz), 3.14 (2 H, t, *J*=6.6 Hz).
HPLC/MS (30 min) retention time 9.10 min.
LRMS: *m*/*z* 362 (M+).

### EXAMPLE 24

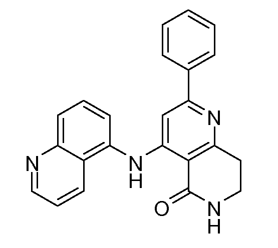

### 2-Phenyl-4-(quinolin-5-ylamino)-7,8-dihydro-1,6-naphthyridin-5(6H)-one

The title compound of Preparation 23 (85 mg, 0.20 mmol) was treated with quinolin-5-amine (149 mg, 1.00 mmol) according to the method of Example 18 followed by purification by reverse phase using the SP1 purification system to give 18 mg (0.04 mmol, 22%) as a pale yellow solid. Purity: 99%.
¹H NMR (200 MHz, CHLOROFORM-d) δ ppm 11.15 (1 H, s), 8.98 (1 H, d, *J*=2.7 Hz), 8.44 (1 H, d, *J*=8.2 Hz), 8.07 (1 H, d, *J*=8.6 Hz), 7.68 - 7.85 (3 H, m), 7.57 - 7.68 (1 H, m), 7.30 - 7.52 (5 H, m), 6.99 (1 H, s), 6.07 (1 H, br. s.), 3.69 (2 H, t, *J*=6.4 Hz), 3.23 (2 H, t, *J*=6.6 Hz).
HPLC/MS (30 min) retention time 8.90 min.
LRMS: *m*/*z* 366 (M+).

### EXAMPLE 25

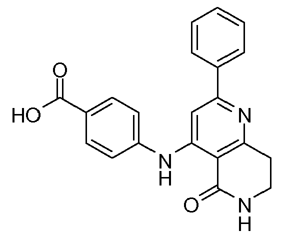

### 4-[(5-Oxo-2-phenyl-5,6,7,8-tetrahydro-1,6-naphthyridin-4-yl)amino]benzoic acid

The title compound of Preparation 23 (85 mg, 0.20 mmol) was treated with 4-aminobenzoic acid (139 mg, 1.00 mmol) according to the method of Example 18 followed by purification by reverse phase using the SP1 purification system to give 18 mg (0.05 mmol, 24%) as a white solid. Purity: 97%.
¹H NMR (200 MHz, DMSO-*d*₆) δ ppm 11.41 (1 H, s), 8.29 (1 H, br. s.), 8.01 - 8.10 (1 H, m), 7.99 (2 H, d, *J*=8.2 Hz), 7.35 - 7.63 (5 H, m), 3.48 (2 H, t, *J*=6.5 Hz), 3.03 (2 H, t, *J*=6.4 Hz).
HPLC/MS (30 min) retention time 8.25 min.
LRMS: *m*/*z* 359 (M+).

### EXAMPLE 26

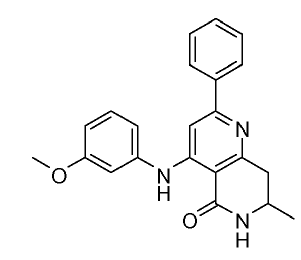

### 4-[(3-Methoxyphenyl)amino]-7-methyl-2-phenyl-7,8-dihydro-1,6-naphthyridin-5(6H)-one

The title compound of Preparation 30 (80 mg, 0.19 mmol) was treated with 3-methoxyaniline (123 mg, 1.00 mmol) according to the Example 18. The residue was dissolved in dichloromethane, washed with aqueous sodium carbonate (x3) and brine, dried over magnesium sulphate, filtered and evaporated. The crude was purified by flash chromatography (ethyl acetate-hexane, gradient from 30:70 to 50:50) to give 39 mg (0.11 mmol, 56%) of the title compound as a white solid. Purity: 99%.
¹H NMR (200 MHz, CHLOROFORM-d) δ ppm 10.78 (1 H, s), 7.83 - 7.93 (2 H, m), 7.30 - 7.49 (5 H, m), 6.92 (1 H, d, *J*=8.6 Hz), 6.84 (1 H, s), 6.76 (1 H, d, *J*=8.6 Hz), 5.60 (1 H, br. s.), 3.85 - 4.06 (1 H, ddt, *J*=11.0, 5.0, 6.5 Hz), 3.82 (3 H, s), 3.17 (1 H, dd, *J*=16.0, 5.0 Hz), 2.93 (1 H, dd, *J*=16.0, 11.0 Hz), 1.39 (3 H, d, *J*=6.6 Hz).
HPLC/MS (30 min) retention time 11.39 min.
LRMS: *m*/*z* 359 (M+).

### EXAMPLE 27

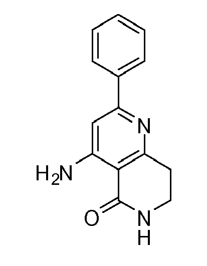

### 4-Amino-2-phenyl-7,8-dihydro-1,6-naphthyridin-5(6H)-one

The title compound of Preparation 23 (125 mg, 0.30 mmol) was treated with 7N ammonia in methanol (2.00 mL, 14.00 mmol) and the mixture was heated in the microwave at 70°C for 2 h and at 100°C for 5 h. The solvent was evaporated and the crude was purified by reverse phase in the SP1 purification system to give 38 mg (0.15 mmol, 52%) of the title compound as a white solid. Purity: 98%.
¹H NMR (200 MHz, DMSO-*d*₆) δ ppm 7.94 (2 H, d, *J*=5.9 Hz), 7.79 (1 H, br. s.), 7.36 - 7.54 (3 H, m), 7.03 (1 H, s), 3.37 (2 H, td, *J*=6.6, 2.5 Hz), 2.89 (2 H, t, *J*=6.8 Hz).
HPLC/MS (30 min) retention time 8.66 min.
LRMS: *m*/*z* 239 (M+).

### EXAMPLE 28

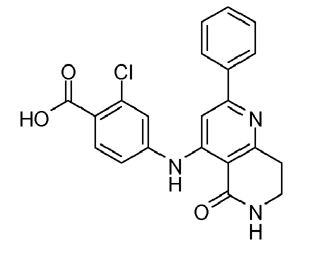

### 2-Chloro-4-[(5-oxo-2-phenyl-5,6,7,8-tetrahydro-1,6-naphthyridin-4-yl)amino]benzoic acid

The title compound of Preparation 23 (80 mg, 0.19 mmol) was treated with 4-amino-2-chlorobenzoic acid (165 mg, 0.96 mmol) following the experimental procedure of Example 18. The crude was purified by reverse phase using the SP1 purification system to give 3 mg (0.007 mmol, 3%) of the title compound. Purity: 98%.
HPLC/MS (30 min) retention time 9.23 min.
LRMS: *m*/*z* 393 (M+).

### EXAMPLE 29

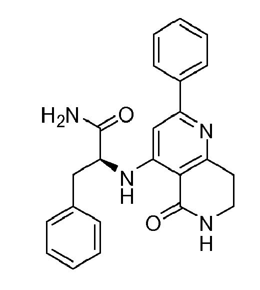

### N-(5-Oxo-2-phenyl-5,6,7,8-tetrahydro-1,6-naphthyridin-4-yl)-L-phenylalaninamide

The title compound of Preparation 23 (150 mg, 0.37 mmol) was treated with (*S*)-2-amino-3-phenylpropanamide (305 mg, 1.86 mmol) and the mixture was heated at 70°C overnight and in the microwave at 160°C for 30 min. Dichloromethane was added and this solution was washed with aqueous potassium carbonate, dried with magnesium sulphate and evaporated. The crude was purified by reverse phase in the SP1 purification system to give 21 mg (0.053 mmol, 14%) of the title compound. Purity: 98%.
¹H NMR (200 MHz, CHLOROFORM-d) δ ppm 9.41 (1 H, d, *J*=5.5 Hz), 7.89 (1 H, qt), 7.26 - 7.49 (8 H, m), 6.74 (1 H, s), 6.23 (1 H, br. s.), 5.79 (1 H, br. s.), 5.44 (1 H, br. s.), 4.23 (1 H, ddd, *J*=8.2, 6.0, 4.5 Hz), 3.57 (2 H, td, *J*=6.7, 2.5 Hz), 3.38 (1 H, dd, *J*=14.0, 4.5 Hz), 3.19 (1 H, dd, *J*=14.0, 8.2 Hz), 3.11 (2 H, t, *J*=6.6 Hz).
HPLC/MS (30 min) retention time 8.38 min.
LRMS: *m*/*z* 386 (M+).

### EXAMPLE 30

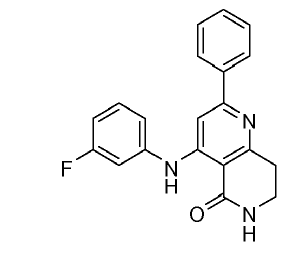

### 4-[(3-Fluorophenyl)amino]-2-phenyl-7,8-dihydro-1,6-naphthyridin-5(6H)-one

The title compound of Preparation 23 (150 mg, 0.37 mmol) was treated with 3-fluoroaniline (0.18 mL, 1.87 mmol) according to the method of Example 18. Dichloromethane was added to the residue, the organic solution was washed with 4% aqueous solution of potassium carbonate, dried with magnesiumsulphate, filtered and evaporated. The crude was purified by reverse phase in the SP1 purification system to give 24 mg (0.072 mmol, 19%) of the title compound. Purity: 98%.
¹H NMR (200 MHz, CHLOROFORM-*d*) δ ppm 10.87 (1 H, br. s.), 7.79 - 7.95 (2 H, m), 7.29 - 7.53 (5 H, m), 6.97 - 7.14 (2 H, m), 6.89 (1 H, t, *J*=8.2 Hz), 5.88 (1 H, br. s.), 3.64 (2 H, dt, *J*=6.5, 2.0 Hz), 3.19 (2 H, t, *J*=6.6 Hz).
HPLC/MS (30 min) retention time 11.81 min.
LRMS: *m*/*z* 333 (M+).

### EXAMPLE 31

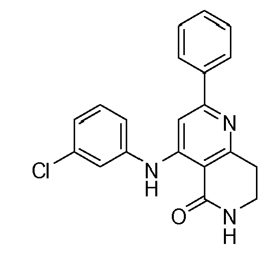

### 4-[(3-Chlorophenyl)amino]-2-phenyl-7,8-dihydro-1,6-naphthyridin-5(6H)-one

The title compound of Preparation 23 (150 mg, 0.37 mmol) was treated with 3-chloroaniline (0.20 mL, 1.89 mmol) according to the method of Example 18. The crude was purified by reverse phase using the SP1 purification system to give 22 mg (0.062 mmol, 17%) of the title compound. Purity: 99%.
¹H NMR (200 MHz, CHLOROFORM-d) δ ppm 10.84 (1 H, s), 7.79 - 7.93 (2 H, m), 7.36 - 7.51 (3 H, m), 7.28 - 7.36 (3 H, m), 7.19 (2 H, t, *J*=7.2 Hz), 5.89 (1 H, br. s.), 3.64 (2 H, td, *J*=6.6, 2.7 Hz), 3.18 (2 H, t, *J*=6.8 Hz).
HPLC/MS (30 min) retention time 12.82 min.
LRMS: *m*/*z* 349 (M+).

### EXAMPLE 32

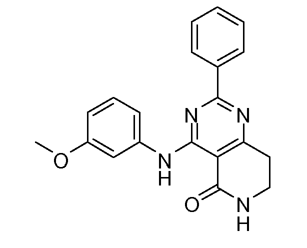

### 4-[(3-Methoxyphenyl)amino]-2-phenyl-7,8-dihydropyrido[4,3-d]pyrimidin-5(6H)-one

The crude title compound of Preparation 34 (84 mg) was treated with 3-methoxyaniline (0.16 mL, 1.40 mmol) according to the method of Example 18 to give 43 mg (0.12 mmol, 83% over two steps) of the title compound as a yellow solid. Purity: 99%.
¹H NMR (200 MHz, DMSO-*d*₆) δ ppm 11.61 (1 H, s), 8.26 - 8.53 (3 H, m), 7.47 - 7.71 (4 H, m), 7.33 (1 H, t, *J*=7.8 Hz), 7.21 (1 H, d, *J*=8.0 Hz), 6.71 (1 H, d, *J*=7.8 Hz), 3.83 (3 H, s), 3.50 (2 H, td, *J*=6.5, 2.0 Hz), 3.01 (2 H, t, *J*=6.8 Hz).
HPLC/MS (30 min) retention time 15.70 min.
LRMS: *m*/*z* 346 (M+).

### EXAMPLE 33

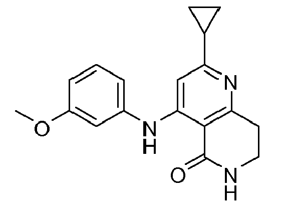

### 2-Cyclopropyl-4-[(3-methoxyphenyl)amino]-7,8-dihydro-1,6-naphthyridin-5(6H)-one

The title compound of Preparation 32 (13 mg, 0.03 mmol) was treated with 3-methoxyaniline (25 mg, 0.20 mmol) according to the Example 18. The mixture was evaporated, resuspended in dichloromethane and washed successively with 4% aqueous sodium carbonate (x3) solution, brine and then dried over sodium sulphate. Evaporation gave a residue which was purified by flash chromatography (ethyl acetate-hexane, gradient 25:75 to 40:60) to give 3 mg (0.01 mmol, 31%) of the title compound as an off-white solid. Purity: 100%.
¹H NMR (200 MHz, CHLOROFORM-d) δ ppm 10.82 (1 H, br. s.), 7.29 - 7.37 (1 H, m), 6.84 (1 H, d, *J*=8.2 Hz), 6.71 - 6.80 (2 H, m), 6.64 (1 H, s), 5.75 (1 H, br. s.), 3.58 (3 H, td, *J*=6.6, 2.3 Hz), 3.15 (2 H, t, *J*=6.6 Hz), 1.96 - 2.12 (1 H, m), 0.75 - 1.05 (4 H, m).
HPLC/MS (30 min) retention time 8.13 min.
LRMS: *m*/*z* 309 (M+).

### EXAMPLE 34

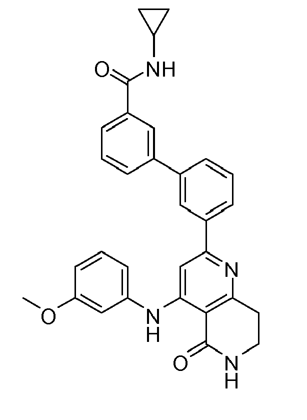

### N-Cyclopropyl-3'-{4-[(3-methoxyphenyl)amino]-5-oxo-5,6,7,8-tetrahydro-1,6-naphthyridin-2-yl}biphenyl-3-carboxamide

The title compound of Example 7 (100 mg, 0.24 mmol) was treated with 3-(cyclopropylcarbamoyl)phenylboronic acid (72 mg, 0.35 mmol), 2M cesium carbonate solution (0.35 mL, 0.70 mmol) and 1,1'-bis(diphenylphosphino)ferrocene-palladium(II) dichloride dichloromethane complex (10 mg, 0.01 mmol) ) according to the method of Preparation 42 to give 58 mg (0.11 mmol, 48%) of the title compound. Purity: 100%.
¹H NMR (200 MHz, CHLOROFORM-d) δ ppm 10.81 (1 H, s), 8.15 (1 H, s), 7.98 (1 H, s), 7.67 - 7.85 (3 H, m), 7.57 - 7.67 (1 H, m), 7.49 (2 H, t, *J*=7.6 Hz), 7.41 (1 H, s), 7.29 - 7.37 (1 H, m), 6.92 (1 H, d, *J*=8.2 Hz), 6.85 (1 H, t, *J*=2.0 Hz), 6.75 (1 H, dd, *J*=8.0, 2.5 Hz), 6.31 (1 H, br. s.), 5.78 (1 H, br. s.), 3.81 (3 H, s), 3.65 (2 H, td, *J*=6.6, 2.3 Hz), 3.19 (2 H, t, *J*=6.6 Hz), 2.86 - 3.01 (1 H, m), 0.89 (2H, td, *J*=7.0, 6.0 Hz), 0.58 - 0.70 (2 H, m).
HPLC/MS (30 min) retention time 12.48 min.
LRMS: *m*/*z* 504 (M+).

### EXAMPLE 35

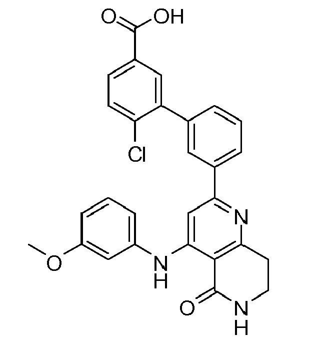

### 6-Chloro-3'-{4-[(3-methoxyphenyl)amino]-5-oxo-5,6,7,8-tetrahydro-1,6-naphthyridin-2-yl}biphenyl-3-carboxylic acid

The title compound of Example 7 (100 mg, 0.24 mmol) was treated with 4-chloro-3-(4,4,5,5-tetramethyl-1,3,2-dioxaborolan-2-yl)benzoic acid (100 mg, 0.35 mmol), 2M cesium carbonate solution (0.35 mL, 0.70 mmol) and 1,1'-bis(diphenylphosphino)ferrocene-palladium(II) dichloride dichloromethane complex (10 mg, 0.01 mmol) ) according to the method of Preparation 42. The crude was purified using the SP1 purification system (gradient hexane-ethyl acetate) followed by a second purification (gradient ethyl acetate-methanol) to give 4.5 mg (0.008 mmol, 4%) of the title compound. Purity: 97%.
¹H NMR (partial 200 MHz, DMSO-*d*₆) δ ppm 11.10 (1 H, s), 8.20 (1 H, br. s.), 8.00 (1 H, s), 7.79 - 7.95 (3 H, m), 7.41 - 7.65 (4 H, m), 7.34 (1 H, t, *J*=8.2 Hz), 6.85 - 7.02 (2 H, m), 6.76 (1 H, d, *J*=7.8 Hz), 3.77 (3 H, s), 3.01 (2 H, t, *J*=6.1 Hz).
HPLC/MS (30 min) retention time 14.39 min.
LRMS: *m*/*z* 499 (M+).

### EXAMPLE 36

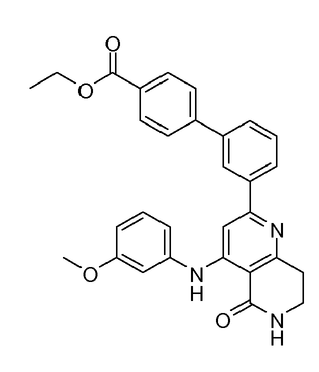

### Ethyl 3'-{4-[(3-methoxyphenyl)amino]-5-oxo-5,6,7,8-tetrahydro-1,6-naphthyridin-2-yl}biphenyl-4-carboxylate

The title compound of Example 7 (100 mg, 0.24 mmol) was treated with 4-(ethoxycarbonyl)phenylboronic acid (70 mg, 0.36 mmol), 2M cesium carbonate solution (0.35 mL, 0.70 mmol) and 1,1'-bis(diphenylphosphino)ferrocene-palladium(II) dichloride dichloromethane complex (10 mg, 0.01 mmol) ) according to the method of Preparation 42. The crude was purified using the SP1 purification system (gradient hexane-ethyl acetate) to give 22 mg (0.052 mmol, 22%) of the title compound. Purity: 100%.
¹H NMR (200 MHz, CHLOROFORM-d) δ ppm 10.81 (1 H, s), 8.17 (1 H, s), 8.12 (2 H, d, *J*=8.2 Hz), 7.83 (1 H, d, *J*=7.8 Hz), 7.70 (2 H, d, *J*=8.2 Hz), 7.59 - 7.65 (1 H, m), 7.51 (1 H, t, *J*=7.6 Hz), 7.41 (1 H, s), 7.29 - 7.38 (1 H, m), 6.92 (1 H, d, *J*=7.8 Hz), 6.85 (1 H, s), 6.76 (1 H, dd, *J*=8.6, 2.5 Hz), 5.77 (1 H, br. s.), 4.41 (2 H, q, *J*=7.3 Hz), 3.82 (3 H, s), 3.65 (2 H, td, *J*=6.8, 2.3 Hz), 3.19 (2 H, t, *J*=6.8 Hz), 1.42 (3 H, t, *J*=7.0 Hz).
HPLC/MS (30 min) retention time 16.4 min.
LRMS: *m*/*z* 493 (M+).

### EXAMPLE 37

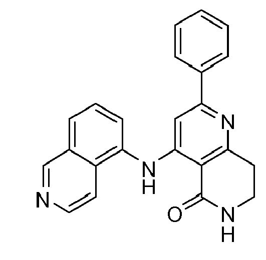

### 4-(Isoquinolin-5-ylamino)-2-phenyl-7,8-dihydro-1,6-naphthyridin-5(6H)-one

The title compound of Preparation 23 (150 mg, 0.37 mmol) was treated with isoquinolin-5-amine (271 mg, 1.86 mmol) according to the method of Example 18. The crude was purified by reverse phase using the SP1 purification system to give 10 mg (0.026 mmol, 7%) of the title compound as a solid. Purity: 100%.
¹H NMR (200 MHz, CHLOROFORM-d) δ ppm 11.21 (1 H, s), 9.33 (1 H, s), 8.57 (1 H, d, *J*=5.9 Hz), 7.84 - 7.96 (2 H, m), 7.72 - 7.82 (3 H, m), 7.66 (1 H, t, *J*=8.0 Hz), 7.32 - 7.42 (3 H, m), 7.02 (1 H, s), 5.93 (1 H, br. s.), 3.70 (2 H, td, *J*=6.6, 2.3 Hz), 3.23 (2 H, t, *J*=6.6 Hz).
HPLC/MS (30 min) retention time 7.63 min.
LRMS: *m*/*z* 366 (M+).

### EXAMPLE 38

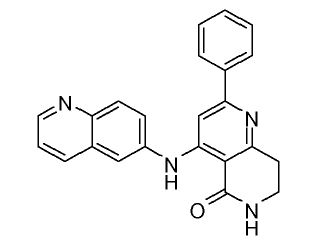

### 2-Phenyl-4-(quinolin-6-ylamino)-7,8-dihydro-1,6-naphthyridin-5(6H)-one

The title compound of Preparation 23 (150 mg, 0.37 mmol) was treated with quinolin-6-amine (274 mg, 1.86 mmol) and *N,N'*-diisopropylethylamine (0.32 mL, 1.86 mmol) according to the method of Example 18. The crude was purified by reverse phase using the SP1 purification system followed by a purification by flash chromatography using a Varian cartridge with 2g of silica (hexane/ethyl acetate) to give 15 mg (0.039 mmol, 11 %) of the title compound as a pale yellow solid. Purity: 100%.
¹H NMR (200 MHz, CHLOROFORM-d) δ ppm 11.06 (1 H, s), 8.88 (1 H, d, *J*=2.7 Hz), 8.13 (2 H, t, *J*=8.6 Hz), 7.87 (1 H, dd, *J*=6.0, 2.5 Hz), 7.63 - 7.77 (1 H, m), 7.41 (5 H, d, *J*=5.1 Hz), 6.02 (1 H, br. s.), 3.67 (2 H, td, *J*=6.6, 2.5 Hz), 3.21 (2 H, t).
HPLC/MS (30 min) retention time 8.57 min.
LRMS: *m*/*z* 366 (M+).

### EXAMPLE 39

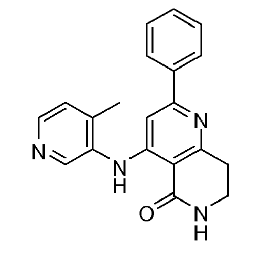

### 4-[(4-Methylpyridin-3-yl)amino]-2-phenyl-7,8-dihydro-1,6-naphthyridin-5(6H)-one

The title compound of Example 28 (45 mg, 0.19 mmol) was treated with 3-bromo-4-methylpyridine (26 mg, 0.23 mmol), cesium carbonate (85 mg, 0.26 mmol), bis(diphenylphosphino)-9,9-dimethylxanthene (22 mg, 0.04 mmol) and tris(dibenzylidene acetone)dipalladium (0) (17 mg, 0.02mol) according to the method of Preparation 54 followed by purification by reverse phase using the SP1 purification system to give 26 mg (0.078 mmol, 42%) of the title compound as a white solid. Purity: 100%.
¹H NMR (200 MHz, CHLOROFORM-d) δ ppm (partial) 10.66 (1 H, s), 8.62 (1 H, br. s.), 8.40 (1 H, br. s.), 7.77 - 7.88 (2 H, m), 7.34 - 7.45 (2 H, m), 6.89 (1 H, s), 5.83 (1 H, br. s.), 3.67 (2 H, td, *J*=6.7, 2.5 Hz), 3.21 (2 H, t, *J*=6.8 Hz), 2.33 (3 H, s).
HPLC/MS (30 min) retention time 6.89 min.
LRMS: *m*/*z* 330 (M+).

### EXAMPLE 40

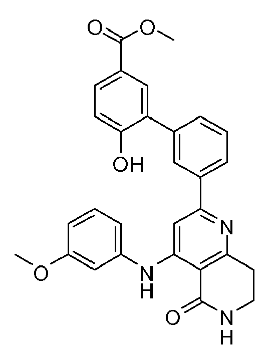

### Methyl 6-hydroxy-3'-{4-[(3-methoxyphenyl)amino]-5-oxo-5,6,7,8-tetrahydro-1,6-naphthyridin-2-yl}biphenyl-3-carboxylate

The title compound of Example 7 (120 mg, 0.28 mmol) was treated with the title compound of Preparation 36 (120 mg, 0.43 mmol), 2M cesium carbonate solution (0.42 mL, 0.84 mmol) and 1,1'-bis(diphenylphosphino)ferrocene-palladium(II) dichloride dichloromethane complex (12 mg, 0.01 mmol) ) according to the method of Preparation 42. The crude was purified using the SP1 purification system (gradient hexane-ethyl acetate) to give 55 mg (0.11 mmol, 39%) of the title compound. Purity: 100%.
¹H NMR (200 MHz, DMSO-*d*₆) δ ppm 11.11 (1 H, s), 10.58 (1 H, br. s.), 8.19 (1 H, br. s.),8.08 (1 H, s), 7.78 - 7.93 (3 H, m), 7.57 - 7.66 (1 H, m), 7.51 (1 H, t, *J*=7.8 Hz), 7.45 (1 H, s), 7.34 (1 H, t, *J*=8.0 Hz), 7.06 (1 H, d, *J*=8.2 Hz), 6.86 - 7.00 (2 H, m), 6.76 (1 H, d, *J*=8.6 Hz), 3.81 (3 H, s), 3.77 (3 H, s), 3.47 (2 H, td, *J*=6.6, 2.5 Hz), 3.01 (2 H, t, *J*=6.4 Hz).
HPLC/MS (30 min) retention time 12.83 min.
LRMS: *m*/*z* 495 (M+).

### EXAMPLE 41

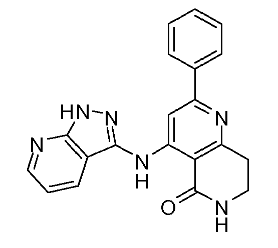

### 2-Phenyl-4-(1H-pyrazolo[3,4-b]pyridin-3-ylamino)-7,8-dihydro-1,6-naphthyridin-5(6H)-one

The title compound of Preparation 23 (140 mg, 0.35 mmol) was treated with the title compound of Preparation 37 (140 mg, 1.04 mmol) according to the method of Example 18 followed by purification by reverse phase using the SP1 purification system to give 66 mg (0.18 mmol, 53%) of the title compound as a pale yellow solid. Purity: 100%.
¹H NMR (200 MHz, DMSO-*d*₆) δ ppm 13.19 (1 H, s), 12.37 (1 H, s), 8.67 (1 H, s), 8.58 (1 H, d, *J*=4.3 Hz), 8.34 (1 H, br. s.), 8.10 (1 H, d, *J*=8.2 Hz), 8.02 (2 H, d, *J*=5.9 Hz), 7.39 - 7.62 (3 H, m), 7.23 (1 H, dd, *J*=8.0, 4.5 Hz), 3.51 (2 H, br. s.), 3.08 (2 H, t, *J*=6.4 Hz).
HPLC/MS (30 min) retention time 9.10 min.
LRMS: *m*/*z* 356 (M+).

### EXAMPLE 42

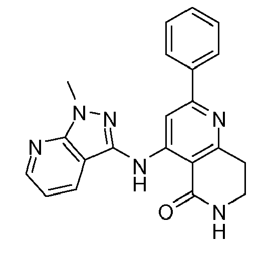

### 4-[(1-methyl-1H-pyrazolo[3,4-b]pyridin-3-yl)amino]-2-phenyl-7,8-dihydro-1,6-naphthyridin-5(6H)-one

The title compound of Preparation 23 (150 mg, 0.37 mmol) was treated with the title compound of Preparation 38 (170 mg, 1.15 mmol) according to the method of Example 18 followed by purification by reverse phase using the SP1 purification system to give 7 mg (0.018 mmol, 5%) of the title compound. Purity: 100%.
¹H NMR (200 MHz, CHLOROFORM-d) δ ppm 11.94 (1 H, s), 8.63 (1 H, s), 8.57 (1 H, d, *J*=4.3 Hz), 8.07 (3 H, t, *J*=8.4 Hz), 7.37 - 7.57 (3 H, m), 7.10 (1 H, dd, *J*=7.8, 4.7 Hz), 5.81 (1 H, br. s.), 4.14 (3 H, s), 3.68 (2 H, td, *J*=6.8, 2.5 Hz), 3.25 (2 H, t, *J*=6.8 Hz).
HPLC/MS (30 min) retention time 10.87 min.
LRMS: *m*/*z* 370 (M+).

### EXAMPLE 43

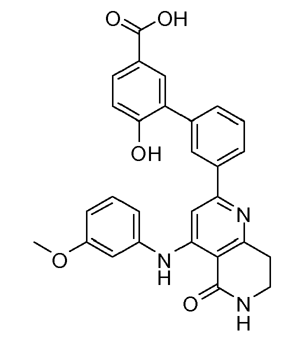

### 6-Hydroxy-3'-{4-[(3-methoxyphenyl)amino]-5-oxo-5,6,7,8-tetrahydro-1,6-naphthyridin-2-yl}biphenyl-3-carboxylic acid

The title compound of Example 40 (50 mg, 0.10 mmol) in a mixture of tetrahydrofuran/water was treated with lithium hydroxide (42 mg, 1.00 mmol) and the mixture was stirred at 40°C for 3 days. The solvent was evaporated, water was added and the pH was adjusted to 5. The resulting suspension was filtered, the solid was washed with diethyl ether and dried to give 25 mg (0.051 mmol, 51%) of the title compound. Purity: 100%.
1 H NMR (400 MHz, DMSO-*d*₆) δ ppm (partial) 10.87 (1 H, br. s.), 8.53 (1 H, br. s.), 8.00 (0 H, br. s.), 7.89 (1 H, br. s.), 7.76 - 7.83 (2 H, m), 7.67 - 7.76 (1 H, m), 7.58 (1 H, br. s.), 7.40 (1 H, t, *J*=7.6 Hz), 7.25 - 7.35 (1 H, m), 7.20 (1 H, d, *J*=8.6 Hz), 6.94 - 7.06 (1 H, m), 6.87 (1 H, br. s.), 3.78 (3 H, s).
HPLC/MS (30 min) retention time 11.00 min.
LRMS: *m*/*z* 481 (M+).

### EXAMPLE 44

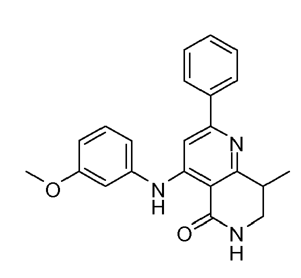

### 4-[(3-Methoxyphenyl)amino]-8-methyl-2-phenyl-7,8-dihydro-1,6-naphthyridin-5(6H)-one

The title compound of Preparation 29 (84 mg, 0.20 mmol) was treated with 3-methoxyaniline (123 mg, 1.00 mmol) according to the method of Example 18 followed by purification by flash chromarography (gradient hexane/ethyl acetate from 75:25 to 50:50) to give 55 mg (0.15 mmol, 75%) of the title compound as a brown solid. Purity: 99%.
¹H NMR (200 MHz, CHLOROFORM-d) δ ppm 10.80 (1 H, s), 7.86 - 7.97 (2 H, m), 7.35 - 7.47 (4 H, m), 7.31 (1 H, t, *J*=7.8 Hz), 6.91 (1 H, d, *J*=7.8 Hz), 6.84 (1 H, s), 6.75 (1 H, dd, *J*=8.2, 2.2 Hz), 5.77 (1 H, br. s.), 3.82 (3 H, s), 3.74 (1 H, ddd, *J*=12.0, 5.0, 2.3 Hz), 3.26 - 3.40 (1 H, m), 3.14 - 3.26 (1 H, m), 1.47 (3 H, d, *J*=6.6 Hz).
HPLC/MS (30 min) retention time 11.46 min.
LRMS: *m*/*z* 359 (M+).

### EXAMPLE 45

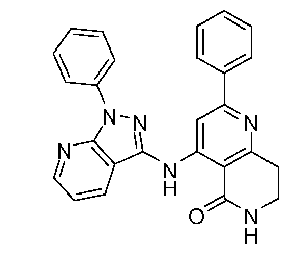

### 2-Phenyl-4-[(1-phenyl-1H-pyrazolo[3,4-b]pyridin-3-yl)amino]-7,8-dihydro-1,6-naphthyridin-5(6H)-one

The title compound of Preparation 23 (150 mg, 0.37 mmol) was treated with the title compound of Preparation 39 (260 mg, 1.15 mmol) according to the method of Example 18 to give 64 mg (0.15 mmol, 40%) of the title compound. Purity: 100%.
¹H NMR (200 MHz, CHLOROFORM-d) δ ppm 12.27 (1 H, s), 9.08 (1 H, s), 8.66 (1 H, d, *J*=4.3 Hz), 8.41 (2 H, d, *J*=8.2 Hz), 8.17 (3 H, t, *J*=7.0 Hz), 7.41 - 7.63 (5 H, m), 7.10 - 7.33 (2 H, m), 5.90 (1 H, br. s.), 3.69 (2 H, td, *J*=6.8, 2.3 Hz), 3.27 (2 H, t, *J*=6.8 Hz).
HPLC/MS (30 min) retention time 19.95 min.
LRMS: *m*/*z* 432 (M+).

### EXAMPLE 46

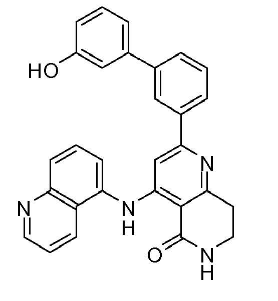

### 2-(3'-Hydroxybiphenyl-3-yl)-4-(quinolin-5-ylamino)-7,8-dihydro-1,6-naphthyridin-5(6H)-one

The title compound of Preparation 42 (103 mg, 0.31 mmol) was treated with naphtyridin-5(6H)-one (80mg, 0.37 mmol), cesium carbonate (146 mg, 0.43 mmol), bis(diphenylphosphino)-9,9-dimethylxanthene (36 mg, 0.06 mmol) and tris(dibenzylidene acetone)dipalladium (0) (28 mg, 0.03 mmol) according to the method of Preparation 54 followed by purification by reverse phase using the SP1 purification system to give 75 mg (0.16 mmol, 52%) of the title compound as a white solid. Purity: 100%.
¹H NMR (200 MHz, DMSO-*d*₆) δ ppm 11.63 (1 H, s), 9.57 (1 H, br. s.), 8.99 (1 H, d, *J*=3.9 Hz), 8.40 (1 H, d, *J*=8.6 Hz), 8.31 (1 H, br. s.), 8.07 (1 H, s), 7.91 - 8.01 (1 H, m), 7.67 - 7.91 (3 H, m), 7.56 - 7.68 (1 H, m), 7.48 (1 H, d, *J*=7.4 Hz), 7.17 - 7.34 (2 H, m), 6.94 - 7.11 (2 H, m), 6.78 (1 H, d, *J*=8.6 Hz), 3.46 - 3.62 (2 H, m), 3.08 (2 H, t, *J*=6.4 Hz).
HPLC/MS (30 min) retention time 10.63 min.
LRMS: *m*/*z* 458 (M+).

### EXAMPLE 47

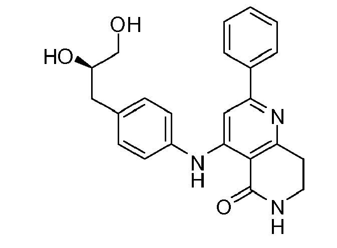

### 4-({4-[(2R)-2,3-dihydroxypropyl]phenyl}amino)-2-phenyl-7,8-dihydro-1,6-naphthyridin-5(6H)-one

The title compound of Preparation 23 (200 mg, 0.50 mmol) was treated with the title compound of Preparation 43 (305 mg, 1.47 mmol) according to the method of Example 18 followed by purification using the SP1 purification system (gradient hexane/ethyl acetate) to give 42 mg (0.097 mmol, 19%). Purity: 100%.
¹H NMR (400 MHz, DMSO-*d*₆) δ ppm 11.03 (1 H, s), 8.16 (1 H, br. s.), 7.89 (2 H, d, *J*=6.3 Hz), 7.39 - 7.51 (3 H, m), 7.27 - 7.32 (3 H, m), 7.22 - 7.27 (2 H, m), 4.58 - 4.64 (2 H, m), 3.60 - 3.70 (1 H, m), 3.46 (2 H, td, *J*=6.5, 2.5 Hz), 3.00 (2 H, t, *J*=6.7 Hz), 2.80 (1 H, dd, *J*=13.5, 4.5 Hz), 2.54 (1 H, dd, *J*=13.5, 8.5 Hz).
HPLC/MS (30 min) retention time 7.40 min.
LRMS: *m*/*z* 389 (M+).

### EXAMPLE 48

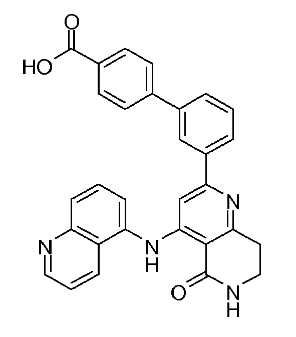

### 3'-[5-Oxo-4-(quinolin-5-ylamino)-5,6,7,8-tetrahydro-1,6-naphthyridin-2-yl]biphenyl-4-carboxylic acid

The title compound of Preparation 46 (102 mg, 0.28 mmol) was treated with 5-bromoquinoline (73 mg, 0.34 mmol), cesium carbonate (134 mg, 0.40 mmol), bis(diphenylphosphino)-9,9-dimethylxanthene (33 mg, 0.06 mmol) and tris(dibenzylidene acetone)dipalladium (0) (26 mg, 0.03 mmol) according to the method of Preparation 54 followed by purification by reverse phase using the SP1 purification system to give 13 mg (0.26 mmol, 9%) of the title compound as a pale yellow solid. Purity: 100%.
¹H NMR (200 MHz, DMSO-*d*₆) d ppm 12.98 (1 H, br. s.), 11.68 (1 H, s), 8.99 (1 H, d, *J*=2.7 Hz), 8.41 (1 H, d, *J*=8.6 Hz), 8.35 (1 H, br. s.), 8.17 (1 H, s), 8.03 (2 H, d, *J*=8.2 Hz), 7.93 (1 H, t, *J*=8.2 Hz), 7.70 - 7.87 (5 H, m), 7.63 (1 H, dd, *J*=8.6, 4.3 Hz), 7.53 (1 H, t, *J*=7.6 Hz), 7.25 (1 H, s), 3.54 (2 H, br. s.), 3.10 (2 H, t, *J*=6.4 Hz).
HPLC/MS (30 min) retention time 10.92 min.
LRMS: *m*/*z* 486 (M+).

### EXAMPLE 49

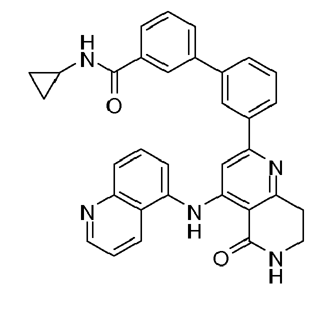

### N-Cyclopropyl-3'-[5-oxo-4-(quinolin-5-ylamino)-5,6,7,8-tetrahydro-1,6-naphthyridin-2-yl]biphenyl-3-carboxamide

The title compound of Preparation 45 (170 mg, 0.43 mmol) was treated with 5-bromoquinoline (107 mg, 0.51 mmol), cesium carbonate (200 mg, 0.60 mmol), bis(diphenylphosphino)-9,9-dimethylxanthene (49 mg, 0.09 mmol) and tris(dibenzylidene acetone)dipalladium (0) (39 mg, 0.04 mmol) according to the method of Preparation 54. Purification by reverse phase using the SP1 purification system followed by trituration using hot ethanol and then acetonitrile gave 49 mg (0.087 mmol, 20%) of the title compound as a white solid. Purity: 100%.
¹H NMR (200 MHz, DMSO-*d*₆) δ ppm 11.66 (1 H, s), 8.98 (1 H, d, *J*=2.7 Hz), 8.54 (1 H, d, *J*=3.9 Hz), 8.41 (1 H, d, *J*=8.2 Hz), 8.32 (1 H, br. s.), 8.15 (1 H, s), 8.07 (1 H, s), 7.91 - 8.00 (1 H, m), 7.79 (6 H, d, *J*=9.4 Hz), 7.46 - 7.69 (3 H, m), 7.25 (1 H, s), 3.54 (2 H, t, *J*=6.5 Hz), 3.09 (2 H, t, *J*=6.6 Hz), 2.88 (1 H, tt, *J*=7.0, 3.5 Hz), 0.66 - 0.80 (2 H, m), 0.49 - 0.65 (2 H, m).
HPLC/MS (30 min) retention time 10.91 min.
LRMS: *m*/*z* 525 (M+).

### EXAMPLE 50

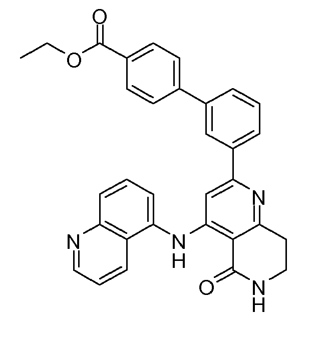

### Ethyl 3'-[5-oxo-4-(quinolin-5-ylamino)-5,6,7,8-tetrahydro-1,6-naphthyridin-2-yl]biphenyl-4-carboxylate

The title compound of Preparation 46 (162 mg, 0.42 mmol) was treated with 5-bromoquinoline (106 mg, 0.50 mmol), cesium carbonate (197 mg, 0.59 mmol), bis(diphenylphosphino)-9,9-dimethylxanthene (48 mg, 0.08 mmol) and tris(dibenzylidene acetone)dipalladium (0) (38 mg, 0.04 mmol) according to the method of Preparation 54. The crude was triturated in methanol, filtered and dried to give 143 mg (0.27 mmol, 64%) of the title compound as a brown solid. Purity: 97%.
¹H NMR (200 MHz, CHLOROFORM-d) δ ppm 11.19 (1 H, br. s.), 8.98 (1 H, d, *J*=3.1 Hz), 8.45 (1 H, d, *J*=8.2 Hz), 7.98 - 8.20 (4 H, m), 7.77 (1 H, t, *J*=8.0 Hz), 7.63 (5 H, d, *J*=7.8 Hz), 7.37 - 7.54 (2 H, m), 7.03 (1 H, s), 5.92 (1 H, br. s.), 4.40 (2 H, q, *J*=7.0 Hz), 3.71 (2 H, t, *J*=6.5 Hz), 3.25 (2 H, t, *J*=6.2 Hz), 1.41 (3 H, t, *J*=7.0 Hz).
HPLC/MS (30 min) retention time 14.75 min.
LRMS: *m*/*z* 514 (M+).

### EXAMPLE 51

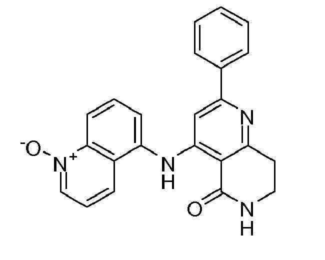

### 4-[(1-Oxidoquinolin-5-yl)amino]-2-phenyl-7,8-dihydro-1,6-naphthyridin-5(6H)-one

The title compound of Example 27 (125 mg, 0.52 mmol) was treated with the title compound of Preparation 47 (141 mg, 0.62 mmol), cesium carbonate (243 mg, 0.72 mmol), bis(diphenylphosphino)-9,9-dimethylxanthene (60 mg, 0.10 mmol) and tris(dibenzylidene acetone)dipalladium (0) (47mg, 0.05 mmol) according to the method of Preparation 54. The crude was purified by reverse phase using the SP1 purification system to give 69 mg (0.18 mmol, 35%) of the title compound as a yellow solid. Purity: 100%.
¹H NMR (200 MHz, DMSO-*d*₆) δ ppm 11.68 (1 H, s), 8.66 (1 H, d, *J*=5.9 Hz), 8.43 (1 H, t, *J*=4.9 Hz), 8.33 (1 H, br. s.), 7.81 - 7.97 (5 H, m), 7.53 (1 H, dd, *J*=8.8, 6.1 Hz), 7.35 - 7.47 (3 H, m), 7.21 (1 H, s), 3.52 (2 H, td, *J*=6.5, 2.0 Hz), 3.06 (2 H, t, *J*=6.6 Hz).
HPLC/MS (30 min) retention time 7.20 min.
LRMS: *m*/*z* 382 (M+).

### EXAMPLE 52

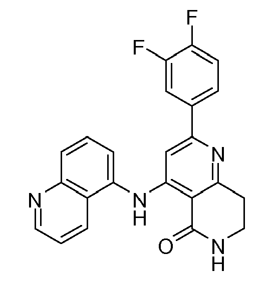

### 2-(3,4-Difluorophenyl)-4-(quinolin-5-ylamino)-7,8-dihydro-1,6-naphthyridin-5(6H)-one

The title compound of Preparation 48 (90 mg, 0.33 mmol) was treated with 5-bromoquinoline (85 mg, 0.39 mmol), cesium carbonate (155 mg, 0.46 mmol), bis(diphenylphosphino)-9,9-dimethylxanthene (38 mg, 0.07 mmol) and tris(dibenzylidene acetone)dipalladium (0) (30 mg, 0.03 mmol) according to the method of Preparation 54. The crude was purified (gradient hexane/ethyl acetate/methanol) using the SP1 purification system to give 52 mg (0.13 mmol, 39%) of the title compound as a white solid. Purity: 100%.
¹H NMR (200 MHz, DMSO-*d*₆) δ ppm 11.65 (1 H, s), 8.98 (1 H, d, *J*=3.9 Hz), 8.38 (1 H, d, *J*=8.6 Hz), 8.33 (1 H, br. s.), 7.91 - 8.03 (2 H, m), 7.87 (1 H, d, *J*=7.4 Hz), 7.55 - 7.83 (3 H, m), 7.35 - 7.54 (1 H, m), 7.21 (1 H, s), 3.45 - 3.61 (2 H, m), 3.06 (2 H, t, *J*=6.6 Hz).
HPLC/MS (30 min) retention time 12.14 min.
LRMS: *m*/*z* 402 (M+).

### EXAMPLE 53

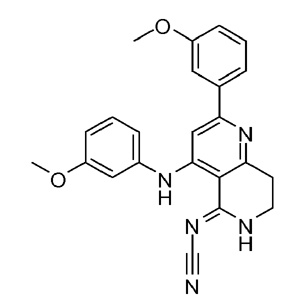

### [(5Z)-2-(3-methoxyphenyl)-4-[(3-methoxyphenyl)amino]-7,8-dihydro-1,6-naphthyridin-5(6H)-ylidene]cyanamide

The title compound of Preparation 50 (12 mg, 0.03 mmol) was treated with cyanamide (5 mg, 0.12 mmol) in 0.8 mL of ethanol. The mixture was stirred at 70°C for 45 min, then cooled to room temperature and filtered to give 6 mg (0.015 mmol, 51%) of the title compound as yellow solid. Purity: 100%.
¹H NMR (200 MHz, CHLOROFORM-d) δ ppm 10.57 (1 H, br. s.), 7.43 - 7.50 (1 H, m), 7.27 - 7.42 (5 H, m), 6.70 - 7.02 (4 H, m), 3.85 (3 H, s), 3.84 (3 H, s), 3.73 (2 H, td, *J*=6.5, 2.5 Hz), 3.21 (2 H, t, *J*=6.4 Hz).
HPLC/MS (30 min) retention time 14.83 min.
LRMS: *m*/*z* 399 (M+).

### EXAMPLE 54

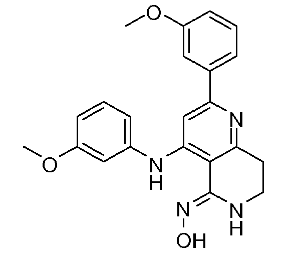

### (5Z)-2-(3-Methoxyphenyl)-4-[(3-methoxyphenyl)amino]-7,8-dihydro-1,6-naphthyridin-5(6H)-one oxime

The title compound of Preparation 50 (12 mg, 0.03 mmol), potassium carbonate (9 mg, 0.06 mmol) and hydroxylamine hydrochloride (4 mg, 0.06 mmol) were suspended in 0.8 mL of ethanol. The mixture was stirred at 70°C for 30 min, diisopropylethylamine (0.05 mL, 0.29 mmol) was added and the mixture was stirred at 70°C overnight. The solvent was evaporated and the crude was purified by flash chromatography (gradient hexane/ethyl acetate from 50:50 to ethyl acetate) to give 3 mg (0.007 mmol, 23%) of the title compound as a solid. Purity: 90%.
¹H NMR (200 MHz, CHLOROFORM-d) δ ppm 10.21 (1 H, br. s.), 7.12 - 7.60 (5 H, m), 6.90 (4 H, m), 5.77 (1 H, br. s.), 3.68 - 3.97 (6 H, m), 3.38 - 3.61 (2 H, m), 3.16 (2 H, br. s.).
HPLC/MS (30 min) retention time 10.70 min.
LRMS: *m*/*z* 390 (M+).

### EXAMPLE 55

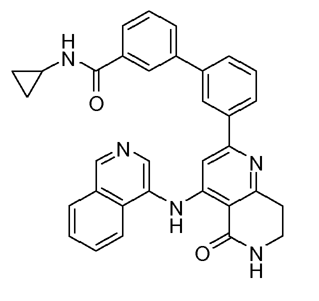

### N-Cyclopropyl-3'-[4-(isoquinolin-4-ylamino)-5-oxo-5,6,7,8-tetrahydro-1,6-naphthyridin-2-yl]biphenyl-3-carboxamide

The title compound of Preparation 45 (415 mg, 1.04 mmol) was treated with 4-bromoisoquinoline (260 mg, 1.25 mmol), cesium carbonate (475 mg, 1.46 mmol), bis(diphenylphosphino)-9,9-dimethylxanthene (120 mg, 0.21 mmol) and tris(dibenzylidene acetone)dipalladium (0) (95 mg, 0.10 mmol) according to the method of Preparation 54. The crude was purified (gradient hexane/ethyl acetate) using the SP1 purification system to give 235 mg (0.44 mmol, 42%) of the title compound as a solid. Purity: 99%.
1 H NMR (200 MHz, CHLOROFORM-d) δ ppm 11.17 (1 H, br. s.), 9.19 (1 H, s), 8.69 (1 H, s), 8.00 - 8.14 (3 H, m), 7.93 (1 H, br. s.), 7.69 (5 H, m), 7.37 - 7.58 (3 H, m), 7.06 (1 H, br. s.), 6.33 (1 H, br. s.), 5.90 (1 H, br. s.), 3.73 (2 H, t, *J*=6.2 Hz), 3.26 (2 H, d, *J*=10.9 Hz), 2.95 (1 H, br. s.), 0.90 (2 H, d, *J*=5.1 Hz), 0.64 (2 H, br. s.).
HPLC/MS (30 min) retention time 11.20 min.
LRMS: *m*/*z* 525 (M+).

### EXAMPLE 56

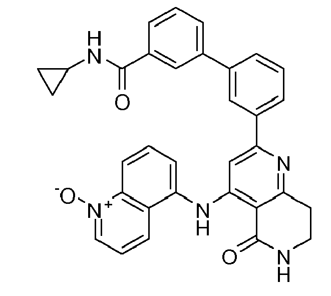

### N-Cyclopropyl-3'-{4-[(1-oxidoquinolin-5-yl)amino]-5-oxo-5,6,7,8-tetrahydro-1,6-naphthyridin-2-yl}biphenyl-3-carboxamide

The title compound of Example 49 (172 mg, 0.28 mmol) was treated with meta-chloroperbenzoic acid (max purity 77%, 142 mg, 0.64 mmol) according to the method of Preparation 23. The crude was purified by reverse phase using the SP1 purification system to give 37 mg (0.065 mmol, 24%) of the title compound as a solid. Purity: 97%.
1 H NMR (200 MHz, DMSO-*d*₆) δ ppm 11.75 (1 H, s), 8.66 (1 H, d, *J*=6.2 Hz), 8.55 (1 H, d, *J*=3.5 Hz), 8.38 (2 H, d, *J*=6.6 Hz), 8.21 (1 H, s), 8.08 (1 H, s), 7.72 - 7.98 (7 H, m), 7.48 - 7.62 (3 H, m), 7.36 (1 H, s), 3.53 (2 H, t), 3.10 (2 H, t, *J*=6.6 Hz), 2.78 - 2.96 (1 H, m), 0.70 (2 H, t, *J*=3.5 Hz), 0.53 - 0.65 (2 H, m).
HPLC/MS (30 min) retention time 9.80 min.
LRMS: *m*/*z* 541 (M+).

### EXAMPLE 57

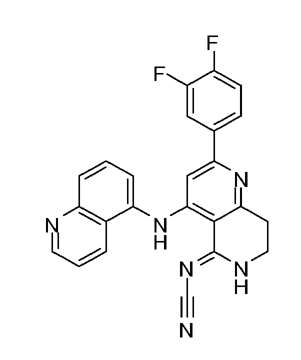

### [(5Z)-2-(3,4-Difluorophenyl)-4-(quinolin-5-ylamino)-7,8-dihydro-1,6-naphthyridin-5(6H)-ylidene]cyanamide

The title compound of Preparation 52 (35 mg, 0.08 mmol) was treated with cyanamide (16 mg, 0.38 mmol) according to the method of Example 53. The crude was purified by flash chromatography (gradient hexane/ethyl acetate 50:50 to ethyl acetate) to give 9 mg (0.021 mmol, 26%) of the title compound as a yellow solid. Purity: 99%.
¹H NMR (200 MHz, CHLOROFORM-d) δ ppm 10.92 (1 H, s), 9.01 (1 H, dd, *J*=4.0, 1.5 Hz), 8.33 (1 H, d, *J*=8.6 Hz), 8.15 (1 H, d, *J*=8.6 Hz), 7.82 (1 H, t, *J*=7.8 Hz), 7.68 (1 H, ddd, *J*=11.5, 8.0, 2.0 Hz), 7.58 (1 H, d, *J*=7.4 Hz), 7.48 (1 H, dd, *J*=8.6, 4.3 Hz), 7.40 (1 H, ddd, *J*=8.8, 4.0, 2.1 Hz), 7.11 (1 H, dd, *J*=18.0, 8.6 Hz), 7.01 (1 H, br. s.), 6.84 (1 H, s), 3.80 (2 H, td, *J*=6.8, 2.7 Hz), 3.25 (2 H, t, *J*=6.8 Hz).
HPLC/MS (30 min) retention time 15.46 min.
LRMS: *m*/*z* 426 (M+).

### EXAMPLE 58

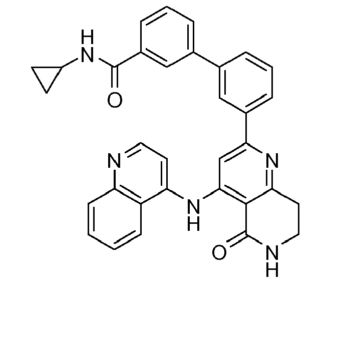

### N-Cyclopropyl-3'-[5-oxo-4-(quinolin-4-ylamino)-5,6,7,8-tetrahydro-1,6-naphthyridin-2-yl]biphenyl-3-carboxamide

The title compound of Preparation 45 (410 mg, 1.03 mmol) was treated with 4-bromoquinoline (257 mg, 1.24 mmol), cesium carbonate (470 mg, 1.44 mmol), bis(diphenylphosphino)-9,9-dimethylxanthene (120 mg, 0.21 mmol) and tris(dibenzylidene acetone)dipalladium (0) (95 mg, 0.10 mmol) according to the method of Preparation 54. The crude was purified in the SP1 purification system (hexane/ethyl acetate) to give 185 mg (0.35 mmol, 34%) of the title compound. Purity: 100%.
¹H NMR (200 MHz, DMSO-*d*₆) δ ppm 12.44 (1 H, s), 8.83 (1 H, d, *J*=5.1 Hz), 8.43 - 8.64 (2 H, m), 8.34 (1 H, br. s.), 8.01 - 8.22 (4 H, m), 7.98 (1 H, s), 7.47 - 7.93 (8 H, m), 3.55 (2 H, br. s.), 3.14 (2 H, t, *J*=6.2 Hz), 2.75 - 2.98 (1 H, m), 0.72 (2 H, d, *J*=6.6 Hz), 0.52 - 0.65 (2 H, m).
HPLC/MS (30 min) retention time 11.73 min.
LRMS: *m*/*z* 525 (M+).

### EXAMPLE 59

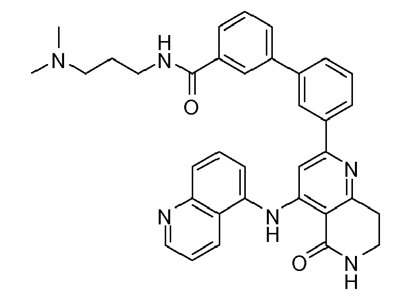

### N-[3-(Dimethylamino)propyl]-3'-[5-oxo-4-(quinolin-5-ylamino)-5,6,7,8-tetrahydro-1,6-naphthyridin-2-yl]biphenyl-3-carboxamide

The title compound of Preparation 55 (150 mg, 0.31 mmol) was dissolved in 5 mL of *N,N'-*dimethylformamide, 1-hydroxybenzotriazole hydrate (60 mg, 0.45 mmol) and *N-*(3-dimethylaminopropyl)-N'-ethylcarbodiimide hydrochloride (86 mg, 0.45 mmol) were added. The mixture was stirred at room temperature for 30 min. *N*¹,*N*¹-dimethylpropane-1,3-diamine (0.077 mL, 0.62 mmol) was added and the mixture was stirred at 50°C for 4 hours. The solvent was evaporated, 4% potassium carbonate aqueous solution and ethyl acetate were added, the organic layer was separated, washed twice with water and twice with brine, dried with magnesium sulphate, filtered and concentrated. The crude was purified by reverse phase using the SP1 purification system to give 28 mg (0.049 mmol, 16%) of the title compound as a white solid. Purity: 100%.
¹H NMR (200 MHz, CHLOROFORM-d) δ ppm 11.20 (1 H, s), 8.97 (1 H, d, J=3.1 Hz), 8.45 (3 H, d, J=8.6 Hz), 8.14 (2 H, d, J=5.1 Hz), 8.05 (1 H, d, J=8.2 Hz), 7.56 - 7.94 (5 H, m), 7.35 - 7.56 (3 H, m), 7.05 (1 H, s), 6.02 (1 H, br. s.), 3.44 - 3.83 (4 H, m), 3.24 (2 H, t, J=6.6 Hz), 2.81 (2 H, t, J=6.2 Hz), 2.51 (6 H, s), 1.97 (2 H, quin, J=5.8 Hz).
HPLC/MS (30 min) retention time 7.98 min.
LRMS: *m*/*z* 570 (M+).

### EXAMPLE 60

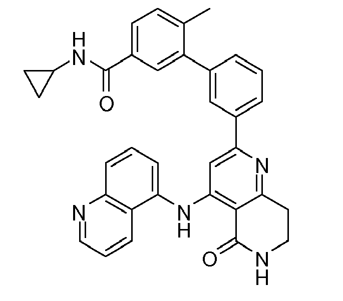

### N-Cyclopropyl-6-methyl-3'-[5-oxo-4-(quinolin-5-ylamino)-5,6,7,8-tetrahydro-1,6-naphthyridin-2-yl]biphenyl-3-carboxamide

The title compound of Preparation 56 (176 mg, 0.43 mmol) was treated with 5-bromoquinoline (110 mg, 0.51 mmol), cesium carbonate (194 mg, 0.60 mmol), bis(diphenylphosphino)-9,9-dimethylxanthene (49 mg, 0.08 mmol) and tris(dibenzylidene acetone)dipalladium (0) (39 mg, 0.04 mmol) according to the method of Preparation 54. The crude was purified by reverse phase using the SP1 purification system to give 103 mg (0.19 mmol, 45%) of the title compound as a yellow solid. Purity: 99%.
1 H NMR (200 MHz, DMSO-*d*₆) δ ppm 11.62 (s, 1 H), 8.97 (dd, *J*=4.29, 1.56 Hz, 1 H), 8.23 - 8.48 (m, 3 H), 7.71 - 8.00 (m, 6 H), 7.56 - 7.70 (m, 2 H), 7.33 - 7.55 (m, 3 H), 7.20 (s, 1 H), 3.46 - 3.59 (m, 2 H), 3.05 (t, *J*=6.64 Hz, 2 H), 2.77 - 2.92 (m, 1 H), 2.20 (s, 3 H), 0.61 - 0.74 (m, 2 H), 0.48 - 0.60 (m, 1 H).
HPLC/MS (30 min) retention time 11.63 min.
LRMS: *m*/*z* 539 (M+).

### EXAMPLE 61

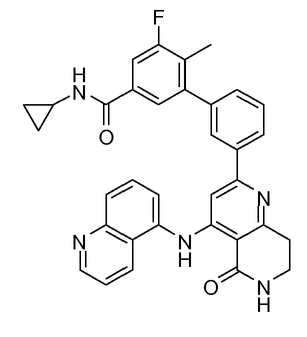

### N-Cyclopropyl-5-fluoro-6-methyl-3'-[5-oxo-4-(quinolin-5-ylamino)-5,6,7,8-tetrahydro-1,6-naphthyridin-2-yl]biphenyl-3-carboxamide

The title compound of Preparation 57 (100 mg, 0.23 mmol) was treated with 5-bromoquinoline (60 mg, 0.29 mmol), cesium carbonate (105 mg, 0.32 mmol), bis(diphenylphosphino)-9,9-dimethylxanthene (27 mg, 0.05 mmol) and tris(dibenzylidene acetone)dipalladium (0) (21 mg, 0.02 mmol) according to the method of Preparation 54. The crude was pufified by reverse phase using the SP1 purification system to give 28 mg (0.047 mmol, 20%) of the title compound. Purity: 94%.
¹H NMR (200 MHz, CHLOROFORM-d) δ ppm 11.19 (1 H, s), 8.97 (1 H, dd, *J*=4.3, 1.5 Hz), 8.44 (1 H, d, *J*=8.2 Hz), 8.07 (1 H, d, *J*=8.6 Hz), 7.72 - 7.83 (2 H, m), 7.58 - 7.70 (2 H, m), 7.39 - 7.51 (3 H, m), 7.27 - 7.39 (2 H, m), 7.00 (1 H, s), 6.22 (1 H, br. s.), 5.86 (1 H, br. s.), 3.70 (2 H, td, *J*=6.7, 2.5 Hz), 3.22 (2 H, t, *J*=6.6 Hz), 2.80 - 2.97 (1 H, m), 2.13 (3 H, d, *J*=2.3 Hz), 0.78 - 0.93 (2 H, m), 0.53 - 0.66 (2 H, m).
HPLC/MS (30 min) retention time 12.86 min.
LRMS: *m*/*z* 557 (M+).

### PHARMACOLOGICAL ACTIVITY

### PDE4 Assay Procedure

Measurement of Phosphodiesterase Activity was done using a [3H] cAMP Scintillation Proximity Assay (SPA) (GE Healthcare). Compounds to be tested were disolved in DMSO at a stock concentration of 1mM and serial dilutions were prepared in 50% DMSO to determine IC50s.

The reactions were conducted in 96-well plates (Corning, Ref.3604) at room temperature, in 0.1 mL of reaction buffer containing (final concentrations): 50 mM Tris-HCl, pH 7.5, 8.3 mM MgCl₂, 1.7 mM EGTA, 30 nM [3H] cAMP (approximately 150000 dpm/well) with or without the inhibitors. The reaction was initiated by adding yeast extract of recombinant PDE4 enzyme.

Plates were shaken for 1 h at room temperature and the incubation was terminated by adding 50 µL (0.5 mg /well) of SPA ytrium silicate beads (RPNQ 0150; GE Healthcare) in the presence of zinc sulfate. Plates were stored overnight in the dark and read on a TRILUX microtiter plate reader (Perkin Elmer).

The results are shown in Table 1.

| **Example** | **IC₅₀ PDE4 (nM)** |
|---|---|
| **1** | 1.6 |
| **2** | 12 |
| **8** | 8 |
| **9** | 32 |
| **12** | 0.28 |
| **13** | 0.34 |
| **14** | 0.23 |
| **16** | 1.6 |
| **17** | 25 |
| **18** | 1.5 |
| **19** | 2.3 |
| **22** | 3.6 |
| **24** | 0.96 |
| **25** | 9 |
| **26** | 117 |
| **32** | 52 |
| **33** | 9 |
| **35** | 0.81 |
| **37** | 14 |
| **38** | 2.3 |
| **41** | 6 |
| **43** | 10 |
| **44** | 640 |
| **47** | 9 |
| **49** | 0.17 |
| **51** | 0.43 |
| **56** | 0.05 |
| **57** | 0.9 |
| **59** | 0.105 |
| **61** | 0.05 |

It can be seen from Table 1 that the compounds of formula (I) are potent inhibitors of phosphodiesterase 4 (PDE 4). Preferred 7,8-dihydro-1,6-naphthyridin-5(6H)-one derivatives of the invention possess an IC₅₀ value for the inhibition of PDE4 (determined as defined above) of less than 10 nM, preferably less than 1 nM and most preferably less than 0.5 nM. The compounds are also capable of blocking the production of some pro-inflammatory cytokines such as, for example, TNFα.

Thus, they can be used in the treatment of allergic, inflammatory and immunological diseases, as well as those diseases or conditions where the blockade of pro-inflammatory cytokines or the selective inhibition of PDE 4 could be of benefit. These disease states include asthma, chronic obstructive pulmonary disease (COPD), allergic rhinitis, rheumatoid arthritis, osteoarthritis, osteoporosis, bone-formation disorders, glomerulonephritis, multiple sclerosis, ankylosing spondylitis, Graves ophtalmopathy, myasthenia gravis, diabetes insipidus, graft rejection, gastrointestinal disorders such as irritable bowel disease, ulcerative colitis or Crohn disease, septic shock, adult distress respiratory syndrome, and skin diseases such as atopic dermatitis, contact dermatitis, acute dermatomyositis and psoriasis. They can also be used as improvers of cerebrovascular function as well as in the treatment of other CNS related diseases such as dementia, Alzheimer's disease, depression, and as nootropic agents.

Like other PDE4 inhibitors (see references above) the compounds of the invention can also be used for blocking, after preventive and/or curative treatment, the erosive and ulcerogenic effects induced by a variety of etiological agents, such as antiinflammatory drugs (steroidal or non-steroidal antiinflammatory agents), stress, ammonia, ethanol and concentrated acids.

They can be used alone or in combination with antacids and/or antisecretory drugs in the preventive and/or curative treatment of gastrointestinal pathologies like drug-induced ulcers, peptic ulcers, H. Pylori-related ulcers, esophagitis and gastro-esophageal reflux disease.

They can also be used in the treatment of pathological situations where damage to the cells or tissues is produced through conditions like anoxia or the production of an excess of free radicals. Examples of such beneficial effects are the protection of cardiac tissue after coronary artery occlusion or the prolongation of cell and tissue viability when the compounds of the invention are added to preserving solutions intended for storage of transplant organs or fluids such as blood or sperm. They are also of benefit on tissue repair and wound healing.

### Combinations

The compounds of formula (I) of the present invention can also be used in combination with other drugs known to be effective in the treatment of the diseases or the disorders indicated above. For example the compounds of the present invention can be combined with (a) β2-adrenergic agonists, (b) anti-cholinergics, (c) anti-allergic agents, (d) anti-inflammatory agents, (e) immunosuppressants, and (f) anti-infectives; for simultaneous, separate or sequential use in the treatment of the human or animal body.

Accordingly, another embodiment of the invention is the use of the compounds of formula (I) of the present invention for the manufacture of a medicament for treatment or prevention of pathological conditions, diseases and disorders known to be susceptible of amelioration by inhibition of PDE4, as well as a method for treating a subject afflicted with a pathological condition or disease susceptible to amelioration by inhibition of PDE4, which comprises administering to said subject an effective amount of a compound of formula (I).

The compounds or pharmaceutically acceptable salts, solvates, N-oxides or deuterated derivatives thereof according to the invention may also be used in combination with another therapeutically active agent, for example a) a β2-adrenoreceptor agonist, b) an anti-cholinergic agent, c) an anti-allergic, d) an anti-inflammatory agent, e) an immunosuppressant, or f) an anti-infective agent.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day, most preferably once a day.

Examples of suitable β2-agonists that can be combined with the PDE4 inhibitors of the invention are terbutaline sulphate, eformoterol fumarate, formoterol fumarate, bambuterol, procaterol hydrochloride, sibenadet hydrochloride, mabuterol hydrochloride, albuterol sulphate, salbutamol sulphate, salmeterol xinafoate, carmoterol hydrochloride, (R)-albuterol hydrochloride, Levalbuterol hydrochloride; Levosalbutamol hydrochloride; (-)-Salbutamol hydrochloride, (R,R)-Formoterol tartrate; Arformoterol tartrate, Bedoradrine sulphate, Indacaterol, Trantinterol hydrochloride, AZD-3199, GSK-159802; GSK-597901, GSK-678007, GSK-642444;GSK-961081; AR-C98955AA, Milveterol hydrochloride, BI-1744-CL, and compounds described in international patent applications Nos. WO2007/124898, W02006/122788A1, WO2008/046598 and WO2008095720

Examples of suitable corticosteroids and glucocorticoids that can be combined with the PDE4 inhibitors of the invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Examples of suitable M3 antagonists (anticholinergics) that can be combined with the PDE4 inhibitors of the invention are tiotropium salts, oxitropium salts, flutropium salts, ipratropium salts, glycopyrronium salts, trospium salts, revatropate, espatropate, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-allergic agents that can be combined with the PDE4 inhibitors of the invention are anti-histamines (e.g. Methapyrilene, Mequitazine, Azelastine hydrochloride, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, Vapitadine hydrochloride, AZD-1744, GSK-1004723D, GSK-835726, SUN-1334H), CRTH2 antagonists (e.g. Ramatroban, AMG-009, OC-000459), or mast cell stabilizers (e.g. nedocromil, pemirolast potassium, chromoglycate sodium, suplatast tosilate)

Examples for suitable immunosupressants that can be combined with the PDE4 inhibitors of the invention are picremolimus, tacromilus, cyclosporine A, leflunomide, methotrexate, anti-TNF agents and compounds described in International patent applications Nos. WO2009/021696 and WO2008/077639.

Examples for suitable anti-infectives that can be combined with the PDE4 inhibitors of the invention are mupiricin, retapamulin, clotrimazole, ketoconazole and terbinafine.

Other possible combinations include, for example, a combination comprising a PDE4 inhibitor of the invention with another anti-inflammatory agent such as a non-steroidal anti-inflammatory drug (NSAID) such as a leukotriene antagonist (e.g. Ibudilast, Pranlukast hydrate, Zafirlukast, Montelukast, Tipelukast), a FLAP inhibitor, a lipoxygenase inhibitor, a cyclooxygenase inhibitor (e.g. diclofenac, ibuprofen, celecoxib); an elastase inhibitor, a Syk kinase inhibitor, a PI3Kδγ inhibitor or another PDE inhibitor (e.g. theophylline).

Examples of suitable Syk kinase inhibitors that can be combined with the PDE4 inhibitors of the invention are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Examples of suitable antagonists of the PI3Kδγ inhibitors that can be combined with the PDE4 inhibitors of the invention are 2-Methyl-2-[4-[3-methyl-2-oxo-8-(3-quinolinyl)-2,3-dihydro-1H-imidazo[4,5-c]quinolin-1-yl]phenyl]propanenitrile (BEZ-235 from Novartis), CAL-101 (from Calistoga Pharmaceuticals) and N-Ethyl-N'-[3-(3,4,5-trimethoxyphenylamino)pyrido[2,3-b]pyrazin-6-yl]thiourea (AEZS-126 from Aeterna Zentaris).

Particularly preferred pharmaceutical compositions according to the invention comprise a compound of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, formoterol, salmeterol, indacaterol, carmoterol, compounds described in International patent applications Nos. WO2009/021696 and WO2008/077639, methapyrilene, cetirizine, loratadine, ebastine, desloratadine, fexofenadine, azelastine, levocabastine, olopatadine, Montelukast, picremolimus, tacromilus, mupiricin, retapamulin, clotrimazole, ketoconazole, terbinafine.

Thus, in one aspect of the invention, the composition comprises a compound of formula (I) and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In another aspect of the invention, the composition comprises a compound of formula (I) and an anticholinergic agent. Particulary preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]octane salts. The composition may further comprise a corticosteroid selected from the group consisting of mometasone furoate, ciclesonide, budesonide, fluticasone furoate and fluticasone propionate.

In a still other aspect of the invention, the composition comprises a compound of formula (I) and a suitable β2-agonist. Particularly preferred β2-agonists are those selected from the group consisting of formoterol, salmeterol, indacaterol, carmoterol and compound described in International patent applications Nos. WO2007/124898, WO2006/122788A1, WO2008/046598 and WO2008095720. The composition may further comprise a corticosteroid selected from the group consisting of mometasone furoate, ciclesonide, budesonide, fluticasone furoate, and fluticasone propionate. In addition to the salt of the invention and to the β2-agonist, the composition may further comprise an anticholinergic agent selected from the group consisting of tiotropium salts, glycopyrronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

The compounds of formula (I) and the combinations of the invention may be used in the treatment of respiratory, skin and inflammatory diseases, wherein the use of a PDE4 inhibitor is expected to have a beneficial effect, for example asthma, chronic obstructive pulmonary disease (COPD), allergic rhinitis, rheumatoid arthritis, multiple sclerosis, atopic dermatitis, psoriasis and inflammatory bowel disease.

The active compounds in the combination may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

### Pharmaceutical Compositions

Pharmaceutical compositions according to the present invention comprise the compounds of the invention in association with a pharmaceutically acceptable diluent or carrier.

The invention further provides pharmaceutical compositions comprising the compounds of the invention in association with a pharmaceutically acceptable diluent or carrier together with one or more other therapeutic agents such as the previously described for use in the treatment of a pathological condition or disease susceptible to amelioration by inhibition of phosphodiesterase IV.

The invention is also directed to pharmaceutical compositions of the invention for use in the treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

The invention also provides a method of treatment of a pathological condition or disease susceptible to amelioration by inhibition of phosphodiesterase IV, in particular wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a pharmaceutically acceptable excipient such as a carrier or diluent. The active ingredient may comprise 0.001 % to 99% by weight, preferably 0.01 % to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

The pharmaceutically acceptable excipients which are admixed with the active compound or salts of such compound, to form the compositions of this invention are well-known per se and the actual excipients used depend inter alia on the intended method of administering the compositions.

Additional suitable carriers for formulations of the compounds of the present invention can be found in Remington: The Science and Practice of Pharmacy, 20th Edition, Lippincott Williams & Wilkins, Philadelphia, Pa., 2000. The following non-limiting examples illustrate representative pharmaceutical compositions of the invention.

Compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 0.01-3000 mg, more preferably 0.5-1000 mg of active ingredient or the equivalent amount of a pharmaceutically acceptable salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Compositions for topical administration may take the form of ointments, creams or lotions, all containing the compound of the invention; such preparations may be made by methods well-known in the art.

Effective doses are normally in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-50 mg, more preferably 0.01-5 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuiar® (formerly known as Novolizer SD2FL), which is described the following patent applications Nos: WO97/000703, WO03/000325 and WO03/061742.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the respimat® which is described, for example, in PCT Patent Applications Nos. W0 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetrafluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate. Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The active substance compositions according to the invention are preferably administered in the form of compositions for inhalation delivered with the help of inhalers, especially dry powder inhalers; however, any other form of nasal, topical, parenteral or oral application is possible. Here, the application of inhaled compositions embodies the preferred application form, especially in the therapy of obstructive lung diseases or for the treatment of asthma.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of 4-[(3-Methoxyphenyl)amino]-2-phenyl-7,8-dihydro-1,6-naphthyridin-5(6H)-one (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 4-[(3-Methoxyphenyl)amino]-2-phenyl-7,8-dihydro-1,6-naphthyridin-5(6H)-one (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, or solvate, or N-oxide, or stereoisomer or deuterated derivate thereof: 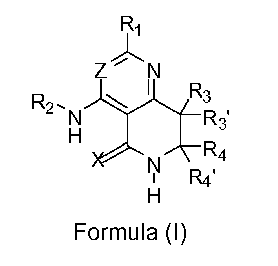 wherein
Z represents a nitrogen atom or a -CR group;
X represents an oxygen atom or a -NR₅ group;
R represents a hydrogen atom or a linear or branched C₁-C₄ alkyl group;
R₁ represents a C₃-C₁₀ cycloalkyl group or a C₃-C₁₀ cycloalkenyl group, a monocyclic or polycyclic C₅-C₁₄ aryl group or a 5- to 14- membered heteroaryl group containing at least one heteroatom selected from O, S and N;
wherein the cycloalkyl, cycloalkenyl, aryl and heteroaryl groups are unsubstituted or substituted by one or more substituents selected from a halogen atom, a C₁-C₄ alkoxy group, a -SR₆ group or a phenyl group, which phenyl group is unsubstituted or substituted by one or more substituents selected from a halogen atom, a hydroxy group, a linear or branched C₁-C₄ alkyl group, a -C(O)OR₇ group, a -C(O)NR₈R₉ group, or a -C(O)N(R₇)-(CH₂)ₙ-NR₈R₉ group, wherein n is 1, 2, 3 or 4;
R₂ represents a hydrogen atom, a linear or branched C₁-C₄ alkyl group, a C₁-C₄ haloalkyl group, a monocyclic or polycyclic C₅-C₁₄ aryl group, a 5- to 14- membered heteroaryl group containing at least one heteroatom selected from O, S and N, or a 5- to 14- membered heterocyclyl group containing at least one heteroatom selected from O, S and N,
wherein the alkyl and haloalkyl groups are unsubstituted or substituted by one or more substituents selected from a cyano group, a pyridyl group, a phenyl group, a benzyl group, a -C(O)OR₇' group or a -C(O)NR₈'R₉' group; and the aryl, heteroaryl and heterocyclyl groups are unsubstituted or substituted by one or more substituents selected from a halogen atom, a cyano group, a linear or branched C₁-C₄ alkyl group, a C₁-C₄ alkoxy group, a C₁-C₄ hydroxyalkyl group, a phenyl group, a benzyl group, a -C(O)OR₇' group or a -C(O)NR₈'R₉' group;
R₃ and R₃' independently represent a hydrogen atom or a linear or branched C₁-C₄ alkyl group,
R₄ and R₄' independently represent a hydrogen atom or a linear or branched C₁-C₄ alkyl group,
R₅ represents a hydrogen atom, a hydroxy group, a cyano group, a linear or branched C₁-C₄ alkyl group or a C₁-C₄ alkoxy group;
R₆ represents a phenyl group which is unsubstituted or substituted by one or more substituents selected from a halogen atom, a linear or branched C₁-C₄ alkyl group or a C₁-C₄ alkoxy group; and
R₇, R₇', R₈, R₈', R₉ and R₉' independently represent a hydrogen atom, a linear or branched C₁-C₄ alkyl group or a C₃-C₆ cycloalkyl group; or R₈ and R₉ or R₈' and R₉', together with the nitrogen atom to which they are bounded optionally form a 3- to 10- membered, saturated N-containing heterocyclyl group, containing 1, 2 or 3 other heteroatoms selected from O, S and N, wherein said heterocyclyl group is unsubstituted or substituted by one or more substituents selected from a halogen atom, a hydroxy group or a linear or branched C₁-C₄ alkyl group.

2. A compound according to claim 1, wherein Z represents a -CR group wherein R is as defined in claim 1; preferably Z represents a -CR group wherein R is a hydrogen atom or a methyl group; more preferably Z represents a -CH group.

3. A compound according to claim 1 or claim 2, wherein X represents an oxygen atom.

4. A compound according to any one of the preceding claims, wherein R₁ represents a cyclopropyl group, a phenyl group or a pyridyl group, wherein the cyclopropyl, phenyl and pyridyl groups are unsubstituted or substituted by 1, 2 or 3 substituents selected from a halogen atom, a C₁-C₄ alkoxy group, a -SR₆ group or a phenyl group, which phenyl group is unsubstituted or substituted by one or more substituents selected from a halogen atom, a hydroxy group, a linear or branched C₁-C₄ alkyl group, a -C(O)OR₇ group, a -C(O)NR₈R₉ group or a -C(O)N(R₇)-(CH₂)ₙ-NR₈R₉ group, wherein n is 1, 2, 3 or 4; and wherein R₆, R₇, R₈ and R₉ are as defined in claim 1.

5. A compound according to claim 4, wherein R₁ represents a pyridyl group, wherein said pyridyl group is optionally in the N-oxide form.

6. A compound according to claim 4, wherein R₁ represents a phenyl group which is unsubstituted or substituted by 1, 2 or 3 substituents selected from a halogen atom, a C₁-C₄ alkoxy group, a -SR₆ group or a phenyl group, which phenyl group is substituted by 1, 2 or 3 substituents selected from a halogen atom, a hydroxy group, a linear or branched C₁-C₄ alkyl group, a -C(O)OR₇ group, a -C(O)NR₈R₉ group or a -C(O)N(R₇)-(CH₂)ₙ-NR₈R₉ group, wherein n is 1, 2, 3 or 4; and wherein R₆, R₇, R₈ and R₉ are as defined in claim 1.

7. A compound according to claim 6, wherein R₁ represents a phenyl group which is unsubstituted or substituted by 1 or 2 substituents selected from a halogen atom, a methoxy group, a -SR₆ group or a phenyl group, which phenyl group is substituted by 1, 2 or 3 substituents selected from a halogen atom, a hydroxy group, a methyl group, a -C(O)OR₇ group, a -C(O)NR₈R₉ group or a -C(O)N(R₇)-(CH₂)ₙ-NR₈R₉ group, wherein n is 1, 2, 3 or 4; and wherein R₆ represents a phenyl group substituted by 1, 2 or 3 C₁-C₄ alkoxy groups; and wherein R₇, R₈ and R₉ independently represent a hydrogen atom, a linear or branched C₁-C₄ alkyl group or a C₃-C₆ cycloalkyl group.

8. A compound according to any one of the preceding claims, wherein R₂ represents a a linear or branched C₁-C₄ alkyl group, a phenyl group, a naphthalenyl group, a 5- to 14-membered heteroaryl group containing 1, 2 or 3 nitrogen atoms, or a 5- to 6- membered, saturated N-containing heterocyclyl ring; wherein the alkyl group is unsubstituted or substituted by 1, 2 or 3 substituents selected from a pyridyl group, a phenyl group, a benzyl group or a -C(O)NR₈'R₉' group; and the phenyl, naphthalenyl, heteroaryl and heterocyclyl groups are unsubstituted or substituted by 1, 2 or 3 substituents selected from a halogen atom, a cyano group, a linear or branched C₁-C₄ alkyl group, a C₁-C₄ alkoxy group, a C₁-C₄ hydroxyalkyl group, a phenyl group, a -C(O)OR₇' group or a -C(O)NR₈'R₉' group; and wherein R₇', R₈' and R₉' independently represent a hydrogen atom, a linear or branched C₁-C₄ alkyl group or a C₃-C₆ cycloalkyl group.

9. A compound according to claim 8, wherein R₂ represents a linear or branched C₁-C₄ alkyl group substituted by a pyridyl group, or R₂ represents a phenyl group, a naphthalenyl group, a pyridyl group, a quinolyl group, an isoquinolyl group, a cinnolinyl group, a phthalazinyl group, a quinazolinyl group, a quinoxalinyl group, a 1,8-naphthyridinyl group, a 1,6-naphthyridinyl group, a 1H-pyrazolo[3,4-b]pyridinyl group or a piperidinyl group, wherein the phenyl, naphthalenyl, pyridyl, quinolyl, isoquinolyl, cinnolinyl, phthalazinyl, quinazolinyl, quinoxalinyl, 1,8-naphthyridinyl, 1,6-naphthyridinyl, 1H-pyrazolo[3,4-b]pyridinyl or piperidinyl groups are unsubstituted or substituted by 1, 2 or 3 substituents selected from a halogen atom, a methyl group, a methoxy group, a C₁-C₄ hydroxyalkyl group, a phenyl group, a -C(0)OR₇' group or a -C(O)NR₈'R₉' group; wherein R₇', R₈'and R₉' independently represent a hydrogen atom, a linear or branched C₁-C₃ alkyl group or a C₃-C₄ cycloalkyl group.

10. A compound according to claim 8, wherein when R₂ represents a heteroaryl group or a heterocyclyl group, said group is in the N-oxide form.

11. A compound according to any one of the preceding claims, wherein R₃ and R₃' independently represent a hydrogen atom or a methyl group; preferebly R₃ and R₃' independently represent a hydrogen atom.

12. A compound according to any one of the preceding claims, wherein R₄ and R₄' independently represent a hydrogen atom or a methyl group; preferebly R₄ and R₄' independently represent a hydrogen atom.

13. A compound according to claim 1 or claim 2 or claims 4 to 12, wherein R₅ represents a hydrogen atom, a hydroxy group or a cyano group.

14. A compound according to any one of the preceding claims, wherein R₇ represents a hydrogen atom or a linear or branched C₁-C₃ alkyl group, and/or R₈ represents a hydrogen atom, a linear or branched C₁-C₃ alkyl group or a C₃-C₄ cycloalkyl group, and/or R₉ represents a hydrogen atom, a linear or branched C₁-C₃ alkyl group or a C₃-C₄ cycloalkyl group, and/or R₇' represents a hydrogen atom or a linear or branched C₁-C₃ alkyl group, and/or R₈' represents a hydrogen atom, a linear or branched C₁-C₃ alkyl group or a C₃-C₄ cycloalkyl group and/or R₉' represents a hydrogen atom, a linear or branched C₁-C₃ alkyl group or a C₃-C₄ cycloalkyl group.

15. A compound according to any one of the preceding claims, wherein n is 2 or 3.

16. A compound according to any one of the preceding claims, wherein R₆ represents a phenyl group substituted by 1, 2 or 3 C₁-C₄ alkoxy groups.

17. A compound according to claim 1, wherein
Z represents a nitrogen atom or a -CH group;
X represents an oxygen atom or a -NR5 group;
R₁ represents a cyclopropyl group, a pyridyl group, wherein said pyridyl group is optionally in the N-oxide form, or a phenyl group, wherein the phenyl group is unsubstituted or substituted by 1 or 2 substituents selected from a halogen atom, a methoxy group, a -SR₆ group or a phenyl group, which phenyl group is substituted by 1, 2 or 3 substituents selected from a halogen atom, a hydroxy group, a methyl group, a -C(O)OR₇ group, a - C(O)NR₈R₉ group or a -C(O)N(R₇)-(CH₂)ₙ-NR₈R₉ group, wherein n is 1, 2, 3 or 4; and wherein R₆ represents a phenyl group substituted by 1, 2 or 3 C₁-C₄ alkoxy groups; and wherein R₇, R₈ and R₉ independently represent a hydrogen atom, a linear or branched C₁-C₃ alkyl group or a C₃-C₄ cycloalkyl group,
R₂ represents a hydrogen atom, a linear or branched C₁-C₄ alkyl group, a phenyl group, a naphthalenyl group, a pyridyl group, a quinolyl group, an isoquinolyl group, a 1H-pyrazolo[3,4-b]pyridinyl group or a piperidinyl group, wherein the pyridyl, quinolyl, isoquinolyl, 1H-pyrazolo[3,4-b]pyridinyl group or piperidinyl groups are optionally in the N-oxide form; wherein the alkyl group is substituted by a pyridyl group, a phenyl group, a benzyl group or -C(O)NR₈'R₉' group; and the phenyl, naphthalenyl, pyridyl, quinolyl, isoquinolyl, 1H-pyrazolo[3,4-b]pyridinyl or piperidinyl groups are unsubstituted or substituted by 1, 2 or 3 substituents selected from a halogen atom, a cyano group, a methyl group, a C₁-C₄ alkoxy group, a C₁-C₄ hydroxyalkyl group, a phenyl group, a -C(O)OR₇' group, or a -C(O)NR₈'R₉' group; and wherein R₇', R₈'and R₉' independently represent a hydrogen atom, a linear or branched C₁-C₃ alkyl group or a C₃-C₄ cycloalkyl group;
R₃ and R₃' independently represent a hydrogen atom or a methyl group;
R₄ and R₄' independently represent a hydrogen atom or a methyl group; and
R₅ represents a hydroxy group or a cyano group.

18. A compound according to claim 14, wherein
Z represents a -CH group;
X represents an oxygen atom;
R₁ represents a cyclopropyl group, a pyridyl group, wherein said pyridyl group is optionally in the N-oxide form, and a phenyl group, wherein the phenyl group is unsubstituted or substituted by 1 or 2 substituents selected from a halogen atom, a methoxy group, a -SR₆ group or a phenyl group, which phenyl group is substituted by 1, 2 or 3 substituents selected from a halogen atom, a hydroxy group, a methyl group, a -C(O)OR₇ group, a -C(O)NR₈R₉ group or a -C(O)N(R₇)-(CH₂)ₙ-NR₈R₉ group, wherein n is 1, 2, 3 or 4; and wherein R₆ represents a phenyl group substituted by 1, 2 or 3 C₁-C₄ alkoxy groups; and wherein R₇, R₈ and R₉ independently represent a hydrogen atom, a linear or branched C₁-C₃ alkyl group or a C₃-C₄ cycloalkyl group,
R₂ represents a hydrogen atom, a linear or branched C₁-C₄ alkyl group substituted by a pyridyl group, or R₂ represents a phenyl group, a naphthalenyl group, a pyridyl group, a quinolyl group, an isoquinolyl group, a 1H-pyrazolo[3,4-b]pyridinyl group or a piperidinyl group, wherein the pyridyl, quinolyl, isoquinolyl, 1H-pyrazolo[3,4-b]pyridinyl group or piperidinyl groups are optionally in the N-oxide form; wherein the phenyl, naphthalenyl, pyridyl, quinolyl, isoquinolyl, 1H-pyrazolo[3,4-b]pyridinyl or piperidinyl groups unsubstituted or substituted by 1, 2 or 3 substituents selected from a halogen atom, a cyano group, a methyl group, a C₁-C₄ alkoxy group, a C₁-C₄ hydroxyalkyl group, a phenyl group, a -C(O)OR₇' group, or a -C(O)NR₈'R₉' group; and wherein R₇', R₈' and R₉' independently represent a hydrogen atom, a linear or branched C₁-C₃ alkyl group or a C₃-C₄ cycloalkyl group;
R₃ and R₃' independently represent a hydrogen atom;
R₄ and R₄' independently represent a hydrogen atom.

19. A compound according to claim 1 which is one of:
4-[(3-methoxyphenyl)amino]-2-phenyl-7,8-dihydro-1,6-naphthyridin-5(6H)-one;
4-{[4-(2-hydroxyethyl)phenyl]amino}-2-phenyl-7,8-dihydro-1,6-naphthyridin-5(6H)-one;
4-(1-naphthylamino)-2-phenyl-7,8-dihydro-1,6-naphthyridin-5(6H)-one;
2-phenyl-4-(piperidin-4-ylamino)-7,8-dihydro-1,6-naphthyridin-5(6H)-one;
3-[(5-oxo-2-phenyl-5,6,7,8-tetrahydro-1,6-naphthyridin-4-yl)amino]benzonitrile;
4-{[3-(hydroxymethyl)phenyl]amino}-2-phenyl-7,8-dihydro-1,6-naphthyridin-5(6H)-one;
2-(3-bromophenyl)-4-[(3-methoxyphenyl)amino]-7,8-dihydro-1,6-naphthyridin-5(6H)-one;
3'-{4-[(3-methoxyphenyl)amino]-5-oxo-5,6,7,8-tetrahydro-1,6-naphthyridin-2-yl}biphenyl-3-carboxamide;
3'-{4-[(3-methoxyphenyl)amino]-5-oxo-5,6,7,8-tetrahydro-1,6-naphthyridin-2-yl}biphenyl-4-carboxylic acid;
N-[2-(dimethylamino)ethyl]-3'-{4-[(3-methoxyphenyl)amino]-5-oxo-5,6,7,8-tetrahydro-1,6-naphthyridin-2-yl}biphenyl-3-carboxamide;
2-(3'-hydroxybiphenyl-3-yl)-4-[(3-methoxyphenyl)amino]-7,8-dihydro-1,6-naphthyridin-5(6H)-one;
2-(3-methoxyphenyl)-4-[(3-methoxyphenyl)amino]-7,8-dihydro-1,6-naphthyridin-5(6H)-one;
2-(2-methoxyphenyl)-4-[(3-methoxyphenyl)amino]-7,8-dihydro-1,6-naphthyridin-5(6H)-one;
4-[(3-methoxyphenyl)amino]-2-{3-[(3-methoxyphenyl)thio]phenyl}-7,8-dihydro-1,6-naphthyridin-5(6H)-one;
4-[(3-fluoro-2-methylphenyl)amino]-2-phenyl-7,8-dihydro-1,6-naphthyridin-5(6H)-one;
4-[(3,5-dimethoxyphenyl)amino]-2-phenyl-7,8-dihydro-1,6-naphthyridin-5(6H)-one;
2-phenyl-4-[(pyridin-4-ylmethyl)amino]-7,8-dihydro-1,6-naphthyridin-5(6H)-one;
4-[(5-oxo-2-phenyl-5,6,7,8-tetrahydro-1,6-naphthyridin-4-yl)amino]benzamide;
2-methoxy-4-[(5-oxo-2-phenyl-5,6,7,8-tetrahydro-1,6-naphthyridin-4-yl)amino]benzoic acid;
3-[(5-oxo-2-phenyl-5,6,7,8-tetrahydro-1,6-naphthyridin-4-yl)amino]benzamide;
2-phenyl-4-[(2-pyridin-4-ylethyl)amino]-7,8-dihydro-1,6-naphthyridin-5(6H)-one;
4-[(3-methoxyphenyl)amino]-2-pyridin-4-yl-7,8-dihydro-1,6-naphthyridin-5(6H)-one;
4-[(3-methoxyphenyl)amino]-2-(1-oxidopyridin-4-yl)-7,8-dihydro-1,6-naphthyridin-5(6H)-one;
2-phenyl-4-(quinolin-5-ylamino)-7,8-dihydro-1,6-naphthyridin-5(6H)-one;
4-[(5-oxo-2-phenyl-5,6,7,8-tetrahydro-1,6-naphthyridin-4-yl)amino]benzoic acid;
4-[(3-methoxyphenyl)amino]-7-methyl-2-phenyl-7,8-dihydro-1,6-naphthyridin-5(6H)-one;
4-amino-2-phenyl-7,8-dihydro-1,6-naphthyridin-5(6H)-one;
2-chloro-4-[(5-oxo-2-phenyl-5,6,7,8-tetrahydro-1,6-naphthyridin-4-yl)amino]benzoic acid;
N-(5-oxo-2-phenyl-5,6,7,8-tetrahydro-1,6-naphthyridin-4-yl)-L-phenylalaninamide;
4-[(3-fluorophenyl)amino]-2-phenyl-7,8-dihydro-1,6-naphthyridin-5(6H)-one;
4-[(3-chlorophenyl)amino]-2-phenyl-7,8-dihydro-1,6-naphthyridin-5(6H)-one;
4-[(3-methoxyphenyl)amino]-2-phenyl-7,8-dihydropyrido[4,3-d]pyrimidin-5(6H)-one;
2-cyclopropyl-4-[(3-methoxyphenyl)amino]-7,8-dihydro-1,6-naphthyridin-5(6H)-one;
N-cyclopropyl-3'-{4-[(3-methoxyphenyl)amino]-5-oxo-5,6,7,8-tetrahydro-1,6-naphthyridin-2-yl}biphenyl-3-carboxamide;
6-chloro-3'-{4-[(3-methoxyphenyl)amino]-5-oxo-5,6,7,8-tetrahydro-1,6-naphthyridin-2-yl}biphenyl-3-carboxylic acid;
ethyl 3'-{4-[(3-methoxyphenyl)amino]-5-oxo-5,6,7,8-tetrahydro-1,6-naphthyridin-2-yl}biphenyl-4-carboxylate;
4-(isoquinolin-5-ylamino)-2-phenyl-7,8-dihydro-1,6-naphthyridin-5(6H)-one;
2-phenyl-4-(quinolin-6-ylamino)-7,8-dihydro-1,6-naphthyridin-5(6H)-one;
4-[(4-methylpyridin-3-yl)amino]-2-phenyl-7,8-dihydro-1,6-naphthyridin-5(6H)-one;
methyl 6-hydroxy-3'-{4-[(3-methoxyphenyl)amino]-5-oxo-5,6,7,8-tetrahydro-1,6-naphthyridin-2-yl}biphenyl-3-carboxylate;
2-phenyl-4-(1H-pyrazolo[3,4-b]pyridin-3-ylamino)-7,8-dihydro-1,6-naphthyridin-5(6H)-one;
4-[(1-methyl-1H-pyrazolo[3,4-b]pyridin-3-yl)amino]-2-phenyl-7,8-dihydro-1,6-naphthyridin-5(6H)-one;
6-hydroxy-3'-{4-[(3-methoxyphenyl)amino]-5-oxo-5,6,7,8-tetrahydro-1,6-naphthyridin-2-yl}biphenyl-3-carboxylic acid;
4-[(3-methoxyphenyl)amino]-8-methyl-2-phenyl-7,8-dihydro-1,6-naphthyridin-5(6H)-one;
2-phenyl-4-[(1-phenyl-1H-pyrazolo[3,4-b]pyridin-3-yl)amino]-7,8-dihydro-1,6-naphthyridin-5(6H)-one;
2-(3'-hydroxybiphenyl-3-yl)-4-(quinolin-5-ylamino)-7,8-dihydro-1,6-naphthyridin-5(6H)-one;
4-({4-[(2R)-2,3-dihydroxypropyl]phenyl}amino)-2-phenyl-7,8-dihydro-1,6-naphthyridin-5(6H)-one;
3'-[5-oxo-4-(quinolin-5-ylamino)-5,6,7,8-tetrahydro-1,6-naphthyridin-2-yl]biphenyl-4-carboxylic acid;
N-cyclopropyl-3'-[5-oxo-4-(quinolin-5-ylamino)-5,6,7,8-tetrahydro-1,6-naphthyridin-2-yl]biphenyl-3-carboxamide;
ethyl 3'-[5-oxo-4-(quinolin-5-ylamino)-5,6,7,8-tetrahydro-1,6-naphthyridin-2-yl]biphenyl-4-carboxylate;
4-[(1-oxidoquinolin-5-yl)amino]-2-phenyl-7,8-dihydro-1,6-naphthyridin-5(6H)-one;
2-(3,4-difluorophenyl)-4-(quinolin-5-ylamino)-7,8-dihydro-1,6-naphthyridin-5(6H)-one;
[(5Z)-2-(3-methoxyphenyl)-4-[(3-methoxyphenyl)amino]-7,8-dihydro-1,6-naphthyridin-5(6H)-ylidene]cyanamide;
(5Z)-2-(3-methoxyphenyl)-4-[(3-methoxyphenyl)amino]-7,8-dihydro-1,6-naphthyridin-5(6H)-one oxime;
N-cyclopropyl-3'-[4-(isoquinolin-4-ylamino)-5-oxo-5,6,7,8-tetrahydro-1,6-naphthyridin-2-yl]biphenyl-3-carboxamide;
N-cyclopropyl-3'-{4-[(1-oxidoquinolin-5-yl)amino]-5-oxo-5,6,7,8-tetrahydro-1,6-naphthyridin-2-yl}biphenyl-3-carboxamide;
[(5Z)-2-(3,4-difluorophenyl)-4-(quinolin-5-ylamino)-7,8-dihydro-1,6-naphthyridin-5(6H)-ylidene]cyanamide;
N-cyclopropyl-3'-[5-oxo-4-(quinolin-4-ylamino)-5,6,7,8-tetrahydro-1,6-naphthyridin-2-yl]biphenyl-3-carboxamide;
N-[3-(dimethylamino)propyl]-3'-[5-oxo-4-(quinolin-5-ylamino)-5,6,7,8-tetrahydro-1,6-naphthyridin-2-yl]biphenyl-3-carboxamide;
N-cyclopropyl-6-methyl-3'-[5-oxo-4-(quinolin-5-ylamino)-5,6,7,8-tetrahydro-1,6-naphthyridin-2-yl]biphenyl-3-carboxamide;
N-cyclopropyl-5-fluoro-6-methyl-3'-[5-oxo-4-(quinolin-5-ylamino)-5,6,7,8-tetrahydro-1,6-naphthyridin-2-yl]biphenyl-3-carboxamide;
and pharmaceutically acceptable salts, solvates, N-oxides or deuterated derivatives thereof.

20. A compound according to any one of claims 1 to 19 for use in the treatment of a pathological condition or disease susceptible to amelioration by inhibition of phosphodiesterase IV.

21. A compound according to claim 20, wherein the pathological condition or disease is selected from asthma, chronic obstructive pulmonary disease (COPD), allergic rhinitis, rheumatoid arthritis, multiple sclerosis, atopic dermatitis, psoriasis or inflammatory bowel disease.

22. A pharmaceutical composition comprising at least a compound as defined in any one of claims 1 to 19 in association with a pharmaceutically acceptable diluent or carrier.

23. Use of a compound as defined in any one of claims 1 to 19 for the manufacture of a medicament for the treatment of a pathological condition or disease as defined in claims 20 or 21.

24. A method for treating a subject afflicted with a pathological condition or disease as defined in claims 20 or 21, which comprises administering to said subject an effective amount of a compound as defined in any one of claims 1 to 19, or a pharmaceutical composition as defined in claim 22.

25. A combination product comprising
(i) at least a compound as defined in any one of claims 1 to 19, and
(ii) one or more active ingredients selected from the group consisting of (a) β2-adrenergic agonists, (b) anti-cholinergics (c) antiinflammatory agents, (d) anti-allergic agents, (e) immunosuppressants and (f) anti-infectives;
for simultaneous, separate or sequential use in the treatment of the human or animal body.
